# EUROPÄISCHE PATENTSCHRIFT

(11) **EP 0 905 131 B1**
(45) Veröffentlichungstag und Bekanntmachung des Hinweises auf die Patenterteilung: **10.04.2002**
(21) Anmeldenummer: 98117809.8
(22) Anmeldetag: 19.09.1998
(51) Int. Cl.: C07D 311/68, C07D 311/70, C07D 311/72, C07D 405/12, C07D 417/12, C07D 413/12, A61K 31/35

(54) **Sulfonamid-substituierte Chromane, Verfahren zu ihrer Herstellung, ihre Verwendung als Medikament sowie sie enthaltende pharmazeutische Zubereitungen**
Sulfonamid-substituted chromans, process for their preparation, their use as medicine, and pharmaceutical preparation containing them
Chromanes substitués par une sulfonamide, procédé pour leur préparation, leur utilisation comme médicament et compositions pharmaceutiques les contenant

(30) Priorität: 26.09.1997 DE 19742509
(43) Veröffentlichungstag der Anmeldung: 31.03.1999
(73) Patentinhaber: Aventis Pharma Deutschland GmbH, 65929 Frankfurt am Main (DE)
(72) Erfinder: Brendel, Joachim Dr., 61118 Bad Vilbel (DE); Gerlach, Uwe Dr., 65795 Hattersheim (DE); Lang, Hans Jochen Dr., 65719 Hofheim (DE); Weidmann, Klaus Dr., 61476 Kronberg (DE)

(56) Entgegenhaltungen:
- EP-A- 0 315 009
- EP-A- 0 389 861
- EP-A- 0 807 629
- EP-A- 0 860 440
- E.LOHRMANN ET AL.: "A NEW CLASS OF INHIBITORS OF c-AMPMEDIATED CL- SECRETION IN RABBIT COLON" PFLÜGERS ARCH.-EUR. J. PHYSIOL., Bd. 429, Nr. 4, 1995, Seiten 517-530, XP002038768 DE

## Beschreibung

Die Erfindung betrifft Verbindungen der Formel I, 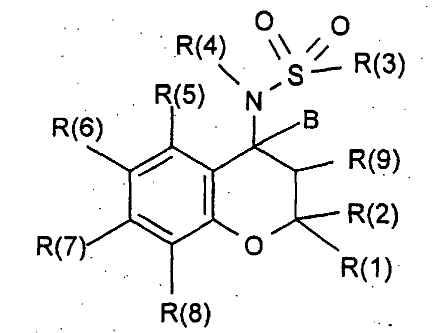 worin R(1), R(2), R(3), R(4), R(5), R(6), R(7), R(8), R(9) und B die im folgenden angegebenen Bedeutungen haben, ihre Herstellung und ihre Verwendung, insbesondere in Arzneimitteln. Die Verbindungen beeinflussen den durch cyclisches Adenosinmonophosphat (cAMP) geöffneten Kaliumkanal bzw. den I_{Ks}-Kanal und eignen sich in hervorragender Weise als Arzneimittelwirkstoffe, beispielsweise zur Prophylaxe und Therapie von Herz-Kreislauferkrankungen, insbesondere Arrhythmien, zur Behandlung von Ulcera des Magen-Darm-Bereichs oder zur Behandlung von Durchfallerkrankungen.

In der Arzneimittelchemie wurde in den letzten Jahren die Klasse der 4-Acylaminochroman-Derivate intensiv bearbeitet. Prominentester Vertreter dieser Klasse ist das Cromakalim der Formel A (J. Med. Chem. 1986, 29, 2194).

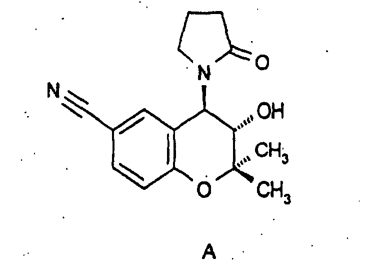

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺lonen induzierte Anstieg von negativer Ladung in der Zelle (Hyperpolarisation) wirkt über Sekundärmechanismen der Erhöhung der intrazellulären Ca²⁺-Konzentration und damit einer Zellaktivierung entgegen, die z. B. zu einer Muskelkontraktion führt.

Von diesen Acylamino-Derivaten unterscheiden sich die erfindungsgemäßen Verbindungen der Formel I strukturell unter anderem durch den Ersatz der Acylaminogruppe durch eine Sulfonylaminofunktion. Während Cromakalim (Formel A) und analoge Acylaminoverbindungen als Öffner ATP-sensitiver K⁺-Kanäle wirken, zeigen die erfindungsgemäßen Verbindungen der Formel I mit der Sulfonylaminostruktur jedoch keine öffnende Wirkung auf diesen K⁺(ATP)-Kanal, sondern überraschenderweise eine starke und spezifische blockierende (schließende) Wirkung auf einen K⁺-Kanal, der durch cyclisches Adenosinmonophosphat (cAMP) geöffnet wird und sich grundlegend vom erwähnten K⁺(ATP)-Kanal unterscheidet. Neuere Untersuchungen zeigen, daß dieser an Dickdarmgewebe identifizierte K⁺(cAMP) -Kanal sehr ähnlich, vielleicht sogar identisch, mit dem am Herzmuskel identifizierten I_{Ks}-Kanal ist. In der Tat konnte für die erfindungsgemäßen Verbindungen der Formel I eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit.

Neben den oben genannten Cromakalim- bzw. Acylaminochroman-Derivaten werden in der Literatur auch Verbindungen mit 4-Sulfonylaminochroman-Struktur beschrieben, die sich aber sowohl in der Struktur als auch in der biologischen Wirkung deutlich von den erfindungsgemäßen Verbindungen der Formel I unterscheiden. So werden in der EP-A-315 009 Chromanderivate mit 4-Phenylsulfonylaminostruktur beschrieben, die sich durch antithrombotische und antiallergische Eigenschaften auszeichnen. In der EP-A-389 861 und der JP 01294677 werden 3-Hydroxychroman- bzw. Chromen-Derivate mit einer cyclischen 4-Sulfonylaminogruppe beschrieben (z.B. Verbindung B), die über eine Aktivierung des K⁺(ATP)-Kanals als Antihypertensiva wirken sollen. In der EP-A-370 901 werden 3-Hydroxychroman- bzw. Chromen-Derivate mit einer 4-Sulfonylaminogruppe, wobei die restliche Valenz des N-Atoms ein Wasserstoffatom trägt, beschrieben, die über ZNS-Wirkungen verfügen. Weitere 4-Sulfonylaminochroman-Derivate werden in Bioorg. Med. Chem. Lett. 4 (1994), 769 - 773: "N-sulfonamides of benzopyran-related potassium channel openers: conversion of glyburyde insensitive smooth muscle relaxants to potent smooth muscle contractors" sowie in FEBS Letters 396 (1996), 271-275: "Specific blockade of slowly activating I_{sK} channels by chromanols ..." und Pflügers Arch. - Eur. J. Physiol. 429 (1995), 517-530: "A new class of inhibitors of cAMP-mediated Cl- secretion in rabbit colon, acting by the reduction of cAMP-activated K⁺ conductance" beschrieben.

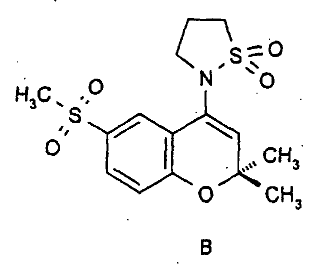

Die vorliegende Erfindung betrifft Verbindungen der Formel I, 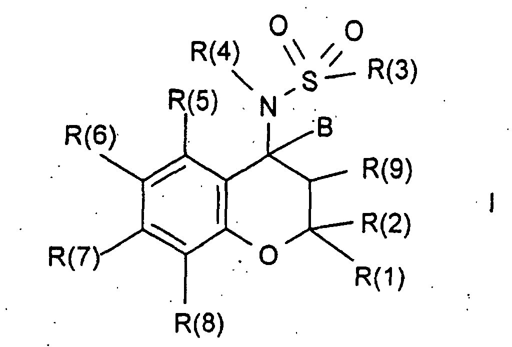 worin bedeuten:
- R(1) und R(2): unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
- R(1) und R(2): gemeinsam eine Alkylenkette mit 2, 3, 4, 5, 6, 7, 8, 9 oder 10 C-Atomen;
- R(3): R(10)-CₙH₂ₙ-NR(11)- oder R(10)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-;
R(12a) Wasserstoff, Methyl oder Ethyl;
R(10) Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen; oder
R(10) und R(11) gemeinsam eine Bindung, sofern n nicht kleiner als 3 ist;
- R(4): R(13)-CᵣH₂ᵣ-Z-C_{q}H_{2q}-;
q 0, 1, 2, 3, 4, 5, 6, 7 oder 8;
r 0, 1, 2, 3, 4, 5, 6, 7 oder 8;
Z -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O-oder
-CO-O-CₓH₂ₓ-NR(14)-,
wobei jeweils beide Verknüpfungsrichtungen möglich sind;
x 2, 3 oder 4;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, C₃F₇, Cycloalkyl mit 3, 4, 5, 6, 7 oder 8 C-Atomen, -NR(15)R(16), -CONR(15)R(16), -C(=NR(17))NR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(15) und R(16) unabhängig voneinander Wasserstoff, Alkyl mit 1, 2, 3 oder 4 C-Atomen oder -C_{z}H_{2z}-phenyl,
worin Phenyl unsubstituiert ist oder substituiert mit 1 or 2 Substituenten ausgewählt aus der Gruppe bestehend aus F, Cl, Br, CF₃, NO₂, CN OH, Methyl, Ethyl, Methoxy, Dimethylamino, Sulfamoyl, Methylsulfonyl, Aminosulfonyl und Methylsulfonylamino; oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen;
- R(5), R(6), R(7) und R(8): unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO2-, -SO₂-O-, -SO₂NR(10c), -NR(10c)- oder -CONR(10c)-;
R(10c) Wasserstoff oder Alkyl mit 1, 2 oder 3 C-Atomen;
s Null, 1, 2, 3, 4, 5 oder 6;
R(18) Wasserstoff, CF₃, C₂F₅, C₃F₇, Cycloalkyl mit 3, 4, 5, 6, 7 oder 8 C-Atomen, -COOR(21), 1-Piperidyl, 1-Pyrrolidinyl, 4-Morpholinyl, 4-Methylpiperazin-1-yl, Pyridyl, Thienyl, Imidazolyl, Chinolyl, Isochinolyl oder Phenyl,
R(21) Wasserstoff oder Alkyl mit 1, 2 oder 3 C-Atomen;
- R(9): Wasserstoff, OR(10d) oder OCOR(10d);
R(10d) Wasserstoff oder Alkyl mit 1, 2 oder 3 C-Atomen;
- B: Wasserstoff;
oder
- R(9) und B: gemeinsam eine Bindung;
sowie ihre physiologisch verträglichen Salze.

Bevorzugt sind Verbindungen der Formel I, in welcher bedeuten:
- R(1) und R(2): unabhängig voneinander Wasserstoff, CF₃ oder Alkyl mit 1, 2, 3, 4, 5 oder 6 C-Atomen;
oder
- R(1) und R(2): gemeinsam eine Alkylenkette mit 2, 3, 4, 5 oder 6 C-Atomen;
- R(3): R(10)-CₙH₂ₙ-;
R(10) Methyl, CF₃ oder C₂F₅;
n Null, 1 oder 2;
- R(4): R(13)-CᵣH₂ᵣ-Z-C_{q}H_{2q}-;
q 1, 2, 3, 4, 5, 6, 7 oder 8;
r 0, 1, 2, 3, 4, 5, 6, 7 oder 8;
Z -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- oder
-CO-O-CₓH₂ₓ-NR(14)-,
wobei jeweils beide Verknüpfungsrichtungen möglich sind;
x 2, 3 oder 4;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅; C₃F₇, Cycloalkyl mit 3, 4, 5, 6, 7 oder 8 C-Atomen, -NR(15)R(16), -CONR(15)R(16), -C(=NR(17))NR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(15) und R(16) unabhängig voneinander Wasserstoff, Alkyl mit 1, 2, 3 oder 4 C-Atomen oder -C_{z}H_{2z}-phenyl,
worin Phenyl unsubstituiert ist oder substituiert mit 1 or 2 Substituenten ausgewählt aus der Gruppe bestehend aus F, Cl, Br, CF₃, NO₂, CN OH, Methyl, Ethyl, Methoxy,
Dimethylamino, Sulfamoyl, Methylsulfonyl, Aminosulfonyl und Methylsulfonylamino; oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff oder Alkyl mit 1, 2 oder 3 C-Atomen; oder
- R(5), R(6), R(7) und R(8): unabhängig voneinander Wasserstoff, F, Cl, Br, I, Alkyl mit 1, 2, 3, 4 oder 5 C-Atomen, Cycloalkyl mit 3, 4, 5, 6 oder 7 C-Atomen, -CN, -CF₃, -C₂F₅, -C₃F₇, -NO₂, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -SO₂-O-, -SO₂NR(10c), -NR(10c)-oder-CONR(10c)-;
R(10c) Wasserstoff oder Alkyl mit 1, 2 oder 3 C-Atomen;
s Null, 1, 2, 3, 4, 5 oder 6;
R(18) Wasserstoff, CF₃, C₂F₅, C₃F₇, Cycloalkyl mit 3, 4, 5, 6 oder 7 C-Atomen, -COOR(21), 1-Piperidyl, 1-Pyrrolidinyl, 4-Morpholinyl, 4-Methylpiperazin-1-yl, Pyridyl, Imidazolyl oder Phenyl,
R(21) Wasserstoff oder Alkyl mit 1, 2 oder 3 C-Atomen;
- R(9): Wasserstoff oder OR(10d);
R(10d) Wasserstoff oder Methyl;
- B: Wasserstoff;
oder
- R(9) und B: gemeinsam eine Bindung;
sowie ihre physiologisch verträglichen Salze.

Besonders bevorzugt sind Verbindungen der Formel I, in der bedeuten:
- R(1) und R(2): unabhängig voneinander Wasserstoff, CF₃ oder Alkyl mit 1, 2 oder 3 C-Atomen;
oder
- R(1) und R(2): gemeinsam eine Alkylenkette mit 2, 3, 4 oder 5 C-Atomen;
- R(3): R(10)-CₙH₂ₙ-;
R(10) Methyl, CF₃ oder C₂F₅;
n Null, 1 oder 2;
- R(4): R(13)-CᵣH₂ᵣ-Z-C_{q}H_{2q}-;
q 1, 2, 3 oder 4;
r 0, 1, 2 oder 3;
Z -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O-oder
-CO-O-CₓH₂ₓ-NR(14)-,
wobei jeweils beide Verknüpfungsrichtungen möglich sind;
x 2 oder 3;
R(14) Wasserstoff, Alkyl mit 1 oder 2 C-Atomen;
R(13) CH₃, CF₃, C₂F₅, Cycloalkyl mit 3, 4, 5, 6 oder 7 C-Atomen, -NR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(15) und R(16) unabhängig voneinander Wasserstoff, Alkyl mit 1, 2, 3 oder 4 C-Atomen oder -C_{z}H_{2z}-phenyl,
worin Phenyl unsubstituiert ist oder substituiert mit 1 or 2 Substituenten ausgewählt aus der Gruppe bestehend aus F, Cl, Br, CF₃, NO₂, CN OH, Methyl, Ethyl, Methoxy, Dimethylamino, Sulfamoyl, Methylsulfonyl, Aminosulfonyl und Methylsulfonylamino; oder
R(15) und R(16) gemeinsam eine Kette von 4 oder 5 Methylengruppen, von denen eine CH₂-Gruppe durch -O-,-S-,-NH-, -N(CH₃)-oder -N(Benzyl)-ersetzt sein kann;
R(17) Wasserstoff oder Alkyl mit 1 oder 2 C-Atomen;
- R(5) und R(6): unabhängig voneinander Wasserstoff, F, Cl, Br, Alkyl mit 1, 2, 3, 4 oder 5 C-Atomen, Cycloalkyl mit 3, 4, 5, 6 oder 7 C-Atomen, -CN, -CF₃, -C₂F₅, -NO₂, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -SO₂- oder -CONR(10c)-;
R(10c) Wasserstoff oder Alkyl mit 1 oder 2 C-Atomen;
s Null, 1, 2, 3, 4, 5 oder 6;
R(18) Wasserstoff, CF₃, C₂F₅, Cycloalkyl mit 3, 4, 5, 6 oder 7 C-Atomen, 1-Piperidyl, 1-Pyrrolidinyl, 4-Morpholinyl, 4-Methylpiperazin-1-yl, Pyridyl, Imidazolyl oder Phenyl,
- R(7) und R(8): Wasserstoff;
- R(9): Wasserstoff oder OR(10d);
R(10d) Wasserstoff oder Methyl;
- B: Wasserstoff;
oder
- R(9) und B: gemeinsam eine Bindung;
sowie ihre physiologisch verträglichen Salze.

Ganz besonders bevorzugt sind Verbindungen der Formel I, in der bedeuten:
- R(1) und R(2): Methyl;
- R(3): Methyl oder Ethyl;
- R(4): R(13)-CᵣH₂ᵣ-Z-C_{q}H_{2q}-;
q 1, 2, 3 oder 4;
r 0, 1, 2 oder 3;
Z -CO-NR(14)-,
-OCO-NR(14)-,
-O-CₓH₂ₓ-NR(14)- oder
-CO-O-CₓH₂ₓ-NR(14)-;
x 2 oder 3;
R(14) Wasserstoff oder Methyl;
R(13) CH₃, CF₃, -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(17) Wasserstoff oder Alkyl mit 1 oder 2 C-Atomen;
- R(5): Wasserstoff, Alkyl mit 1, 2, 3 oder 4 C-Atomen, F, Cl, Methoxy oder Ethoxy;
- R(6): F, Cl, Alkyl mit 1, 2, 3, 4 oder 5 C-Atomen, -CF₃, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y -O-, -CO- oder -CONR(10c)-;
R(10c) Wasserstoff oder Methyl;
s 1, 2, 3, 4 oder 5;
R(18) Wasserstoff, CF₃ oder Phenyl,
- R(7) und R(8): Wasserstoff;
- R(9): Wasserstoff;
- B: Wasserstoff;
sowie ihre physiologisch verträglichen Salze.

Ganz besonders bevorzugt sind auch Verbindungen der Formel I, in der bedeuten:
- R(1) und R(2): Methyl;
- R(3): Methyl oder Ethyl;
- R(4): R(13)-CᵣH₂ᵣ-Z-C_{q}H_{2q}-;
q 1, 2, 3 oder 4;
r 0, 1, 2 oder 3;
Z -CO-NR(14)-,
-OCO-NR(14)-,
-O-CₓH₂ₓ-NR(14)- oder
-CO-O-CₓH₂ₓ-NR(14)-;
x 2 oder 3;
R(14) Wasserstoff oder Methyl;
R(13) CH₃, CF₃, -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(17) Wasserstoff, Alkyl mit 1 oder 2 C-Atomen;
- R(5): Wasserstoff, Alkyl mit 1, 2, 3 oder 4 C-Atomen, F, Cl, Methoxy oder Ethoxy;
- R(6): F, Cl, Alkyl mit 1, 2, 3, 4 oder 5 C-Atomen, -CF₃, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y -O-, -CO- oder -CONR(10c)-;
R(10c) Wasserstoff oder Methyl;
s 1, 2, 3, 4 oder 5;
R(18) Wasserstoff, CF₃ oder Phenyl,
- R(7) und R(8): Wasserstoff;
- R(9): OH;
- B: Wasserstoff;
sowie ihre physiologisch verträglichen Salze.

Ganz besonders bevorzugt sind weiterhin Verbindungen der Formel I, in der bedeuten:
- R(1) und R(2): Methyl;
- R(3): Methyl oder Ethyl;
- R(4): R(13)-CᵣH₂ᵣ-Z-C_{q}H_{2q}-;
q 1, 2, 3 oder 4;
r 0, 1, 2 oder 3;
Z -CO-NR(14)-,
-OCO-NR(14)-,
-O-CₓH₂ₓ-NR(14)- oder
-CO-O-CₓH₂ₓ-NR(14)-;
x 2 oder 3;
R(14) Wasserstoff oder Methyl;
R(13) CH₃, CF₃, -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl, Aminosulfonyl und Methylsulfonylamino,
R(17) Wasserstoff oder Alkyl mit 1 oder 2 C-Atomen;
- R(5): Wasserstoff, Alkyl mit 1, 2, 3 oder 4 C-Atomen, F, Cl, Methoxy oder Ethoxy;
- R(6): F, Cl, Alkyl mit 1, 2, 3, 4 oder 5 C-Atomen, -CF₃, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y - O-, -CO- oder -CONR(10c)-;
R(10c) Wasserstoff oder Methyl;
s 1, 2, 3, 4 oder 5;
R(18) Wasserstoff, CF₃ oder Phenyl,
- R(7) und R(8): Wasserstoff;
- R(9) und B: gemeinsam eine Bindung;
sowie ihre physiologisch verträglichen Salze.

Alkylreste und Alkylenreste können geradkettig oder verzweigt sein. Dies gilt auch für die Alkylenreste der Formeln CᵣH₂ᵣ, C_{q}H_{2q}, CₙH₂ₙ und CₛH₂ₛ. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste.

Als N-haltige Heterocyclen mit 1, 2, 3, 4, 5, 6, 7, 8 oder 9 C-Atomen gelten insbesondere die aromatischen Systeme 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder - 5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl.

Besonders bevorzugt sind die N-haltigen Heterocyclen Pyrrolyl, Imidazolyl, Chinolyl, Pyrazolyl, Pyridyl, Pyrazinyl, Pyrimidinyl und Pyridazinyl.

Thienyl steht sowohl für 2- als auch 3-Thienyl.

Monosubstituierte Phenylreste können in der 2-, der 3- oder der 4-Position substituiert sein, disubstituierte in der 2,3-, 2,4-, 2,5-, 2,6-, 3,4- oder 3,5-Position. Entsprechendes gilt sinngemäß analog auch für die N-haltigen Heterocylen oder den Thiophenrest.

Bei Disubstitution eines Restes können die Substituenten gleich oder verschieden sein.

Bedeuten die Reste R(1) und R(2) gemeinsam eine Alkylenkette, so bilden diese Reste mit dem sie tragenden Kohlenstoffatom einen Ring, der mit dem 6-Ring in der Formel I ein Kohlenstoffatom gemeinsam hat, es liegt dann also eine Spiroverbindung vor. Bedeuten R(9) und B gemeinsam eine Bindung so liegt ein 2H-Chromengrundgerüst vor. Bedeuten R(10) und R(11) gemeinsam eine Bindung, so steht die Gruppe R(10)-CₙH₂ₙ-NR(11)- bevorzugt für einen überein Stickstoffatom gebundenen Stickstoffheterocyclus. Wenn R(10) und R(11) gemeinsam eine Bindung bedeuten und die Gruppe R(10)-CₙH₂ₙ-NR(11)- für einen über ein Stickstoffatom gebundenen Stickstoffheterocyclus steht, ist dieser Stickstoffheterocyclus bevorzugt ein 4-Ring oder ein größerer Ring als ein 4-Ring, z. B. ein 5-Ring, 6-Ring oder 7-Ring.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit.Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. sind also in enantiomerenreiner Form, sowohl als links- als auch als rechtsdrehende Antipoden, und auch in Form von Mischungen der beiden Enantiomeren in unterschiedlichen Verhältnissen oder in Form von Racematen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Verbindungen der Formel I sind durch unterschiedliche chemische Verfahren herstellbar, die ebenfalls zu der vorliegenden Erfindung gehören. So erhält man beispielsweise eine Verbindung der Formel I, indem man
a) eine Verbindung der Formel II, 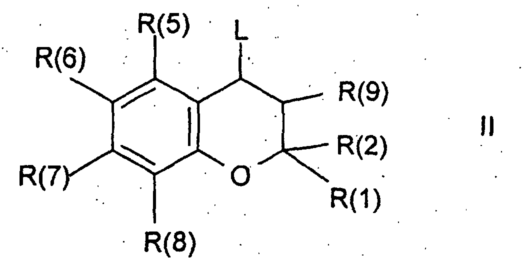 worin R(1), R(2), R(5), R(6), R(7), R(8) und R(9) die oben angegebenen Bedeutungen besitzen und L eine nucleofuge Fluchtgruppe, insbesondere Cl, Br, I, Methansulfonyloxy oder p-Toluolsulfonyloxy, bedeutet, in an sich bekannter Weise umsetzt mit einem Sulfonamid oder dessen Salz der Formel III, 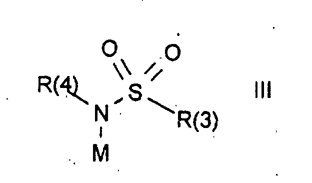 worin R(3) und R(4) die oben angegebenen Bedeutungen besitzen und M für Wasserstoff oder vorzugsweise für ein Metalläquivalent, besonders bevorzugt für Lithium, Natrium oder Kalium steht;
   oder daß man
b) eine Verbindung der Formel IV 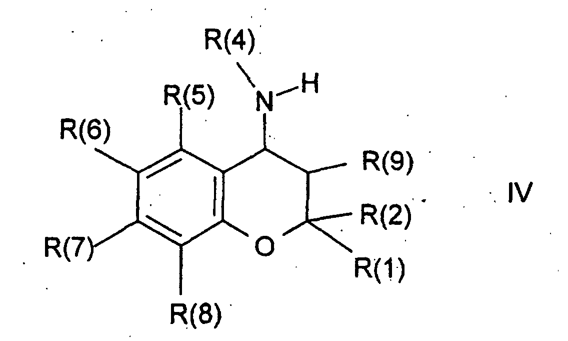 worin R(1), R(2), R(4), R(5), R(6), R(7), R(8) und R(9) die oben angegebenen Bedeutungen besitzen, mit einem Sulfonsäure-Derivat der Formel V 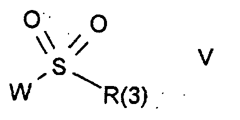 umsetzt, worin R(3) die oben angegebenen Bedeutungen besitzt und W eine nucleofuge Fluchtgruppe, wie z. B. Fluor, Brom, 1-Imidazolyl, insbesondere aber Chlor, bedeutet;
   oder daß man
c) eine Verbindung der Formel VI 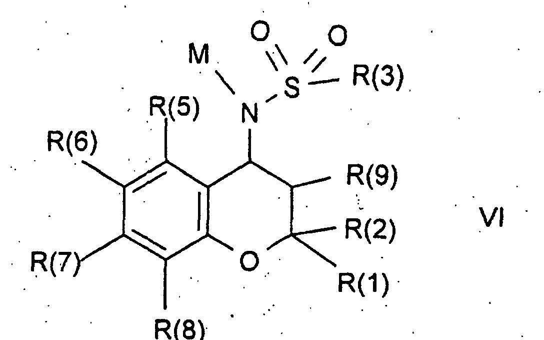 worin R(1), R(2), R(3), R(5), R(6), R(7), R(8), R(9) und M die oben angegebenen Bedeutungen besitzen, in an sich bekannter Weise im Sinne einer Alkylierungsreaktion mit einem Alkylierungsmittel der Formel VII umsetzt,

   R(4)-L VII

   worin R(4) und L die oben angegebenen Bedeutungen besitzen;
   oder daß man
d) in einer Verbindung der Formel I 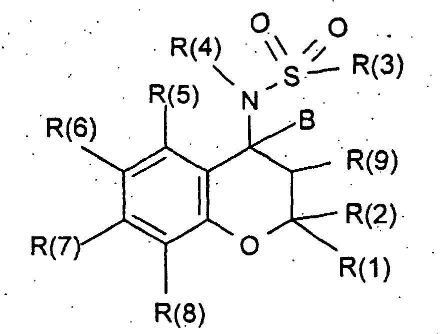 worin R(1) bis R(9) und B die oben angegebenen Bedeutungen besitzen, in mindestens einer der Positionen R(5), R(6), R(7) und R(8) eine elektrophile Substitutionsreaktion durchführt, sofern diese Position Wasserstoff bedeutet;
   oder daß man
e) eine Verbindung der Formel VIII 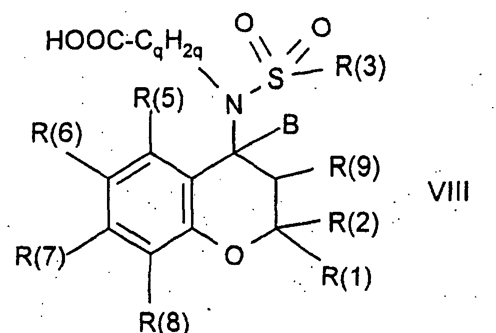 worin R(1), R(2), R(3), R(5), R(6), R(7), R(8), R(9), q und B die oben angegebenen Bedeutungen besitzen, mit einer Verbindung der Formel IX, X oder XI, 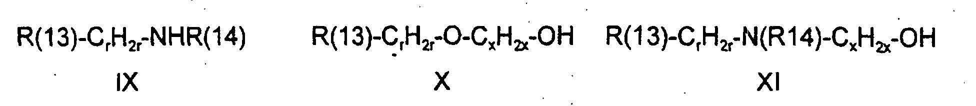 worin R(13), R(14), r und x die oben angegebenen Bedeutungen besitzen, umsetzt im Sinne einer Veresterungs- bzw. Amidierungsreaktion;
   oder daß man
f) eine Verbindung der Formel XII 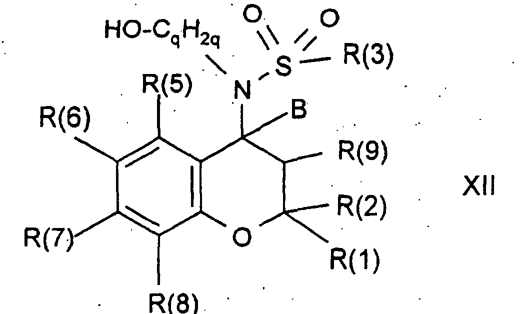 worin R(1), R(2), R(3), R(5), R(6), R(7), R(8), R(9), q und B die oben angegebenen Bedeutungen besitzen, mit einer Verbindung der Formel XIII oder XIV 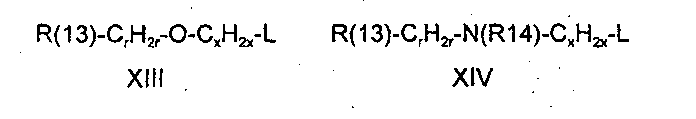 worin R(13), R(14), r, x und L die oben angegebenen Bedeutungen besitzen, umsetzt im Sinne einer Alkylierungsreaktion;
   oder daß man
g) eine Verbindung der Formel XV, 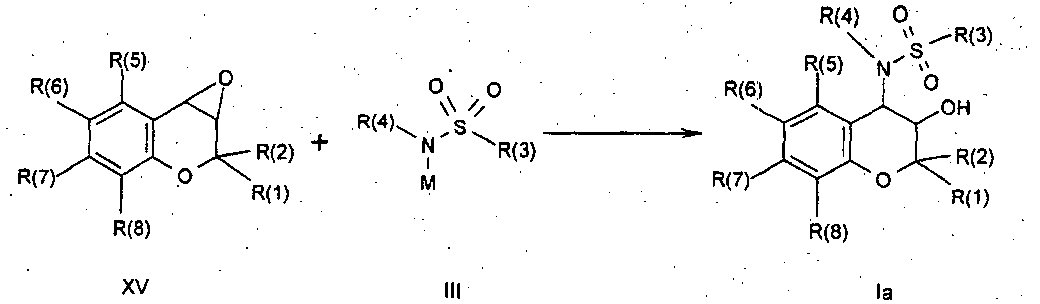 worin R(1), R(2), R(5), R(6), R(7) und R(8) die oben angegebenen Bedeutungen besitzen, mit einem Sulfonamid der Formel III, worin R(3), R(4) und M die oben angegebenen Bedeutungen haben oder M vorteilhaft auch für einen Trialkylsilylrest, z.B. einen Trimethylsilylrest, steht, umsetzt zu einem Chromanol der Formel Ia;
   oder daß man
h) eine Verbindung der Formel Ia, 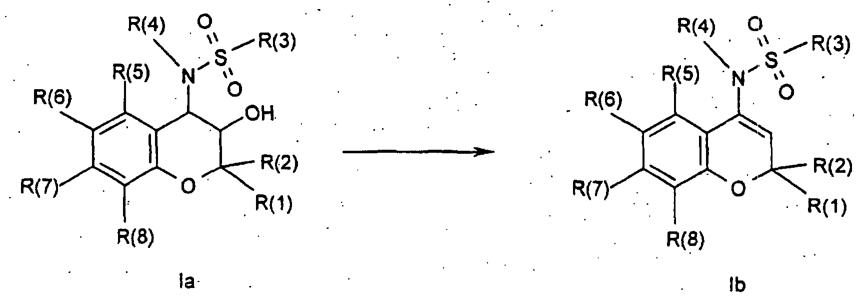 worin R(1) bis R(8) die oben angegebenen Bedeutungen haben, im Sinne einer Eliminierungsreaktion zu einer Verbindung der Formel Ib, worin R(1) bis R(8) die oben angegebenen Bedeutungen haben, umwandelt.

Die Verfahrensweise a) entspricht der nucleophilen Substitution einer Abgangsgruppe in einem reaktiven Bicyclus der Formel II durch ein Sulfonamid bzw. eines seiner Salze der Formel III. Wegen der höheren Nucleophilie und höheren Reaktivität eines in der Salzform vorliegenden Sulfonamids ist bei Verwendung eines freien Sulfonamids (Formel III, M = H) bevorzugt, aus diesem zunächst durch Einwirkung einer Base ein Sulfonamidsalz (Formel III, M = Metallkation) zu .erzeugen. Setzt man ein freies Sulfonamid (Formel III, M = H) ein, so kann die Deprotonierung des Sulfonamids zum Salz in situ erfolgen. Bevorzugt werden solche Basen verwandt, die selbst nicht oder nur wenig alkyliert werden, wie z. B. Natriumcarbonat, Kaliumcarbonat, sterisch stark gehinderte Amine, z. B. Dicyclohexylamin, N,N-Dicyclohexyl-ethylamin, oder andere starke Stickstoffbasen mit geringer Nucleophilie, beispielsweise DBU (Diazabicycloundecen), N,N',N"'-Triisopropylguanidin etc. Es können allerdings auch andere üblich verwendete Basen für die Reaktion eingesetzt werden, wie Kalium-tert-butylat, Natriummethylat, Alkalihydrogencarbonate, Alkalihydroxide, wie beispielsweise LiOH, NaOH oder KOH, oder Erdalkalihydroxide, wie beispielsweise Ca(OH)₂.

Vorzugsweise arbeitet man in einem Lösungsmittel, besonders bevorzugt in. polaren organischen Lösungsmitteln wie z. B. Dimethylformamid (DMF), Dimethylacetamid (DMA), Dimethylsulfoxid (DMSO), Tetramethylhamstoff (TMU), Hexamethylphosphorsäuretriamid (HMPT), Tetrahydrofuran (THF), Dimethoxyethan (DME) oder anderen Ethem, oder beispielsweise auch.in einem Kohlenwasserstoff wie Toluol oder in einem halogenierten Kohlenwasserstoff wie Chloroform oder Methylenchlorid usw. Es kann aber auch in polaren protischen Lösungsmitteln gearbeitet werden, wie z. B. in Wasser, Methanol, Ethanol, Isopropanol, Ethylenglykol oder dessen Oligomeren und deren entsprechenden Halbethern oder auch deren Ethern. Die Reaktion kann auch in Gemischen dieser Lösungsmittel durchgeführt werden. Ebenso kann die Reaktion aber auch ganz ohne Lösungsmittel durchgeführt werden. Die Reaktion wird bevorzugt in einem Temperaturbereich von -10 bis +140 °C, besonders bevorzugt im Bereich von 20 bis 100 °C durchgeführt. In günstiger Weise kann Verfahrensweise a) auch unter den Bedingungen einer Phasentransferkatalyse durchgeführt werden.

Die Verbindungen der Formel II gewinnt man nach literaturbekannten Methoden, beispielsweise aus den entsprechenden Alkoholen (Formel II, L = -OH) durch Einwirkung von Halogenwasserstoff HL (L = Cl, Br, I) oder durch Einwirkung eines anorganischen Säurehalogenids (POCl₃, PCl₃, PCl₅, SOCl₂, SOBr₂) oder durch radikalische Halogenierung der entsprechenden Chroman-Derivate (Formel II, L = H) mit elementarem Chlor oder Brom, oder mit radikalisch aktivierbaren Halogenierungsmitteln wie N-Bromsuccinimid (NBS) oder SO₂Cl₂ (Sulfurylchlorid) in Gegenwart eines Radikalkettenstarters wie energiereichem Licht des sichtbaren oder ultravioletten Wellenbereiches oder durch Verwendung eines chemischen Radikalstarters wie Azodiisobutyronitril.

Verfahrensweise b)
beschreibt die an sich bekannte und häufig angewandte Reaktion einer reaktiven Sulfonylverbindung der Formel V, insbesondere einer Chlorsulfonylverbindung (W = Cl), mit einem Amino-Derivat der Formel IV zum entsprechendenSulfonamid-Derivat der Formel I. Die Reaktion kann prinzipiell ohne Lösungsmittel durchgeführt werden, jedoch werden derartige Reaktionen in den meisten Fällen unter Verwendung eines Lösungsmittels durchgeführt.

Die Reaktionsführung geschieht vorzugsweise unter Verwendung eines polaren Lösungsmittels vorzugsweise in Gegenwart einer Base, die selbst vorteilhaft als Lösungsmittel verwendet werden kann, z.B. bei Verwendung von Triethylamin, insbesondere von Pyridin und dessen Homologen. Ebenfalls verwendete Lösungsmittel sind beispielsweise Wasser, aliphatische Alkohole, z.B. Methanol, Ethanol, Isopropanol, sek. Butanol, Ethylenglykol und dessen monomere und oligomere Monoalkyl- und Dialkylether, Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C.

Die Amine der Formel IV erhält man in literaturbekannter Weise bevorzugt aus den entsprechenden Carbonylverbindungen der Formel XVI, 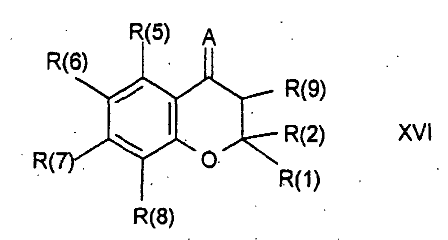 worin R(1), R(2), R(5), R(6), R(7), R(8) und R(9) die oben angegebenen Bedeutungen besitzen und A Sauerstoff bedeutet, entweder mit Ammoniak oder einem Amin der Formel XVII,

R(4)-NH₂ XVII

in der R(4) die angegebenen Bedeutungen hat, unter reduktiven Bedingungen oder reduktiven katalytischen Bedingungen, vorzugsweise bei höherer Temperatur und im Autoklaven. Dabei werden primär durch Kondensationsreaktion der Ketone der Formel XVI (A = Sauerstoff) und der Amine der Formel XVII in situ Schiff-Basen der Formel XVI, in der A für R(4)-N= steht, gebildet, die unmittelbar, d. h. ohne vorherige Isolierung, reduktiv in die Amine der Formel IV überführt werden können. Es können aber auch die bei der Kondensationsreaktion intermediär aus den Verbindungen der Formel XVI und XVII entstehenden Schiff-Basen (Formel XVI, A steht für R(4)-N=) nach literaturbekannten Methoden hergestellt und zunächst isoliert werden, um sie dann in einem separaten Schritt mit einem geeigneten Reduktionsmittel, wie z. B. NaBH₄, LiAlH₄, NaBH₃CN oder durch katalytische Hydrierung in Gegenwart von z. B. Raney-Nickel oder einem Edelmetall wie z. B. Palladium, in die Verbindungen der Formel IV umzuwandeln.

Die Verbindungen der Formel IV, in der R(4) für Wasserstoff steht, können vorteilhaft in literaturbekannter Weise auch durch Reduktion von Oximen oder Oximethern (Formel XVI, A steht für =N-OR, R = H oder Alkyl) oder Hydrazonen (Formel XVI, A steht für =N-NR₂, R z. B. = H oder Alkyl) erhalten werden, z. B. unter Verwendung eines komplexen Metallhydrids oder durch katalytische Hydrierung. Die dafür erforderlichen Oxime und Hydrazone werden vorzugsweise in an sich bekannter Weise aus den Ketonen der Formel XVI (A = Sauerstoff) mit Hydrazin oder einem seiner Derivate oder beispielsweise mit Hydroxylamin-Hydrochlorid unter wasserabspaltenden Bedingungen hergestellt. Besonders vorteilhaft können die Verbindungen der Formel IV, in der R(4) für Wasserstoff steht, auch durch Aminierung mit einer geeigneten Ammoniumverbindung, z.B. Ammoniumacetat, in Gegenwart eines geeigneten Reduktionsmittels, wie z.B. NaCNBH₃, erhalten werden (J. Am. Chem. Soc. 93, 1971, 2897).

Alternativ können die Amino-Derivate der Formel IV auch in an sich literaturbekannter Weise durch Reaktion der reaktiven Verbindungen der Formel II mit R(1), R(2), R(5), R(6), R(7), R(8), R(9) und L in der angegebenen Bedeutung, entweder mit Ammoniak oder einem Amin der Formel XVII, mit R(4) in der angegebenen Bedeutung, erhalten werden.

Verfahrensweise c)
repräsentiert die an sich bekannte Alkylierungsreaktion eines Sulfonamids bzw. eines seiner Salze VI mit einem Alkylierungsmittel der Formel VII. Entsprechend der Reaktionsanalogie mit Verfahrensweise a) gelten für Verfahrensweise c) die bereits ausführlich unter Verfahrensweise a) beschriebenen Reaktionsbedingungen. Neben den dort bereits genannten Basen werden vorzugsweise Natriumhydrid oder eine Phosphazenbase zur Deprotonierung des Sulfonamids eingesetzt.

Die Herstellung der Sulfonamid-Derivate VI (mit M=H) und deren Vorprodukte wurden bereits bei Verfahrensweise b) beschrieben, wobei R(4) dann jeweils Wasserstoff bedeutet. Die Herstellung der Alkylantien VII erfolgt nach Analogvorschriften der Literatur bzw. wie unter Verfahrensweise a) beschrieben, vorzugsweise aus den entsprechenden Hydroxyverbindungen (Formel VII mit L gleich -OH).

Verfahrensweise d)
beschreibt die weitere chemische Umwandlung von erfindungsgemäßen Verbindungen der Formel I in andere Verbindungen der Formel I durch elektrophile Substitutionsreaktionen in einer oder in mehreren der mit R(5) bis R(8) bezeichneten Positionen, die jeweils Wasserstoff bedeuten.

Bevorzugte Substitutionsreaktionen sind
1. die aromatische Nitrierung zur Einführung einer oder mehrerer Nitrogruppen, die in nachfolgenden Reaktionen teilweise oder alle zu Aminogruppen reduziert werden können. Die Aminogruppen können wiederum in nachfolgenden Reaktionen in andere Gruppen umgewandelt werden, beispielsweise in einer Sandmeyerreaktion, z. B. zur Einführung von Cyanogruppen;
2. die aromatische Halogenierung insbesondere zur Einführung von Chlor, Brom oder Jod;
3. die Chlorsulfonierung, z.B. durch Einwirkung von Chlorsulfonsäure, zur Einführung einer Chlorsulfonylgruppe, die in nachfolgenden Reaktionen in andere Gruppen umgewandelt werden kann, z. B. in eine Sulfonamidgruppe;
4. die Friedel-Crafts-Acylierungsreaktion zur Einführung eines Acylrestes oder eines Sulfonylrestes durch Einwirkung der entsprechenden Säurechloride in Gegenwart einer Lewis-Säure als Friedel-Crafts-Katalysator, vorzugsweise in Gegenwart von wasserfreiem Aluminiumchlorid.

Verfahrensweise e)
beschreibt die Veresterung von Carbonsäuren der Formel VIII mit Alkoholen der Formeln X oder XI bzw. die Amidierung mit Aminen der Formel IX. Für diese Reaktionen sind in der Literatur zahlreiche Methoden beschrieben worden. Besonders vorteilhaft können diese Reaktionen durch Aktivierung der Carbonsäure, z.B. mit Dicyclohexylcarbodiimid (DCC), gegebenenfalls unter Zusatz von Hydroxybenzotriazol (HOBT) oder Dimethylaminopyridin (DMAP), oder mit O-[(Cyano(ethoxycarbonyl)methylen)amino]-1,1,3,3-tetramethyluroniumtetrafluoroborat (TOTU), durchgeführt werden. Es können aber auch zunächst nach bekannten Methoden reaktive Säurederivate synthetisiert werden, z.B. Säurechloride durch Umsetzung der Carbonsäuren der Formel VIII mit anorganischen Säurehalögeniden, wie z.B. SOCl₂, oder Säureimidazolide durch Umsetzung mit Carbonyldiimidazol, die dann anschließend, gegebenenfalls unter Zusatz einer Hilfsbase, mit den Alkoholen bzw. Aminen der Formeln IX, X oder XI umgesetzt werden.

Die Carbonsäuren der Formel VIII erhält man nach den unter a) bis d) beschriebenen Methoden, wobei dann jedoch R(4) jeweils -C_{q}H_{2q}COOH bzw. -C_{q}H_{2q}COOAlkyl bedeutet und im letzteren Fall noch eine anschließende Verseifung des Esters erfolgt.

Verfahrensweise f)
beschreibt die Alkylierung eines Alkohols der Formel XII mit einem Alkylierungsmittel der Formel XIII oder XIV. Hierzu wird der Alkohol zunächst durch Einwirkung einer geeigneten Base, wie z.B. Natriumhydrid oder eine Phosphazenbase in ein Alkoholatsalz überführt, das dann in einem geeigneten polaren Lösungsmittel, wie z.B. Dimethylformamid, bei Temperaturen zwischen 20 und 150°C mit dem Alkylierungsmittel umgesetzt wird. Die Deprotonierung des Alkohols zum Salz kann auch in situ erfolgen, wobei dann bevorzugt Basen eingesetzt werden, die selbst nicht alkyliert werden, wie z.B. Kaliumcarbonat.
Die Alkohole der Formel XII erhält man nach den unter a) bis d) beschriebenen Methoden, wobei dann jedoch R(4) jeweils -C_{q}H_{2q}OH bzw. -C_{q}H_{2q}OR (R=geeignete Schutzgruppe, z.B. Acetoxy) bedeutet und im letzteren Fall noch eine anschließende Abspaltung der Schutzgruppe erfolgt.

Verfahrensweise g)
entspricht der nucleophilen Öffnung eines Epoxids der Formel XV durch ein Sulfonamid bzw. eines seiner Salze der Formel III. Die Reaktion kann unter analogen Bedingungen wie für Verfahrensweise a) beschrieben durchgeführt werden. Als besonders vorteilhaft hat sich die Verwendung des freien Sulfonamids in Gegenwart eines Unterschusses, z.B. 20 - 80%, der entsprechenden Base, z.B. Natriumhydrid, erwiesen. Ebenfalls vorteilhaft ist die Verwendung von Sulfonamidderivaten, bei denen M für einen Trialkylsilyl-, z.B. einen Trimethylsilylrest, steht, wobei es dann zweckmäßig ist, die Reaktion in Gegenwart eines Fluorids, z.B. Tetrabutylammoniumfluorid, durchzuführen.

Die Epoxide der Formel XV erhält man nach literaturbekannten Methoden aus den entsprechenden Olefinen der Formel XVIII, 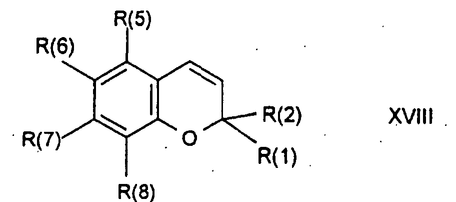 worin R(1), R(2), R(5), R(6), R(7) und R(8) die oben angegebenen Bedeutungen besitzen, z. B. durch Einwirkung eines geeigneten anorganischen oder organischen Peroxids, wie beispielsweise H₂O₂ oder m-Chlorperbenzoesäure, oder durch basenkatalysierte Cyclisierung des entsprechenden Bromhydrins, das aus XVIII z. B. durch Umsetzung mit N-Bromsuccinimid und Wasser erhalten werden kann. Die Epoxide der Formel XV können aus den Olefinen der Formel XVIII auch in optisch reiner Form erhalten werden durch Oxidation in Gegenwart des chiralen Jacobsen-Katalysators, wie z.B. in Tetrahedron Lett. 32, 1991, 5055 beschrieben ist. Die Olefine der Formel XVIII lassen sich erhalten entweder aus den Ketonen der Formel XVI (A=Sauerstoff) durch Reduktion der Carbonylgruppe zu einer OH-Funktion und anschließende säurekatalysierte Eliminierung oder durch thermische Cyclisierung von geeignet substituierten Aryl-propargylethern, wie z.B. in J. Org. Chem. 38 (1973) 3832 beschrieben.

Verfahrensweise h)
beschreibt die Überführung eines Chromanols der Formel Ia in ein Chromen der Formel Ib durch Eliminierung. Hierzu kann das Chromanol entweder direkt in Gegenwart einer Säure oder Base einer Wasserabspaltung unterworfen werden, oder es kann zunächst eine Aktivierung der Hydroxygruppe erfolgen, z.B. durch Acetylierung mit Acetanhydrid oder Mesylierung mit Methansulfonsäurechlorid, woraufhin anschließend eine basenkatalysierte Eliminierung erfolgen kann, z.B. durch Erhitzen mit DBU (Diazabicycloundecen).

Neben den beschriebenen Verfahrensweisen sind eine Reihe weiterer Zugänge zu den erfindungsgemäßen Verbindungen der Formel I denkbar. So kann es z.B. in einzelnen Fällen sinnvoll sein, die unter Verfahrensweisen a) bis h) beschriebenen Reaktionen in einer anderen Reihenfolge miteinander zu kombinieren oder analog zu den beschriebenen Methoden zunächst nicht erfindungsgemäße Verbindungen herzustellen, bei denen die Reste R(1) bis R(8) eine andere als die angegebene Bedeutung haben, und die dann in der letzten Stufe durch eine einfache Umwandlung an einem der Substituenten, wie z.B. Alkylierung, Arnidierung etc., in eine erfindungsgemäße Verbindung umgewandelt werden.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind. in der Literatur beschrieben und können gegebenenfalls ohne Schwierigkeiten dem Einzelfall angepaßt werden.

Es wurde bereits gesagt, daß die Verbindungen der Formel I überraschenderweise eine starke und spezifische blockierende (schließende) Wirkung auf einen K⁺-Kanal haben, der durch cyclisches Adenosinmonophosphat (cAMP) geöffnet wird und sich grundlegend vom wohlbekannten K⁺(ATP)-Kanal unterscheidet, und daß dieser an Dickdarmgewebe identifizierte K⁺(cAMP)-Kanal sehr ähnlich, vielleicht sogar identisch, mit dem am Herzmuskel identifizierten I_{Ks}-Kanal ist. Für die erfindungsgemäßen Verbindungen konnte eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit und eignen sich in hervorragender Weise als Arzneimittelwirkstoffe für die Therapie und Prophylaxe verschiedener Krankheitsbilder.

So zeichnen sich die erfindungsgemäßen Verbindungen der Formel I als neue Wirkstoffklasse potenter Inhibitoren der stimulierten Magensäuresekretion aus. Die Verbindungen der Formel I sind somit wertvolle Arzneimittelwirkstoffe zur Therapie und Prophylaxe von Ulcera des Magens und des intestinalen Bereiches, beispielsweise des Duodenums. Sie eignen sich ebenfalls infolge ihrer starken magensaftsekretionshemmenden Wirkung als ausgezeichnete Therapeutika zur Therapie und Prophylaxe der Refluxösophagitis.

Die erfindungsgemäßen Verbindungen der Formel I zeichnen sich weiterhin durch eine. antidiarrhoische Wirkung aus und Sind deshalb als Arzneimittelwirkstoffe zur Therapie und Prophylaxe von Durchfallerkrankungen geeignet.

Weiterhin eignen sich die erfindungsgemäßen Verbindungen der Formel I als Arzneimittelwirkstoffe zur Therapie und Prophylaxe von Herz-Kreislauferkrankungen. Insbesondere können sie zur Therapie und Prophylaxe aller Typen von Arrhythmien verwendet werden, einschließlich atrialer, ventrikulärer und supraventrikulärer Arrhythmien, vor allem von Herzrhythmusstörungen, die durch Aktionspotential-Verlängerung behoben werden können. Sie können speziell angewandt werden zur Therapie und Prophylaxe von atrialer Fibrillation (Vorhofflimmern) und atrialem Flattern (Vorhofflattern) sowie zur Therapie und Prophylaxe von Reentry-Arrhythmien und zur Verhinderung des plötzlichen Herztodes infolge von Kammerflimmern.

Obwohl bereits zahlreiche antiarrhythmisch wirkende Substanzen auf dem Markt sind, gibt es doch keine Verbindung, die hinsichtlich Wirksamkeit, Anwendungsbreite und Nebenwirkungsprofil wirklich zufriedenstellend ist, so daß weiterhin eine Notwendigkeit zur Entwicklung verbesserter Antiarrhythmika besteht. Die Wirkung zahlreicher bekannter Antiarrhythmika der sogenannten Klasse III beruht auf einer Erhöhung der myocardialen Refraktärzeit durch Verlängerung der Aktionspotentialdauer. Diese wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, von dem zwei Subtypen existieren, ein schnell aktivierter I_{Kr} und ein langsam aktivierter I_{Ks}. Die meisten bekannten Klasse III-Antiarrhythmika blockieren überwiegend oder ausschließlich I_{Kr} (z.B. Dofetilid, d-Sotalol). Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D.M. Roden; "Current Status of Class III Antiarrhythmic Drug Therapy"; Am. J. Cardiol. 72 (1993), 44B-49B). Bei höheren Herzfrequenzen bzw. Stimulation der β-Rezeptoren hingegen ist die das Aktionspotential verlängernde Wirkung der I_{Kr}-Blocker deutlich reduziert, was darauf zurückgeführt wird, daß unter diesen Bedingungen der I_{Ks} stärker zur Repolarisierung beiträgt. Aus diesen Gründen weisen die erfindungsgemäßen Substanzen, die als I_{Ks}-Blocker wirken, wesentliche Vorteile auf gegenüber den bekannten I_{Kr}-Blockern. Inzwischen wurde auch beschrieben, daß eine Korrelation zwischen I_{Ks}-Kanal-inhibitorischer Wirkung und dem Unterbinden von lebensbedrohlichen cardialen Arrhythmien besteht, wie sie beispielsweise durch β-adrenerge Hyperstimulation ausgelöst werden (z. B. T.J. Colatsky, C.H. Follmer und C.F. Starmer; "Channel Specificity in Antiarrhythmic Drug Action; Mechanism of potassium channel block and its role in suppressing and aggravating cardiac arrhythmias"; Circulation 82 (1990), 2235 - 2242; A.E. Busch, K. Malloy, W.J. Groh, M.D. Varnum, J.P. Adelman und J. Maylie; "The novel class III antiarrhythmics NE-10064 and NE-10133 inhibit I_{sK} channels in xenopus oocytes and I_{Ks} in guinea pig cardiac myocytes"; Biochem. Biophys. Res. Commun. 202 (1994), 265 - 270).

Darüber hinaus tragen die Verbindungen zu einer deutlichen Verbesserung der Herzinsuffizienz, insbesondere der Stauungsherzinsuffizienz (Congestive Heart Failure) bei, vorteilhafterweise in der Kombination mit kontraktionsfördernden (positiv inotropen) Wirkstoffen, z.B. Phosphordiesterasehemmern.

Trotz der therapeutisch nutzbaren Vorteile, die durch eine Blockade des erzielt werden können, sind bisher nur sehr wenige Verbindungen beschrieben, die diesen Subtyp des "delayed rectifiers" hemmen. Die in der Entwicklung befindliche Substanz Azimilid weist zwar auch eine blockierende Wirkung auf den I_{Ks} auf, blockiert jedoch vorwiegend den I_{Kr} (Selektivität 1 : 10). In der WO-A-95/14470 wird die Verwendung von Benzodiazepinen als selektive Blocker des I_{Ks} beansprucht.

Weitere I_{Ks}-Blocker sind beschrieben in FEBS Letters 396 (1996), 271-275: "Specific blockade of slowly activating I_{sK} channels by chromanols ..." und Pflügers Arch.-Eur. J. Physiol. 429 (1995), 517-530: "A new class of inhibitors of cAMP-mediated Cl- secretion in rabbit colon, acting by the reduction of cAMP-activated K⁺ conductance". Die Potenz der dort genannten 3-Hydroxychromanole ist jedoch geringer als die der erfindungsgemäßen Verbindungen der Formel I.

Die erfindungsgemäßen Verbindungen der Formel I und ihre physiologisch verträglichen Salze können somit am. Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und. Hilfsstoffen enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine. geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III-Antiarrhythmika, in Frage, wie beispielsweise I_{Kr}-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Austausch-Inhibitoren, Calciumkanalantagonisten, Phosphodiesterasehemmer und andere positiv inotrop wirkende Stoffe, wie z. B. Digitalisglykoside, oder Diuretika. Weiterhin sind Kombinationen mit antibiotisch wirkenden Substanzen und mit Antiulkusmitteln vorteilhaft, beispielsweise mit H₂-Antagonisten (z. B. Ranitidin, Cimetidin, Famotidin, etc.), insbesondere bei der Anwendung zur Behandlung von Magen-Darmerkrankungen.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

### Experimenteller Teil

Liste der Abkürzungen
- CDI: Carbonyldiimidazol
- DCC: Dicyclohexylcarbodiimid
- DMA: N,N-Dimethylacetamid
- DMAP: 4-Dimethylaminopyridin
- DMSO: Dimethylsulfoxid
- EE: Essigsäurethylester
- F.p.: Schmelzpunkt
- HOBT: 1-Hydroxy-1H-benzotriazol
- i. Vak.: im Vakuum
- LM: Lösungsmittel
- NaH: Natriumhydrid; 60proz. Dispersion, sofern nicht anders angegeben.
- PE: Petrolether
- RT: Raumtemperatur
- THF: Tetrahydrofuran
- Phosphazen-Base-P1: Phosphazen-Base-P1-t-Bu-tris-tetramethylen (tert.-Butylimino-tri-pyrrolidino-phosphoran

### Beispiel 1: 5-[Ethylsulfonyl-(2,2,6-trimethyl-chroman-4-yl)-amino]-pentansäureamid

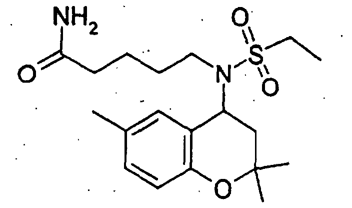

a) Eine Lösung aus 50 g (0,33 mol) 2-Hydroxy-5-methylacetophenon, 240 ml Acetonitril, 56,6 g (0,79 mol) Pyrrolidin und 115 g (1,97 mol) Aceton wird 6 Tage bei RT gerührt. Die Reaktionsmischung wird am Rotationsverdampfer eingeengt und der Rückstand wird mit EE und verd. Salzsäure verrührt. Die organische Phase wird abgetrennt und noch 2mal mit verd. Salzsäure gewaschen. Nach Abdestillieren des Lösungsmittels wird der Rückstand durch Chromatographie mit Cyclohexan/EE 95:5 gereinigt, und man erhält 45 g 2,2,6-Trimethylchroman-4-on.
b) 28,5 g (0,15 mol) 2,2,6-Trimethylchroman-4-on und 116 g (1,5 mol) Ammoniumacetat in 550 ml Methanol werden mit 65,9 g (1,05 mol) Natriumcyanborhydrid versetzt, und es wird 18 auf 60°C erhitzt. Nach dem Abkühlen wird der Ansatz vorsichtig mit konz. Salzsäure angesäuert und über Nacht stehengelassen. Dann gießt man auf 500 ml Wasser, stellt mit Pottasche alkalisch und extrahiert 2mal mit je 500 ml EE. Nach dem Einengen der organischen Phasen wird der Rückstand mit Salzsäure auf pH 1.0 gestellt und mit 2mal mit EE extrahiert. Die wäßrige Phase wird mit Pottasche gesättigt und 3mal mit EE extrahiert. Nach dem Trocknen und Einengen dieser Extrakte erhält man 19, 4 g 4-Amino-2,2,6-trimethylchroman.
c) Zu einer Lösung von 8,7 g (0,045 mol) 4-Amino-2,2,6-trimethylchroman in 130 ml THF werden unter Kühlung im Eisbad 18,3 g (0,18 mol) Triethylamin und 6,4 g (0,05 mol) Ethansulfonsäurechlorid nacheinander zugetropft. Nach Rühren über Nacht bei RT wird der Niederschlag abfiltriert und das Filtrat im Vakuum eingeengt. Das eingeengte Filtrat wird in EE aufgenommen und nacheinander mit verd. Salzsäure und Natriumbicarbonatlösung gewaschen. Nach Trocknen über Magnesiumsulfat und Einengen im Vakuum erhält man 8,8 g 4-Ethylsulfonylamino-2,2,6-trimethylchroman.
d) Zu einer Suspension von 0,49 g (16,2 mmol) Natriumhydrid (80 proz. Dispersion) in 34 ml DMF werden 4,0 g (14,1 mmol) 4-Ethylsulfonylamino-2,2,6-trimethylchroman gelöst in 52 ml DMF zugetropft. Nach 1 h Rühren bei RT gibt man 2,75 g (14,1 mmol) 5-Bromvaleriansäuremethylester zu und rührt über Nacht bei RT. Dann destilliert man das DMF im Vakuum ab, schüttelt den Rückstand mit Wasser und Essigester und wäscht die organische Phase mit verd. Salzsäure und Natriumbicarbonatlösung. Nach Trocknen über Magnesiumsulfat und Einengen im Vakuum erhält man 5,1 g 5-[Ethylsulfonyl-(2,2,6-trimethyl-chroman-4-yl)-amino]-pentansäuremethylester.
e) Eine Lösung aus 0,4 g (1 mmol) 5-[Ethylsulfonyl-(2,2,6-trimethyl-chroman-4-yl)-amino]-pentansäuremethylester und 1,5 ml flüssigen Ammoniak in 10 ml Methanol wird 9 Tage bei RT stehen gelassen. Nach Einengen im Vakuum wird der Rückstand mit Wasser versetzt und mit EE extrahiert. Man erhält 0,37 g 5-[Ethylsulfonyl-(2,2,6-trimethyl-chroman-4-yl)-amino]-pentansäureamid; Fp. 127-129°C.

### Beispiel 2: 5-[Ethylsulfonyl-(2,2,6-trimethyl-chroman-4-yl)-amino]-pentansäure-(2-diethylamino-ethyl)-amid

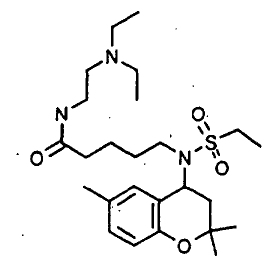

a) 0,5 g (1,25 mmol) 5-[Ethylsulfonyl-(2,2,6-trimethyl-chroman-4-yl)-amino]-pentansäuremethylester (Beispiel 1d) werden mit 0,21 g (3,77 mmol) KOH in 20 ml Methanol über Nacht bei RT gerührt. Nach dem Abziehen des Lösungsmittels im Vakuum wird der Rückstand mit Salzsäure angesäuert und mit EE extrahiert. Man erhält 0,4 g 5-[Ethylsulfonyl-(2,2,6-trimethyl-chroman-4-yl)-amino]-pentansäure.
b) Eine Lösung aus 0,3 g (0,78 mmol) 5-[Ethylsulfonyl-(2,2,6-trimethyl-chroman-4-yl)-amirio]-pentansäure, 0,11 g (0,78 mmol) HOBT und 0,18 g (0,86 mmol) DCC in 4 ml DMF wird 1 h bei 0°C gerührt. Dann fügt man 0,09 g (0,78 mmol) Diethylaminoethylamin zu und rührt über Nacht bei RT. Man versetzt mit 50 ml Wasser und 50 ml EE, wäscht die organische Phase 2mal mit je 25 ml gesättigter Natriumbicarbonatlösung und noch 2mal mit Wasser. Nach Trocknen über Magnesiumsulfat und Einengen im Vakuum erhält man 0,3 g 5-[Ethylsulfonyl-(2,2,6-trimethyl-chroman-4-yl)-amino]-pentansäure-(2-diethylamino-ethyl)-amid.

### Beispiel 3: 5-[Ethylsulfonyl-(2,2,6-trimethyl-chroman-4-yl)-amino]-pentansäure-(3-imidazol-1-yl-propyl)-amid

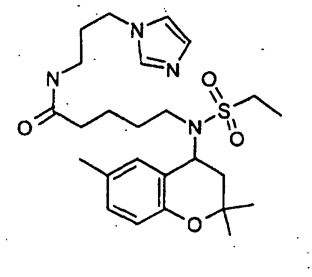

Aus 0,28 g 5-[Ethylsulfonyl-(2,2,6-trimethyl-chroman-4-yl)-amino]-pentansäure (Beispiel 2a), 0,01 g HOBT, 0,17 g DCC und 0,092 g 1-Aminopropylimidazol in 10 ml Methylenchlorid werden analog Beispiel 2b 0,32 g 5-[Ethylsulfonyl-(2,2,6-trimethylchroman-4-yl)-amino]-pentansäure-(3-imidazol-1-yl-propyl)-amid erhalten.

### Beispiel 4: [(2,2-Diethyl-6-methyl-chroman-4-yl)-ethylsulfonyl-amino]-essigsäure-2-(2-ethoxy-ethoxy)-ethyl ester

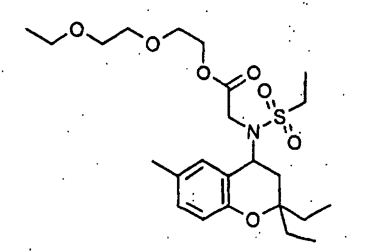

a) Eine Lösung aus 50 g (0,33 mol) 2-Hydroxy-5-methylacetophenon, 240 ml Acetonitril, 56,6 g (0,79 mol) Pyrrolidin und 170 g (1,97 mol) 3-Pentanon wird 5 Tage bei RT gerührt und dann noch 12 h auf 65°C erhitzt. Die Reaktionsmischung wird am Rotationsverdampfer eingeengt und der Rückstand wird mit EE und verd. Salzsäure verrührt. Die organische Phase wird abgetrennt und noch 2mal mit verd. Salzsäure gewaschen. Nach Abdestillieren des Lösungsmittels wird der Rückstand durch Chromatographie mit Cyclohexan/EE 9:1 gereinigt. Zur Entfernung von noch vorhandenem 2-Hydroxy-5-methylacetophenon werden die Produktfraktionen in 800 ml tert.-Butylmethylether gelöst und 4mal mit je 500 ml 1M NaOH extrahiert. Nach Trocknen und Einengen der organischen Phase erhält man 28 g 2,2-Diethyl-6-methylchroman-4-on.
b) Durch reduktive Aminierung von 21,8 g 2,2-Diethyl-6-methylchroman-4-on mit Ammoniumacetat und Natriumcyanborhydrid wie in Beispiel 1b beschrieben, erhält man 21,5 g 4-Amino-2,2-diethyl-6-methylchroman.
c) Aus 11,0 g (0,05 mol) 4-Amino-2,2-diethyl-6-methylchroman, 20,2 g (0,2 mol) Triethylamin und 7,7 g (0,06 mol) Ethansulfonylchlorid in 140 ml THF werden analog Beispiel 1c 11,7 g 2,2-Diethyl-4-ethylsulfonylamino-6-methylchroman erhalten.
d) Zu einer Suspension von 0,22 g (7,4 mmol) Natriumhydrid (80 proz. Dispersion) in 16 ml DMF werden 2,0 g (6,4 mmol) 2,2-Diethyl-4-ethylsulfonylamino-6-methylchroman gelöst in 23 ml DMF zugetropft. Nach 1 h Rühren bei RT gibt man 1,0 g (6,6 mmol) Bromessigsäuremethylester zu und rührt über Nacht bei RT. Dann destilliert man das DMF im Vakuum ab, schüttelt den Rückstand mit Wasser und Essigester und wäscht die organische Phase mit verd. Salzsäure und Natriumbicarbonatlösung. Nach Reinigung durch Chromatographie mit Cyclohexan / EE 98:2 erhält man 1,35 g [(2,2-Diethyl-6-methyl-chroman-4-yl)-ethylsulfonyl-amino]-essigsäuremethylester.
e) Durch Verseifung von 1,0 g (2,6 mmol) [(2,2-Diethyl-6-methyl-chroman-4-yl)-ethylsulfonyl-amino]-essigsäuremethylester mit 0,44 g (7,8 mmol) KOH analog Beispiel 2a erhält man 0,89 g [(2,2-Diethyl-6-methyl-chroman-4-yl)-ethylsulfonyl-amino]-essigsäure.
f) Eine Lösung aus 0,5 g (1,35 mmol) [(2,2-Diethyl-6-methyl-chroman-4-yl)-ethylsuifonyl-amino]-essigsäure, 0,2 g (1,5 mmol) Diethylenglykolmonoethylether, 0,31 g (1,5 mmol) DCC und 2 mg (0,01 mmol) DMAP in 7 ml Methylenchlorid wird über Nacht bei RT gerührt. Man wäscht nacheinander mit 5%iger Essigsäure, gesättigter Natriumbicarbonatlösung und Wasser, trocknet über Magnesiumsulfat und engt im Vakuum ein. Nach anschließender Reinigung durch Chromatographie mit Cyclohexan / EE 4:1 erhält man 0,29 g [(2,2-Diethyl-6-methyl-chroman-4-yl)-ethylsulfonyl-amino]-essigsäure-2-(2-ethoxy-ethoxy)-ethyl ester als viskoses Öl.

### Beispiel 5: 2-[Ethylsulfonyl-(6-fluor-2,2-dimethyl-chroman-4-yl)-amino]-acetamid

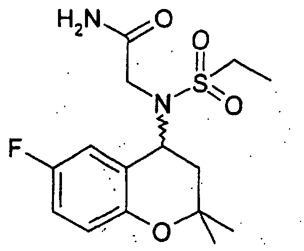

a) Analog der in Beispiel 1a angegebenen Vorschrift erhält man aus 5-Fluor-2-hydroxy-acetophenon und Aceton in Gegenwart von Pyrrolidin 6-Fluor-2,2-dimethyl-4-chromanon.
b) Durch Erhitzen von 10 mmol 6-Fluor-2,2-dimethyl-4-chromanon mit 12 mmol Hydroxylaminhydrochlorid in 5 ml Methanol und 5 ml Pyridin für 2 Stunden auf 80°C erhält man nach Abdestillieren des LM und Ausfällen mit Wasser 6-Fluor-2,2-dimethyl-4-chromanonoxim; Fp. 108-110°C.
c) Durch katalytische Hydrierung von 6-Fluor-2,2-dimethyl-4-chromanonoxim in Methanol im Autoklaven bei 60°C, 100 atm. Druck Wasserstoff in Gegenwart von Raney-Nickel erhält man 4-Amino-6-fluor-2,2-dimethyl-4-chroman (Fp. des Hydrochlorids 226°C).
d) Aus 4-Amino-6-fluor-2,2-dimethyl-4-chroman erhält man analog Vorschrift 1c 4-N-Ethylsulfonylamino-6-fluor-2,2-dimethylchroman als amorphes Produkt.
e) 287 mg (1 mmol) 4-N-Ethylsulfonylamino-6-fluor-2,2-dimethylchroman werden in 5 ml wasserfreiem DMA gelöst und mit 45 mg (1,1 mmol) NaH (60%ig) versetzt. Nach 30 min. Rühren bei RT gibt man 137 mg (1mmol) Bromacetamid zu und rührt über Nacht bei RT. Nach Entfernen des DMA im Vakuum wird EE zugegeben und das LM nochmals im Vakuum entfernt. Der Rückstand wird in Ethylacetat gelöst und mit Natriumhydrogencarbonat-Lösung und Wasser gewaschen. Nach Trocknen und Entfernen des LM im Vakuum erhält man 340 mg (99%) 2-[Ethylsulfonyl-(6-fluor-2,2-dimethyl-chroman4-yl)-amino]-acetamid als Feststoff (Fp. 100-102°C).

### Beispiel 6: 3-[Ethylsulfonyl-(6-fluor-2,2-dimethyl-chroman-4-yl)-amino]-propionamid

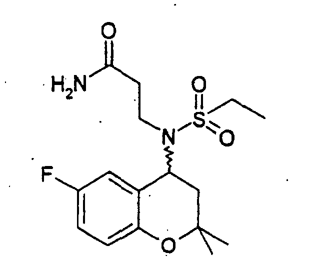

Analog Beispiel 5 erhält man aus 287 mg 4-N-Ethylsulfonylamino-6-fluor-2,2-dimethylchroman und 151mg Brompropionsäureamid 180 mg (50%) : 3-[Ethylsulfonyl-(6-fluor-2,2-dimethyl-chroman-4-yl)-amino]-propionamid als Feststoff (Fp. 134-136°C).

### Beispiel 7: 2-[Ethylsulfonyl-(6-fluor-2,2-dimethyl-chroman-4-yl)-amino]-N-(3-imidazol-1-yl-propyl)-acetamid

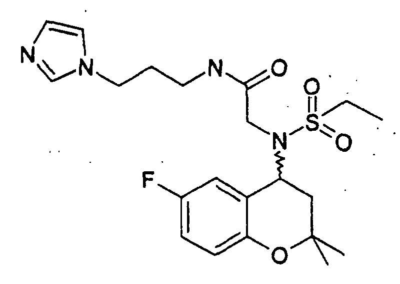

a) 5,74g (20 mmol) 4-N-Ethylsulfonylamino-6-fluor-2,2-dimethylchroman (Beispiel 5d) werden in 100 ml wasserfreiem DMA gelöst und mit 1 g (25 mmol) NaH (60%ig) versetzt. Nach 40 min. Rühren bei RT gibt man 3 ml (27 mmol) Bromessigsäureethylester zu und läßt über Nacht bei RT rühren. Nach Entfernen des DMA im Vakuum wird EE zugegeben und das LM nochmals im Vakuum entfernt. Man verrrührt mit Wasser und saugt den Rückstand ab. Nach Umfällung aus Ethanol/Wasser erhält man 7,2 g (96%) [Ethylsulfonyl-(6-fluor-2,2-dimethyl-chroman-4-yl)-amino]-essigsäureethylester (Fp. 113-114°C).
b) 5,6 g (15 mmol) [Ethylsulfonyl-(6-fluor-2',2-dimethyl-chroman-4-yl)-amino]-essigsäureethylester rührt man über Nacht in einer Mischung aus 200 ml Methanol und 75 ml 2N NaOH. Nach Abdestillieren des Methanol im Vakuum wird mit 100 ml Methylenchlorid gewaschen, und mit konz. HCl angesäuert. Nach Extraktion mit Methylenchlorid und Entfernen des LM im Vakuum erhält 4,65 g (90%) [Ethylsulfonyl-(6-fluor-2,2-dimethyl-chroman-4-yl)-amino]-essigsäure als Schaum (Fp. 154-156°C).
c) 210 mg (0,6 mmol) [Ethylsulfonyl-(6-fluor-2,2-dimethyl-chroman-4-yl)-amino]-essigsäure und 110 mg (0,68 mmol) Carbonylbisimidazol werden 2 Stunden bei RT in 6 ml THF gerührt. Anschließend gibt man 0.080 ml (0.67 mmol) 3-Aminopropylimidazol zu und rührt bei RT über Nacht. Anschließend wird mit 60 ml Wasser verrührt und das THF im Vakuum entfernt. Man extrahiert zweimal mit Ethylacetat, wäscht die organischen Phasen mit 2N NaOH und Wasser, und erhält nach Trocknen und Entfernen des LM 210 mg (77%) 2-[Ethylsulfonyl-(6-fluor-2,2-dimethyl-chroman-4-yl)-amino]-N-(3-imidazol-1-yl-propyl)-acetamid als Öl (Fp. des Hydrochlorids: 188 - 190°C)

### Beispiel 8: 2-[Ethylsulfonyl-(6-fluor-2,2-dimethyl-chroman-4-yl)-amino]-N-(2-morpholin-4-yl-ethyl)-acetamid

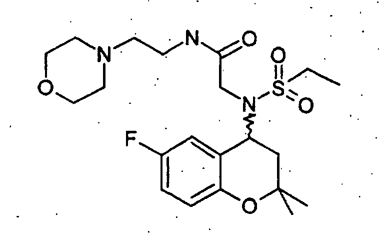

Analog Beispiel 7 erhält man aus 345 mg (1 mmol) [Ethylsulfonyl-(6-fluor-2,2-dimethyl-chroman-4-yl)-amino]-essigsäure und N-Aminoethylmorpholin 325 mg (71%) 2-[Ethylsulfonyl-(6-fluor-2,2-dimethyl-chroman-4-yl)-amino]-N-(2-morpholin-4-yl-ethyl)-acetamid als Öl.

### Beispiel 9: 2-[Ethylsulfonyl-(6-fluor-2,2-dimethyl-chroman-4-yl)-amino]-N-(2-pyridin-2-yl-ethyl)-acetamid

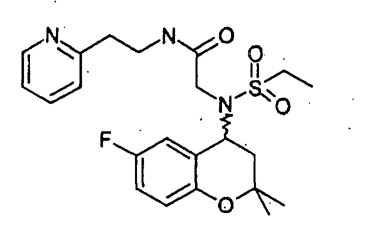

Analog Beispiel 7 erhält man aus 210mg (0,6 mmol) [Ethanesulfonyl-(6-fluoro-2,2-dimethyl-chroman-4-yl)-amino]-essigsäure und 80µl 2-Aminoethylpyridin 250mg (92%) 2-[Ethylsulfonyl-(6-fluor-2,2-dimethyl-chroman-4-yl)-amino]-N-(2-pyridin-2-yl-ethyl)-acetamid als Öl.

### Beispiel 10: 5-[Methansulfonyl-(2,2,6-trimethyl-chroman-4-yl)-amino]-pentansäure-2-(benzyl-methyl-amino)-ethylester

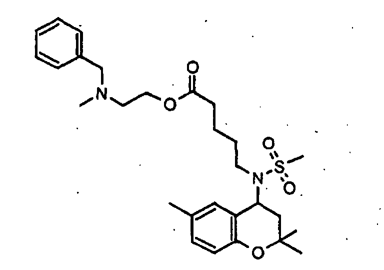

a) Zu einer Lösung von 19,0 g (0,099 mol) 4-Amino-2,2,6-trimethylchroman (Beispiel 1b) in 300 ml THF werden unter Kühlung im Eisbad 40,2 g (0,40 mol) Triethylamin und 12,5 g (0,11 mol) Methansulfonsäurechlorid nacheinander zugetropft. Nach Rühren über Nacht bei RT werden 300 ml Wasser zugegeben, die Reaktionsmischung bis auf 200 ml eingengt und dann mit weiteren 300 ml Wasser verdünnt. Der ausgefallene Niederschlag wird abgesaugt und im Vakuum getrocknet, und man erhält 24,7 g 4-Methylsulfonylamino-2,2,6-trimethylchroman, F.p. 109-111°C.
b) Aus 19,5 g 4-Methylsulfonylamino-2,2,6-trimethylchroman werden analog Beispiel 1d 26,6 g 5-[Methylsulfonyl-(2,2,6-trimethyl-chroman-4-yl)-amino]-pentansäuremethylester erhalten.
c) Durch Verseifung von 20 g 5-[Methylsulfonyl-(2,2,6-trimethyl-chroman-4-yl)-amino]-pentansäuremethylester mit KOH in Methanol / Wasser werden 13,8 g 5-[Methylsulfonyl-(2,2,6-trimethyl-chroman-4-yl)-amino]-pentansäure erhalten, F.p. 105-107°C.
d) Eine Lösung aus 0,5 g (1,35 mmol) 5-[Methylsulfonyl-(2,2,6-trimethyl-chroman-4-yl)-amino]-pentansäure, 0,25 g (1,5 mmol) 2-(N-Benzyl-N-methylamino)ethanol, 0,31 g (1,5 mmol) DCC und einer Spatelspitze DMAP in 10 ml Methylenchlorid wird über Nacht bei RT gerührt. Nach Abfiltrieren des Niederschlags wird die Lösung eingeengt und das Rohprodukt durch Chromatographie an Kieselgel mit Methylenchlorid / Methanol 97:3 gereinigt, und man erhält 0,45 g 5-[Methansulfonyl-(2,2,6-trimethyl-chroman-4-yl)-amino]-pentansäure-2-(benzyl-methyl-amino)-ethylester.

### Beispiel 11: 5-[Methylsulfonyl-(2,2,6-trimethyl-chroman-4-yl)-amino]-pentansäure-(2-(2-pyridyl)-ethyl)-amid

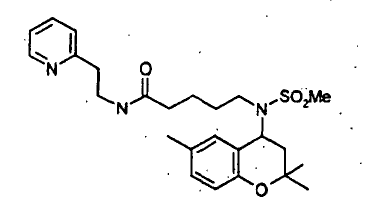

Eine Mischung aus 0,5 g (1,4 mmol) 5-[Methylsulfonyl-(2,2,6-trimethyl-chroman-4-yl)-amino]-pentansäure (Beispiel 10c) und 0,26 g (1,6 mmol) CDI in 20 ml THF wird 3 h bei RT gerührt. Dann werden 0,2 g (1,6 mmol) 2-(2-Aminoethyl)-pyridin zugegeben und über Nacht bei RT weiter gerührt. Nach Einengen der Reaktionsmischung wird der Rückstand in EE und Wasser aufgenommen, und die organische Phase wird 3 mal mit 2M Natronlauge gewaschen. Nach Trocknen über Magnesiumsulfat, Einengen und Reinigung duch Chromatographie an Kieselgel mit Methylenchlorid / Methanol 9:1 werden 0,33 g 5-[Methylsulfonyl-(2,2,6-trimethyl-chroman-4-yl)-amino]-pentansäure-(2-(2-pyridyl)-ethyl)-amid erhalten.

### Beispiel 12: 5-[Methylsulfonyl-(2,2,6-trimethyl-chroman-4-yl)-amino]-pentansäure-(2-pyridyl-methyl)-amid

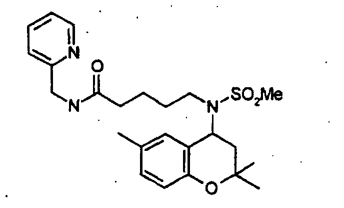

Aus 0,5 g (1,4 mmol) 5-[Methylsulfonyl-(2,2,6-trimethyl-chroman-4-yl)-amino]-pentansäure (Beispiel 10c) und 0,18 g (1,6 mmol) 2-Picolylamin erhält man analog Beispiel 11 0,3 g 5-[Methylsulfonyl-(2,2,6-trimethyl-chroman-4-yl)-amino]-pentansäure-(2-pyridyl-methyl)-amid.

### Beispiel 13: 2-[(6-Fluor-2,2-dimethyl-chroman-4-yl)-ethansulfonyl-amino]-N-(1 H-pyrazol-3-yl)-acetamid

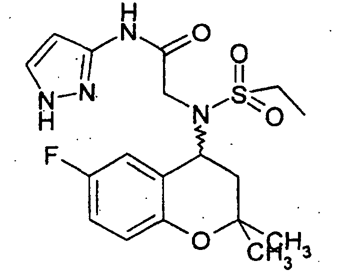

Analog Beispiel 7 erhält man 260 mg der Substanz mit einem Schmelzpunkt von 191°C.

### Beispiel 14: 2-[(6-Fluor-2,2-dimethyl-chroman-4-yl)-ethansulfonyl-amino]-N-pyridin-2-yl-acetamid

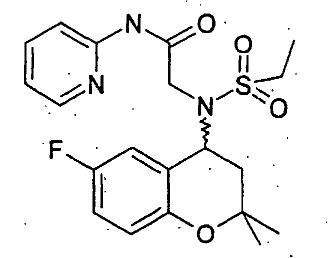

Analog Beispiel 7 erhält man 280 mg der Substanz mit einem Schmelzpunkt von 68°C.

### Beispiel 15 : 2-[(6-Fluor-2,2-dimethyl-chroman-4-yl)-ethansulfonyl-amino]-N-[2-(5-nitro-pyridin-2-yl)-ethyl]-acetamid

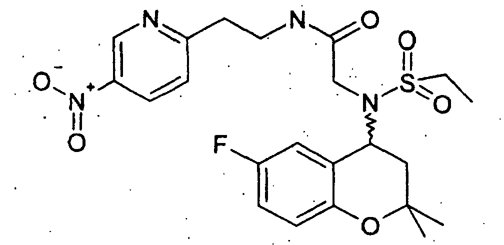

Analog Beispiel 7 erhält man 180 mg der Substanz mit einem Schmelzpunkt von 155°C.

### Beispiel 16: N-[2-(5-Amino-pyridin-2-yl)-ethyl]-2-[(6-fluor-2,2-dimethyl-chroman-4-yl)-ethansulfonyl-amino]-acetamid

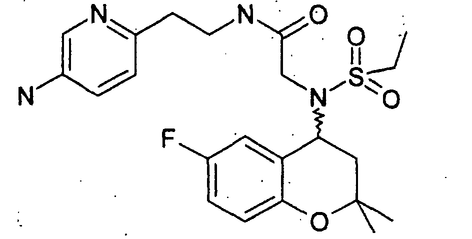

230 mg 2-[(6-Fluor-2,2-dimethyl-chroman-4-yl)-ethansulfonyl-amino]-N-[2-(5-nitro-pyridin-2-yl)-ethyl]-acetamid (Beispiel 15) werden in 10 ml EE gelöst und mit 1,1 g Zinnchlorid-Hydrat versetzt und 2 h zum Rückfluß gekocht. Man versetzt mit NaHCO₃-Lösung bis zur alkalischen Reaktion. Die anorganischen Salze werden abgesaugt und die EE-Phase getrocknet und im Vakuum das LM entfernt. Das Rohprodukt wird mit ethanolischer HCI versetzt und eingeengt. Nach Versetzen mit Wasser wird mit EE gewaschen. Die Wasser-phase wird mit Natriumcarbonat alkalisch eingestellt und mit EE extrahiert. Nach Entfernen des LM erhält man 75 mg des Produkts als Öl.

### Beispiel 17: 2-[(6-Benzyloxy-2,2-dimethyl-chroman-4-yl)-methansulfonyl-amino]-N-(2-pyridin-2-yl-ethyl)-acetamid

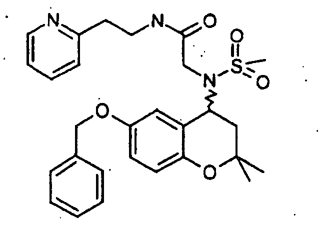

a) [(6-Benzyloxy-2,2-dimethyl-chroman-4-yl)-methansulfonyl-amino]-essigsäuremethylester
   1,08 g 6-Benzyloxy-4-(methylsulfonyl)amino-2,2-dimethylchroman (Beispiel 28 e) werden in 15 ml wasserfreiem DMA gelöst und mit 160 mg NaH (60%ig) bei RT für 45 min gerührt. Dazu gibt man 0,5 ml Bromessigsäuremethylester und rührt 14 h bei RT. Nach Entfernen des LM in Vakuum wird der Rückstand in EE aufgenommen und mit Wasser gewaschen. Nach Trocknen und Entfernen des LM im Vakuum erhält man 1,4g [(6-Benzyloxy-2,2-dimethyl-chroman-4-yl)-methansulfonyl-amino]-essigsäuremethylester als Öl.
b) [(6-Benzyloxy-2,2-dimethyl-chroman-4-yl)-methansulfonyl-amino]-essigsäure 1,4g [(6-Benzyloxy-2,2-dimethyl-chroman-4-yl)-methansulfonyl-amino]-essigsäuremethylester werden in 50 ml Methanol und 15 ml 2N NaOH 4 h bei RT gerührt. Nach Entfernen des Methanols wird mit EE gewaschen und mit HCl angesäuert. Man extrahiert mit EE und entfernt nach Trocknen das LM im Vakuum. Man erhält 500 mg der Säure mit einem Schmelzpunkt von 167-169°C.
c) 2-[(6-Benzyloxy-2,2-dimethyl-chroman-4-yl)-methansulfonyl-amino]-N-(2-pyridin-2-yl-ethyl)-acetamid
   Man erhält das Produkt analog der Vorgehensweise in Beispiel 7 aus [(6-Benzyloxy-2,2-dimethyl-chroman-4-yl)-methansulfonyl-amino]-essigsäure und dem entsprechenden Amin unter Einsatz von Carbonyl-bis-imidazol. F.p. 131°C.

### Beispiel 18 : 4-[Ethansulfonyl-(6-fluor-2,2-dimethyl-chroman-4-yl)-amino]-N-(2-pyridin-2-yl-ethyl)-butyramid

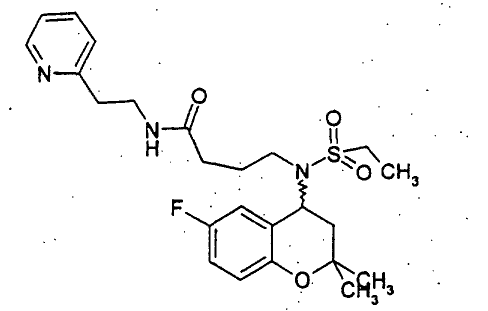

a) 4-[Ethansulfonyl-(6-fluor-2,2-dimethyl-chroman-4-yl)-amino]-butansäureethylester erhält man aus Ethylsulfonylamino-6-fluor-2,2-dimethyl-4-chroman (Beispiel 5d) in analoger Reaktion zu Beispiel 7a unter Verwendung von 4-Brombuttersäureethylester anstelle von Bromessigsäureethylester.
b) 4-[Ethansulfonyl-(6-fluor-2,2-dimethyl-chroman-4-yl)-amino]-butansäure erhält man analog Beispiel 7b.
c) 4-[Ethansulfonyl-(6-fluor-2,2-dimethyl-chroman-4-yl)-amino]-N-(2-pyridin-2-yl-ethyl)-butyramid erhält man analog Reaktion 7c als Öl.

### Beispiel 19: 2-[Ethansulfonyl-(6-fluor-2,2-dimethyl-chroman-4-yl)-amino]-N-(2-piperidin-1-yl-ethyl)-acetamid

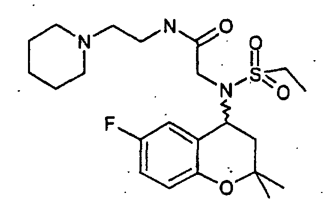

Analog Beispiel 7 erhält man 180mg der Substanz als Öl.

### Beispiel 20: 2-[Ethansulfonyl-(6-fluor-2,2-dimethyl-chroman-4-yl)-amino]-N-(2-piperazin-1-yl-ethyl)-acetamid-dihydrochlorid

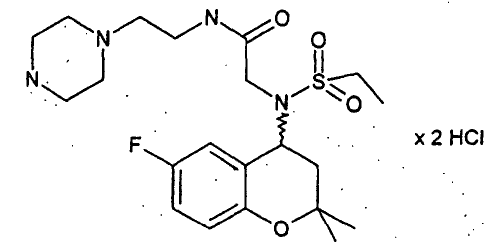

Analog Beispiel 7 erhält man 260mg der Substanz als Öl.

### Beispiel 21: 2-[Ethansulfonyl-(6-fluor-2,2-dimethyl-chroman-4-yl)-amino]-N-phenethyl-acetamid

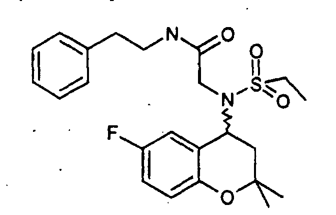

Analog Beispiel 7 erhält man 270 mg der Substanz mit einem Schmelzpunkt von 130°C.

### Beispiel 22: 2-[Ethansulfonyl-(6-fluor-2,2-dimethyl-chroman-4-yl)-amino]-N-pyridin-4-yl-acetamid

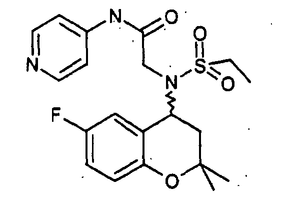

a) [Ethansulfonyl-(6-fluor-2,2-dimethyl-chroman-4-yl)-amino]-acetylchlorid 3,45 g [Ethansulfonyl-(6-fluor-2,2-dimethyl-chroman-4-yl)-amino]-essigsäure (Beispiel 7b) werden in 20 ml Thionylchlorid gelöst und 2 h zum Rückfluß erhitzt. Nach Entfernen des Thionylchlorids in Vakuum wird je einmal mit Toluol und Methylenchlorid verdünnt und diese LM im Vakuum entfernt. Man erhält 3,7 g des Säurechlorids als Öl.
b) 2-[Ethansulfonyl-(6-fluor-2,2-dimethyl-chroman-4-yl)-amino]-N-pyridin-4-yl-acetamid
   141 mg p-Amino-pyridin und eine Spatelspitze DMAP gelöst in 6 ml Pyridin werden mit einer Lösung von 655 mg [Ethansulfonyl-(6-fluor-2,2-dimethyl-chroman-4-yl)-amino]-acetylchlorid in 6ml Methylenchlorid versetzt. Es wird 4 h bei RT gerührt und anschließend im Vakuum konzentriert. Das Produkt wird zwischen EE und Wasser verteilt und die organische Phase mit Wasser gewaschen. Nach Entfernen des LM im Vakuum erhält man 600 mg des Produkts als farblosen Schaum (F.p. 195°C).

### Beispiel 23: 2-[Ethansulfonyl-(6-fluor-2,2-dimethyl-chroman-4-yl)-amino]-N-pyridin-3-yl-acetamid

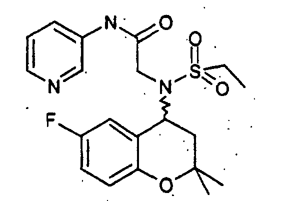

Analog Beispiel 22 erhält man 390 mg der Substanz als Öl.

### Beispiel 24: 2-[Ethansulfonyl-(6-fluor-2,2-dimethyl-chroman-4-yl)-amino]-N-pyrimidin-2-yl-acetamid

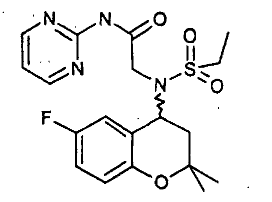

Analog Beispiel 22 erhält man 370 mg der Substanz als Öl.

### Beispiel 25: 2-[Ethansulfonyl-(6-fluor-2,2-dimethyl-chroman-4-yl)-amino]-N-pyrazin-2-yl-acetamid

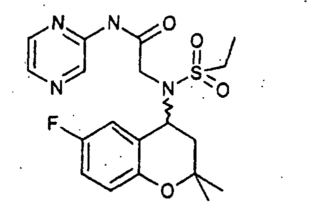

Analog Beispiel 22 erhält man 220 mg der Substanz als Feststoff (F.p. 189°C).

### Beispiel 26: N-Benzothiazol-2-yl-2-[ethansulfonyl-(6-fluor-2,2-dimethyl-chroman-4-yl)-amino]-acetamid

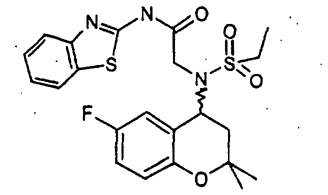

Analog Beispiel 22 erhält man 370 mg der Substanz als Feststoff (F.p. 114°C).

### Beispiel 27: 2-[Ethansulfonyl-(6-fluor-2,2-dimethyl-chroman-4-yl)-amino]-N-(1-methyl-1H-benzoimidazol-2-yl)-acetamid

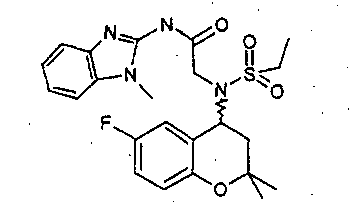

Analog Beispiel 22 erhält man 470 mg der Substanz als Öl.

### Beispiel 28: 4-[(6-Benzyloxy-2,2-dimethyl-chroman-4-yl)-methansulfonyl-amino]-N-butyl-butyramid

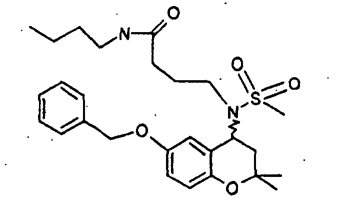

a) 2,2-Dimethyl-6-hydroxychroman-4-on
   Eine Reaktionsmischung aus 100 g (0,65 mol) 2,5-Dihydroxycetophenon in 1 l Acetonitril, 130 ml (1,55 mol) Pyrrolidin und 290 ml (3,95 mol) Aceton wurde 8 h auf 45°C erhitzt. Dann wurden die LM i. Vak. abgezogen und der Rückstand in 1 I EE gelöst. Die organische Phase wurde 2mal mit verdünnter Salzsäure gewaschen, mit Aktivkohle verrührt und über Magnesiumsulfat getrocknet und weitgehend eingeengt. Nach Verrühren des Rückstandes mit Petrolether und Absaugen des Niederschlags wurden 102 g 2,2-Dimethyl-6-hydroxychroman-4-on, F.p. 158°C, erhalten.
b) 6-Benzyloxy-2,2-dimethylchroman-4-on
   25,2 g (131,2 mmol) 6-Hydroxy-2,2-dimethylchroman-4-on wurden bei RT unter Rühren in 350 ml Diethylketon eingetragen und nach Zugabe von 18,0 g (131 mmol) gepulvertem Kaliumcarbonat 30 min bei 75°C gerührt. Nach Abkühlen auf 60°C wurden 15,7 ml (131 mmol) Benzylbromid zugetropft, nach 2 h i.Vak. eingeengt, der Rückstand mit Wasser behandelt und der Feststoff abgesaugt, 37 g, F.p. 105 - 107 °C.
c) 6-Benzyloxy-2,2-dimethylchroman-4-onoxim
   Durch Erhitzen von 11,3 g (40 mmol) 6-Benzyloxy-2,2-dimethylchroman-4-on mit 3,1 g (44 mmol) Hydroxylaminhydrochlorid in 27 ml Ethanol und 27 ml Pyridin für 3 h auf 70°C erhielt man nach Abdestillieren des LM i. Vak. und Ausfällen mit Wasser 12,5 g Produkt, F.p. 105 -108°C. Das Produkt wurde in EE gelöst, getrocknet, eingeengt und mit Petrolether kristallisiert; F.p. 118 - 120°C.
d) 4-Amino-6-benzyloxy-2,2-dimethylchroman
   30 g 6-Benzyloxy-2,2-dimethylchroman-4-onoxim wurden in 900 ml THF/Methanol (1:1) gelöst, mit 25 ml wäßrigem Ammoniak versetzt und mit Raney - Ni in der Schüttelente hydriert. Dann wurde vom Katalysator abgesaugt, das Filtrat i. Vak. eingeengt, der Rückstand in EE gelöst, getrocknet, eingeengt und der Rückstand mit Petrolether zur Kristallisation gebracht, 22,9 g, F.p. 86 - 88°C.
e) 6-Benzyloxy-4-(methylsulfonyl)amino-2,2-dimethylchroman
   4,0 g (14 mmol) 4-Amino-6-benzyloxy-2,2-dimethylchroman wurden in 80 ml THF bei RT mit 4,2 ml (30 mmol) Triethylamin versetzt und 30 min gerührt, anschließend mit 1,95 g (1,3 ml, 17 mmol) Methansulfonsäurechlorid versetzt, wobei die Temperatur auf 40°C anstieg. Dann wurde 2 h zum Rückfluß erhitzt, über Nacht bei RT stehengelassen, i. Vak. eingeengt und der Rückstand mit Wasser behandelt; 4,9 g Produkt, F.p. 162 - 165°C.
f) 4-[(6-Benzyloxy-2,2-dimethyl-chroman-4-yl)-methansulfonyl-amino]-N-buttersäureethylester
   Zu einer Lösung von 1,8 g (45 mmol) NaH (ca. 60 % ige Dispersion in Mineralöl) in 100 ml DMA wurden unter Stickstoffatmosphäre unter Rühren portionsweise 9,0 g (25 mmol) 6-Benzyloxy-4-(methylsulfonyl)amino-2,2-dimethylchroman hinzugegeben und 30 min bei 45°C gerührt. Dann wurden 5,3 ml (30 mmol) 4-Brombuttersäureethylester hinzugetropft und 90 min auf 110°C erhitzt. Nach dem Abkühlen wurde i. Vak. eingeengt, der Rückstand mit 1n wäßriger Salzsäure behandelt, in EE aufgenommen, getrocknet, eingeengt und der Rückstand mit Heptan/EE 2:1 an Kieselgel chromatographiert. Entsprechende Fraktionen wurden mit PE/ DiiE zur Kristallisation gebracht; 10,1 g, F.p. 78-80°C.
g) 4-[(6-Benzyloxy-2,2-dimethyl-chroman-4-yl)-methansulfonyl-amino]-N-buttersäure
   7,0 g des vorstehenden Ethylesters wurden in 200 ml 1,5 n methanolische NaOH eingetragen und 1 h bei 45°C gerührt. Dann wurde eingeengt, der Rückstand in 150 ml Wasser gelöst, unter Kühlung mit konz. Salzsäure auf pH 1 gebracht, der harzige Rückstand in EE aufgenommen, getrocknet, eingeengt und der Rückstand mit PE zur Kristallsation gebracht; 6,4 g Produkt, F.p. 138-140°C.
h) 3,1 g der vorstehenden Carbonsäure wurden in 80 ml THF mit 3 Tropfen N,N-Dimethylacetamid und 1,2 ml (14 mmol) Oxalylchlorid umgesetzt und zu dieser Lösung bei 5°C 1,4 ml (14 mmol) Butylamin in wenig THF hinzugetropft. Nach 30 min wurde eingeengt, mit 1n wäßriger Salzsäure behandelt, der Rückstand in EE aufgenommen, getrocknet, eingeengt und der Rückstand mit EE an Kieselgel chromatographiert. Man erhielt 1,6 g 4-[(6-Benzyloxy-2,2-dimethyl-chroman-4-yl)-methansulfonyl-amino]-N-butyl-butyramid als öliges Produkt.

### Beispiel 29: N-(6-Benzyloxy-2,2-dimethyl-chroman-4-yl)-N-[4-(2-methoxy-ethoxy)-butyl]-methansulfonamid

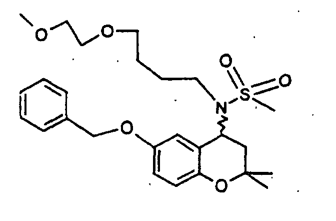

a) N-(6-Benzyloxy-2,2-dimethyl-chroman-4-yl)-N-[4-hydroxy-butyl]-methansulfonamid
   5,7 g (12 mmol) 4-[(6-Benzyloxy-2,2-dimethyl-chroman-4-yl)-methansulfonyl-amino]-N-buttersäureethylester (Bspl. 28 f) wurden in 100 ml THF bei 0°C mit 10 ml 1 M Lösung von Lithiumaluminiumhydrid in THF versetzt. Bei RT versetzte man mit wenig Wasser, dann mit 1n Salzsäure, engte ein und extrahierte den Rückstand 3mal mit EE. Nach Trocknen und Einengen kristallisierte das Produkt über Nacht. Man erhielt 4,9 g, F.p. 113-115°C (aus PE/DiiE).
b) 0,52 g (1,2 mmol) der vorstehenden Verbindung wurden in 20 ml DMA mit 0,12 g (3 mmol) NaH 30 min bei 50°C gerührt. Nach Zugabe von 0,5 ml (5 mmol) 2-Methoxyethylbromid wurde 90 min auf 120°C erhitzt. Nach Zugabe von weiteren 0,36 g NaH und 0,5 ml Bromid war nach 1 h bei 120°C der Umsatz vollständig. Nach Aufarbeitung wurde der Rückstand mit Heptan/EE 1:1 an Kieselgel chromatographiert. Man erhielt 0,26 g der öligen Titelverbindung.

### Beispiel 30: {4-[(6-Benzyloxy-2,2-dimethyl-chroman-4-yl)-methansulfonyl-amino]-butoxy}-essigsäureethylester

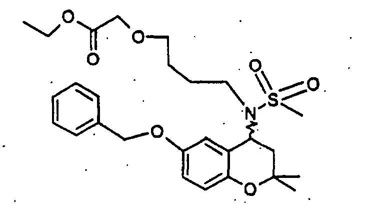

1,5 g (3,5 mmol) N-(6-Benzyloxy-2,2-dimethyl-chroman-4-yl)-N-[4-hydroxy-butyl]-methansulfonamid (Beispiel 29a) wurden in 50 ml DMA bei RT unter Argon mit 0,24 g (6 mmol) NaH versetzt und 30 min bei 50°C gerührt. Dann wurde 0,5 ml (4,5 mmol) Bromessigsäureethylester zugetropft und 1 h auf 80°C erwärmt. Beide Reagenzien wurden in gleichen Mengen noch 2x zugegeben und dann jeweils wieder erhitzt. Nach Aufarbeitung und Säulenchromatographie mit Heptan/EE 1:1 an Kieselgel erhielt man aus entsprechenden Fraktionen 0,7 g der öligen Titelverbindung.

### Beispiel 31: ({4-[(6-Benzyloxy-2,2-dimethyl-chroman-4-yl)-methansulfonyl-amino]-butyryl}-methyl-amino)-essigsäureethylester

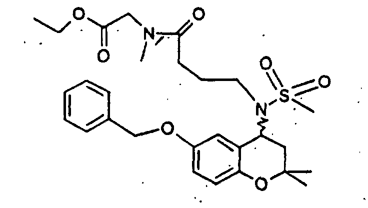

0,9 g (2 mmol) 4-[(6-Benzyloxy-2,2-dimethyl-chroman-4-yl)-methansulfonyl-amino]-N-buttersäure (Beispiel 28g) wurden in 50 ml THF bei RT unter Rühren mit 0,42 g (2,5 mmol) N,N-Carbonyldiimidazol versetzt und 1 h bei 60°C gerührt. Dann gab man eine Suspension von Sarcosinethylester Hydrochlorid und Triethylamin in THF zu und erwärmte 2 h auf 60°C. Nach Aufarbeitung erhielt man 0,75 g harziges Produkt aus EE.

### Beispiel 32: ({2-[(6-Benzyloxy-2,2-dimethyl-chroman-4-yl)-methansulfonyl-amino]-acetyl}-methyl-amino)-essigsäureethylester

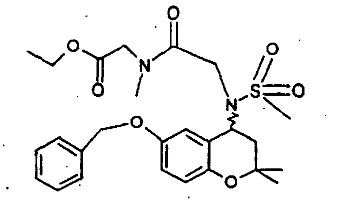

a) 4-[(6-Benzyloxy-2,2-dimethyl-chroman-4-yl)-methansulfonyl-amino]-N-essigsäureethylester
   Zu einer Lösung von 0,72 g (18 mmol) NaH in 50 ml DMA wurden unter Stickstoffatmosphäre unter Rühren portionsweise 3,6 g (10 mmol) 6-Benzyloxy-4-(methylsulfonyl)amino-2,2-dimethylchroman (Beispiel 28 e) hinzugegeben und 30 min bei 50°C gerührt. Dann wurden 1,5 ml (13,5 mmol) Bromessigsäureethylester hinzugetropft und120 min auf 110°C erhitzt. Nach dem Abkühlen wurde i. Vak. eingeengt, der Rückstand mit 1n wäßriger Salzsäure behandelt, in EE aufgenommen, getrocknet, eingeengt und der Rückstand mit Heptan/EE 3:1 an Kieselgel chromatographiert. Entsprechende Fraktionen wurden mit PE zur Kristallisation gebracht; 3,6 g, F.p. 119-121°C.
b) 4-[(6-Benzyloxy-2,2-dimethyl-chroman-4-yl)-methansulfonyl-amino]-N-essigsäure 7,0 g des vorstehenden Ethylesters wurden in 250 ml 1,5 n methanolische NaOH eingetragen und 1 h bei 50°C gerührt. Dann wurde eingeengt, der Rückstand in 150 ml Wasser gelöst, unter Kühlung mit konz. Salzsäure auf pH 1 gebracht, der harzige Rückstand in EE aufgenommen, getrocknet, eingeengt und der Rückstand mit PE zur Kristallsation gebracht; 6,2 g Produkt, F.p. 177-179°C.
c) 0,525 g (1,25 mmol) 4-[(6-Benzyloxy-2,2-dimethyl-chroman-4-yl)-methansulfonyl-amino]-N-essigsäure wurden in 50 ml THF bei RT unter Rühren mit 0,248 g (1,5 mmol) N,N'-Carbonyldiimidazol versetzt und 1 h bei 60°C gerührt. Dann gab man bei RT eine Suspension von 0,184 g (1,2 mmol) Sarcosinethylester Hydrochlorid und 0,18 ml (1,3 mmol) Triethylamin in THF zu und erwärmt 2 h auf 60-70°C. Nach Aufarbeitung erhielt man 0,55 g öliges Produkt aus EE.

### Beispiel 33: 4-[(6-Benzyloxy-2,2-dimethyl-chroman-4-yl)-methansulfonyl-amino]-N-(2-piperidin-1-yl-ethyl)-butyramid-hydrochlorid

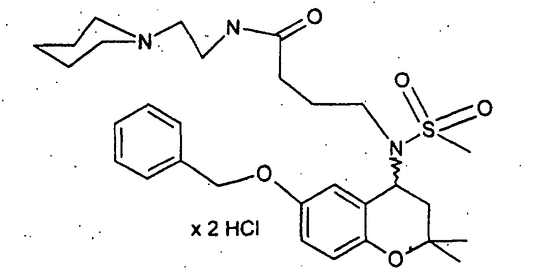

0,7 g (1,5 mmol) 4-[(6-Benzyloxy-2,2-dimethyl-chroman-4-yl)-methansulfonyl-amino]-N-buttersäure (Beispiel 28g) wurden in 40 ml THF mit 0,35 g (2 mmol) N,N'-Carbonyldiimidazol 1h bei 60°C gerührt. Dann wurde 1 ml (ca. 6 mmol) 2-Aminoethylpiperidin zugegeben, 3 h bei 60°C gerührt, eingeengt, mit Wasser behandelt, mit EE extrahiert, die organische Phase getrocknet, eingeengt, der Rückstand mit EE/Methanol 2:1 an Kieselgel gereinigt. 0,65 g des erhaltenen Öls wurden in THF mit etherischer Salzsäure versetzt, eingeengt und mit PE zur Kristallistion gebracht; 0,56 g, F.p. 178-180°C.

### Beispiel 34: {4-[(6-Benzyloxy-2,2-dimethyl-chroman-4-yl)-methansulfonyl-amino]-butoxy)-essigsäure

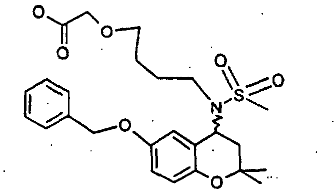

0,3 g {4-[(6-Benzyloxy-2,2-dimethyl-chroman-4-yl)-methansulfonyl-amino]-butoxy}-essigsäureethylester (Beispiel 30) wurden in 25 ml 1,5 n methanolischer NaOH 30 min bei 40°C verseift. Dann wurde eingeengt, mit Salzsäure auf pH 1 gebracht, ausgefallenens Harz in EE aufgenommen, getrocknet, eingeengt und der Rückstand mit DiiE zur Kristallisation gebracht; 90 mg Produkt, F.p. 110-113°C.

### Beispiel 35: ({4-[(6-Benzyloxy-2,2-dimethyl-chroman-4-yl)-methansulfonyl-amino]-butyryl}-methyl-amino)-essigsäure

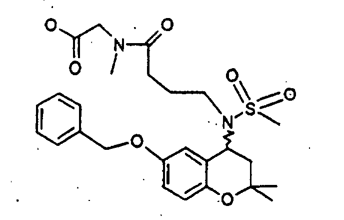

0,4 g (0,75 mmol) ({4-[(6-Benzyloxy-2,2-dimethyl-chromän-4-yl)-methansulfonyl-amino]-butyryl}-methyl-amino)-essigsäureethylester (Beispiel 31)
wurden in 50 ml 1n methanolischer NaOH 2 h bei RT verseift. Nach Aufarbeitung (THF, wäßrige Salzsäure, EE) brachte man 0,34 g der Titelverbindung mit DiiE zur Kristallisation, F.p. ca. 50°C.

### Beispiel 36: 4-({4-[(6-Benzyloxy-2,2-dimethyl-chroman-4-yl)-methansulfonyl-amino]-butyryl}-methyl-amino)-buttersäure

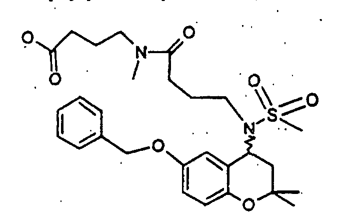

0,7 g (1,5 mmol) 4-[(6-Benzyloxy-2,2-dimethyl-chroman-4-yl)-methansulfonyl-amino]-N-buttersäure (Beispiel 28 g) wurden in 40 ml THF mit 0,35 g (2 mmol) N,N'-Carbonyldiimidazol 1 h bei 60°C gerührt. Dann wurden 0,62 g (4 mmol) 4-Methylaminobuttersäure Hydrochlorid in 25 ml THF und 1,3 ml Triethylamin zugegeben und 3 h zum Rückfluß erhitzt. Nach Aufarbeitung wurden 0,11 g der Titelverbindung als Harz isoliert.

### Beispiel 37: 4-({2-[(6-Benzyloxy-2,2-dimethyl-chroman-4-yl)-methansulfonyl-amino]-acetyl}-methyl-amino)-buttersäure

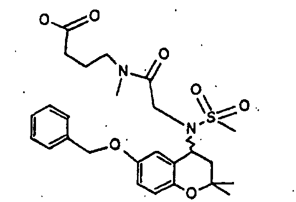

0,525 g (1,25 mmol) 4-[(6-Benzyloxy-2,2-dimethyl-chroman-4-yl)-methansulfonyl-amino]-N-essigsäure wurden in 50 ml THF bei RT unter Rühren mit 0,248 g (1,5 mmol) N,N'-Carbonyldiimidazol versetzt und 1 h bei 60°C gerührt. Dann gab man bei RT eine Suspension von 0,184 g (1,2 mmol) 4-(N-Methylamino)buttersäure Hydrochlorid und 0,45 ml (3,25 mmol) Triethylamin in THF zu und erwärmte 2 h auf 60-70°C. Nach Aufarbeitung erhielt man 0,406 g amorphes Produkt aus EE.

### Beispiel 38: N-(6-Benzyloxy-2,2-dimethyl-chroman-4-yl)-N-[2-(2-methoxy-ethoxy)-ethyl]-methansulfonamid

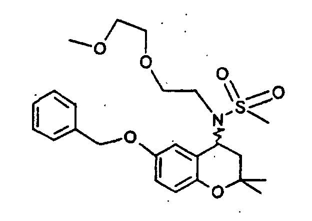

a) N-(6-Benzyloxy-2,2-dimethyl-chroman-4-yl)-N-[2-hydroxyethyl]-methansulfonamid 3,8 g (8,5 mmol) 4-[(6-Benzyloxy-2,2-dimethyl-chroman-4-yl)-methansulfonyl-amino]-N-essigsäureethylester (Beispiel 32 a) wurden in 80 ml THF bei 0°C mit 8 ml 1 M Lösung von Lithiumaluminiumhydrid in THF versetzt. Bei RT versetzte man mit wenig Wasser, dann mit 1n Salzsäure, engte ein und extrahierte den Rückstand 3mal mit EE. Nach Trocknen und Einengen wurde das Rohprodukt mit EE an Kieselgel chromatographiert. Aus entsprechenden Fraktionen brachte man 3,0 g Produkt mit DiiE zur Kristallisation, F.p. 78-80°C.
b) 0,49 g (1,2 mmol) des vorstehenden Alkohols wurden in 20 ml DMA mit 216 mg (5,4 mmol) NaH versetzt und 30 min bei 80-90°C gehalten. Bei 60°C wurden dann 0,4 ml (4 mmol) 2-Methoxyethylbromid hinzugegeben und 2 h bei 110°C gerührt. Nach Aufarbeitung und Reinigung des Rohprodukts mit Heptan/EE 1:1 an Kieselgel erhielt man 70 mg der öligen Titelverbindung.

### Beispiel 39: 2-[(6-Benzyloxy-2,2-dimethyl-chroman-4-yl)-methansulfonyl-amino]-N-methyl-N-phenethyl-acetamid

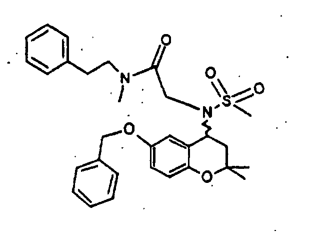

1,58 g (3,25 mmol) 4-[(6-Benzyloxy-2,2-dimethyl-chroman-4-yl)-methansulfonyl-amino]-N-essigsäure (Beispiel 32b) wurden in 100 ml THF bei RT unter Rühren mit 0,975 g (6 mmol) N,N'-Carbonyldiimidazol versetzt und 1 h bei 60-70°C gerührt. Zu der Hälfte dieser Lösung gab man dann bei RT eine Lösung von 0,26 ml (1,8 mmol) N-Methyl-2-phenylethylamin und 0,25 ml (1,8 mmol) Triethylamin in THF zu und erwärmte 2 h auf 60-70°C. Nach Aufarbeitung wurde das Rohprodukt mit Heptan/EE 1:1 an Kieselgel chromatographiert. Aus entsprechenden Fraktionen wurden 0,7 g der Titelverbindung mit DiiE zur Kristallisation gebracht, F.p. 106-107°C.

### Beispiel 40: 2-[(6-Benzyloxy-2,2-dimethyl-chroman-4-yl)-methansulfonyl-amino]-N-methyl-N-(2-pyridin-2-yl-ethyl)-acetamid-hydrochlorid

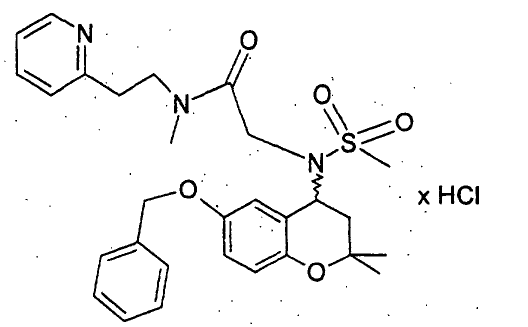

Zu der 2. Hälfte der aktivierten Carbonsäure-Lösung aus Beispiel 39 wurden 0,25 ml (1,8 mmol) N-Methyl-2-(2-pyridyl)ethylamin und 0,25 ml (1,8 mmol) Triethylamin in 30 ml THF hinzugegeben.und 2h unter Rückfluß erhitzt. 0,65 g Produkt kristallisieren nach Aufarbeitung aus wäßriger Salzsäure beim Abkühlen; F.p. 140-143°C.

### Beispiel 41: {2-[(6-Butoxy-2,2-dimethyl-chroman-4-yl)-methansulfonyl-amino]-ethoxy}-essigsäureethylester

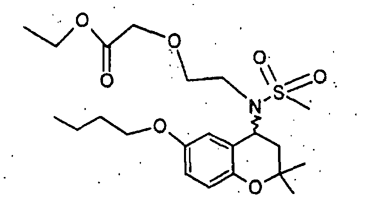

a) 6-Butoxy-2,2-dimethylchroman-4-on
   Zu einer Suspension von 9,0 g (0,3 mol) 80proz. Natriumhydrid in 500 ml DMF wurde eine Lösung von 50 g (0,26 mol) 2,2-Dimethyl-6-hydroxychroman-4-on (Beispiel 28a) in 500 ml DMF zugetropft. Nach 90 min Rühren bei RT wurden 49 g (0,265 mol) lodbutan zugegeben und weitere 90 min bei RT gerührt. Dann wurde die Reaktionsmischung im Vakuum eingeengt, der Rückstand mit Wasser versetzt und mehrmals mit EE extrahiert. Die organischen Phasen wurden mit 5 M Natronlauge gewaschen, mit Aktivkohle und Magnesiumsulfat gerührt, filtriert und eingeengt. Man erhielt 57,6 g 6-Butoxy-2,2-dimethylchroman-4-on.
b) 6-Butoxy-2,2-dimethylchroman-4-onoxim
   Zu einer Lösung von 43,7 g (0,628 mol) Hydroxylammoniumchlorid und 51,5 g (0,628 mol) Natriumacetat in 420 ml Wasser wurde bei 60°C eine Lösung von 52,0 g (0,21 mol) 6-Butoxy-2,2-dimethylchroman-4-on in 420 ml Ethanol in 30 min zugetropft und 3 h bei 60°C gehalten. Die ölige Produktphase wurde abgetrennt und die wäßrige Phase mit Methylenchlorid extrahiert. Nach Trocknen der organischen Phasen über Magnesiumsulfat und Einengen im Vakuum erhielt man 55,5 g 6-Butoxy-2,2-dimethylchroman-4-onoxim, das durch Verrühren mit PE zur Kristallisation gebracht werden konnte.
c) 4-Amino-6-butoxy-2,2-dimethylchroman
   Eine Lösung von 30 g 6-Butoxy-2,2-dimethylchroman-4-onoxim in 500 ml Methanol, 500 ml THF und 30 ml Ammoniaklösung wurde in Gegenwart von Raney-Nickel 8 h in einer Schüttelente hydriert. Nach Abfiltrieren des Katalysators und Einengen im Vakuum wurden 20,5 g 4-Amino-6-butoxy-2,2-dimethylchroman erhalten.
d) 6-Butoxy-4-(methylsulfonyl)amino-2,2-dimethylchroman
   Eine Reaktionsmischung aus 3,0 g (12 mmol) 4-Amino-6-butoxy-2,2-dimethylchroman, 4,9 g (48 mmol) Triethylamin und 1,65 g (14,4 mmol) Methansulfonylchlorid in 25 ml THF wurde 3 h bei RT gerührt. Nach Einengen wurde der Rückstand in Wasser aufgenommen und mit EE extrahiert. Nach Trocknen und Einengen wurde das Produkt mit PE zur Kristallisation gebracht und man erhielt 2,25 g 6-Butoxy-4-(methylsulfonyl)amino-2,2-dimethylchroman; F.p. 123 -127°C.
e) 4-[(6-Butoxy-2,2-dimethyl-chroman-4-yl)-methansulfonyl-amino]-N-essigsäureethylester.
   Zu einer Lösung von 1,16g (29 mmol) NaH in 80 ml DMA wurden unter Stickstoffatmosphäre unter Rühren portionsweise 5,9 g (18 mmol) 6-Butoxy-4-(methylsulfonyl)amino-2,2-dimethylchroman hinzugegeben und 30 min bei 60-70°C gerührt. Dann wurden bei 40°C 2,53 ml (22 mmol) Bromessigsäureethylester hinzugetropft und 120 min auf 110°C erhitzt. Nach dem Abkühlen wurde i. Vak. eingeengt, der Rückstand mit 1n wäßriger Salzsäure behandelt, in EE aufgenommen, getrocknet, eingeengt und der Rückstand mit Heptan/EE 3:1 an Kieselgel chromatographiert. Entsprechende Fraktionen wurden mit Diisopropylether zur Kristallisation gebracht; 5,2 g, F.p. 124-126°C.
f) 5,2 g (12,5 mmol) 4-[(6-Butoxy-2,2-dimethyl-chroman-4-yl)-methansulfonyl-amino]-N-essigsäureethylester wurden in 100 ml THF bei 0°C mit 10 ml 1 M Lösung von Lithiumaluminiumhydrid in THF versetzt. Bei RT versetzte man mit wenig Wasser, dann mit 1n Salzsäure, engte ein und extrahierte den Rückstand 3mal mit EE. Nach Trocknen und Eineengen wurden 4,59 g des entsprechenden Alkohols mit Diisopropylether zur Kristallisation gebracht; F.p. 106-108°C.
g) 1,5 g (4 mmol) des vorstehenden Alkohols wurden in 60 ml wasserfreiem Toluol mit 1,9 g (6 mmol) Phosphazen-Base-P1 versetzt und 30 min auf 80-90°C erhitzt. Dann wurden 0,54 g (4,8 mmol) Bromessigsäureethylester zugegeben, 90 min zum Rückfluß erhitzt, DC-Kontrolle, weitere Zugabe von 1,9 ml Phosphazen-Base-P1 und 1 ml Bromessigsäureethylester und 2 h Erhitzen zum Rückfluß. Nach Aufarbeitung ( Einengen, wäßrige Salzsäure, EE, Trocknen) wurde der Rückstand mit Toluol/EE 15:1 an Kieselgel chromatographiert und man erhielt 670 mg öliges Produkt.

### Beispiel 42: N-(6-Butoxy-2,2-dimethyl-chroman-4-yl)-N-[2-(2-methoxy-ethoxy)-ethyl]-methansulfonamid

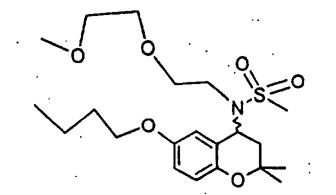

a) 1,37 (3 mmol) des Esters aus Beispiel 41g wurden in 50 ml THF bei 0°C mit 3 ml 1 M Lösung von Lithiumaluminiumhydrid in THF versetzt und 1 h bei RT gerührt. Dann versetzte man mit wenig Wasser, anschließend mit 1n Salzsäure, engte ein und extrahierte den Rückstand 3mal mit EE. Nach Trocknen und Einengen wurde das Rohprodukt mit Heptan/EE 1:1 an Kieselgel chromatographiert, 0,77 g öliges Produkt, das nach 2 Tagen bei RT kristallisierte; F.p. 70-72°C.
b) Die Titelverbindung wurde erhalten, indem man 0,623 g (1,5 mmol) des vorstehenden Alkohols in 20 ml DMA mit 80 mg (2 mmol) NaH 20 min bei 80°C rührte, bei RT 0,6 ml (10,2 mmol) Methyljodid zugab und dann 1 h bei 95-100°C erhitzte. Nach DC-Kontrolle erfolgte weitere Zugabe von 40 mg NaH und 1 ml Methyljodid und weitere 90 min Erhitzen auf 110-115°C. Das Rohprodukt wurde mit Heptan/EE an Kieselgel gereinigt und man erhielt 85 mg öliges Produkt.

### Beispiel 43: {2-[(6-Butoxy-2,2-dimethyl-chroman-4-yl)-methansulfonyl-amino]-ethoxy}-essigsäure

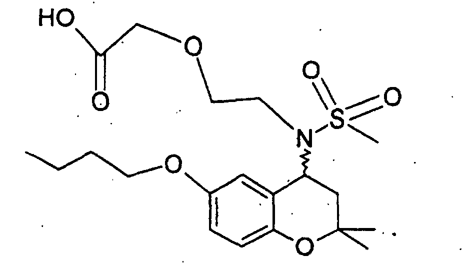

0,6 g {2-[(6-Butoxy-2,2-dimethyl-chroman-4-yl)-methansulfonyl-amino]-ethoxy}-essigsäureethylester (Beispiel 41g) wurden in wenig Methanol gelöst und mit einer Lösung von 3,6 g Lithiumhydroxid in 100 ml Methanol/Wasser 3:1 60 min bei RT gerührt. Nach Aufarbeitung (wäßrige Salzsäure, EE) erhielt man 0,44 g der harzigen Titelverbindung, die nach mehrtägigem Stehen kristallisierte; F.p. 82-84°C.

### Beispiel 44: {3-[(6-Butoxy-2,2-dimethyl-chroman-4-yl)-methansulfonyl-amino]-propoxy}-essigsäureethylester

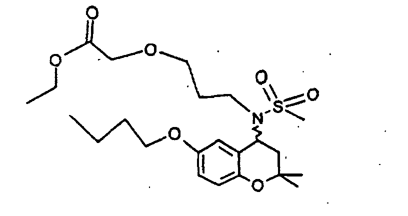

a) N-(6-Butoxy-2,2-dimethyl-chroman-4-yl)-N-[3-benzyloxy-propyl]-methansulfonamid
   Zu 1,96 g (6 mmol) 6-Butoxy-4-(methylsulfonyl)amino-2,2-dimethylchroman (Beispiel 41d) in 20 ml DMA wurden 320 mg (ca. 8 mmol) NaH hinzugegeben und es wurde 30 min bei 70-80°C gerührt. Bei 50°C wurden dann 1,26 ml (ca. 7,5 mmol) 3-Benzyloxy-1-propylbromid zugegeben und 3,5 h bei 110°C gerührt. Nach Aufarbeitung (Wasser, Salzsäure, EE) wurde nach Chromatographie mit Toluol/EE 5:1 an Kieselgel aus entsprechenden Fraktionen 2,7 g Öl erhalten.
b) N-(6-Butoxy-2,2-dimethyl-chroman-4-yl)-N-[3-hydroxy-propyl]-methansulfonamid 2,5 g der vorstehenden Benzylverbindung wurden in THF/Methanol 1:1 mit Pd/Kohle (10%) in der Schüttelente hydriert (Aufnahme ca. 170 ml Wasserstoff). Vom Katalysator wurde abgesaugt, eingeengt und der Rückstand mit DiiE/PE 1:2 zur Kristallisation gebracht; 1,9 g, F.p. 69-71°C.
c) 1,6 g (4,15 mmol) des vorstehenden Alkohols wurden in 50 ml Toluol mit 1,4 g (4,5 mmol) Phosphazen-Base-P1 erhitzt und nach Zugabe von 0,54 ml (4,8 mmol) Bromessigsäureethylester 2 h zum Rückfluß erhitzt. Nach Aufarbeitung wurde nicht umgesetztes Edukt durch Chromatographie mit Toluol/EE 15:1 an Kieselgel abgetrennt. Man erhielt 0,5 g der öligen Titelverbindung, die beim Stehen bei RT fest wurde; F.p. 67-69°C.

### Beispiel 45: N-(6-Butoxy-2,2-dimethyl-chroman-4-yl)-N-[3-(2-hydroxy-ethoxy)-propyl]-methansulfonamid

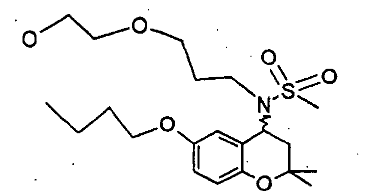

1,25 g (2,5 mmol) des vorstehenden Esters (Beispiel 44) wurden in 40 ml THF bei 0°C mit 3,5 ml 1 M Lösung von Lithiumaluminiumhydrid in THF versetzt und 1 h bei RT gerührt. Dann versetzte man mit wenig Wasser, anschließend mit 1n Salzsäure, engte ein und extrahierte den Rückstand 2mal mit EE. Nach Trocknen und Einengen wurde das Rohprodukt mit Heptan/EE 1:2 an Kieselgel chromatographiert. Man erhielt 0,6 g der öligen Titelverbindung, die beim Stehen bei RT wachsartig wurde.

### Beispiel 46: {3-[(6-Butoxy-2,2-dimethyl-chroman-4-yl)-methansulfonyl-amino]-propoxy}-essigsäure

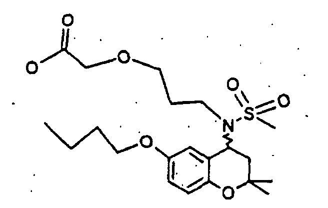

Zu einer klaren Lösung von 2,4 g Lithiumhydroxid in 100 ml Methanol/Wasser 3:1 wurden 0,48 g des Esters aus Beispiel 45 hinzugegeben. Nach 1 h bei RT wurde eingeengt, die wäßrige Lösung mit Diethylether extrahiert, mit wäßriger Salzsäure angesäuert, die kristalline Fällung abgesaugt, mit Wasser gewaschen und getrocknet. Man erhielt 0,36 g der Titelverbindung; F.p. 79-81°C.

### Beispiel 47: (2-Morpholin-4-yl-ethyl)-carbaminsäure-3-[(6-butoxy-2,2-dimethyl-chroman-4-yl)-methansulfonyl-amino]-propylester

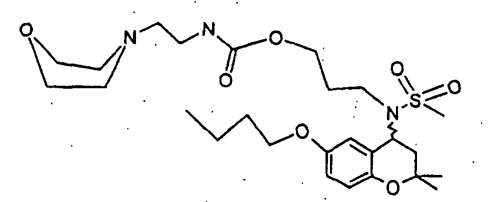

0,77 g (2 mmol) N-(6-Butoxy-2,2-dimethyl-chroman-4-yl)-N-[3-hydroxy-propyl]-methansulfonamid (Beispiel 44 b) wurden in 50 ml THF unter Rühren bei RT mit 96 mg (2,4 mmol) NaH versetzt und 5 min auf 50°C erwärmt. Diese Lösung wurde nach Abkühlen zu einer Lösung von 347 mg (2,1 mmol) N,N'-Carbonyldiimidazol in 40 ml THF getropft und 30 min zum Rückfluß erhitzt. Dann wurden 780 mg (6 mmol) 2-Aminoethylmorpholin in 5 ml THF zugetropft, 2 h zum Rückfluß erhitzt, eingeengt, mit Wasser behandelt, mit Diethylether extrahiert, die etherische Phase mit wäßriger Salzsäure gewaschen, die saure Phase auf pH 8 gebracht, mit EE extrahiert und man erhielt 0,7 g harziges Produkt.

### Beispiel 48: 5-[Methansulfonyl-(2,2,6-trimethyl-chroman-4-yl)-amino]-pentansäure-guanidid-hydrochlorid.

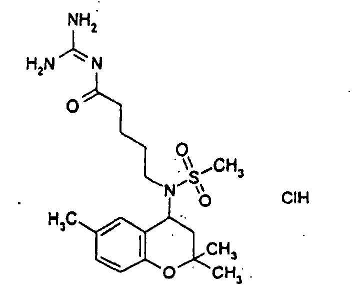

Eine Mischung aus 0,5 g (1,4 mmol) 5-[Methylsulfonyl-(2,2,6-trimethyl-chroman-4-yl)-amino]-pentansäure (Beispiel 10c) und 0,26 g (1,6 mmol) CDI in 20 ml THF wurde 3 h bei RT gerührt. Dann wurden 0,45 g (8 mmol) Guanidin zugegeben und über Nacht bei RT weiter gerührt. Nach Einengen der Reaktionsmischung wurde der Rückstand mit 50 ml Wasser versetzt, und über Nacht bei RT gerührt. Das ausgefallene Produkt wurde abgesaugt und in das Hydrochlorid überführt. Man erhielt 0,2 g 5-[Methansulfonyl-(2,2,6-trimethyl-chroman-4-yl)-amino]-pentansäure-guanidid-hydrochlorid; F.p. 190-195°C.

### Beispiel 49: [Ethansulfonyl-(6-fluor-2,2-dimethyl-chroman-4-yl)-amino]-essigsäure-2-(benzyl-methyl-amino)-ethylester

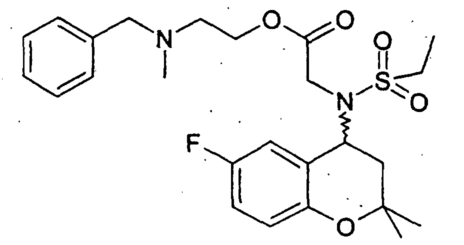

Analog Beispiel 7 erhält man aus 310 mg (0,9 mmol) [Ethylsulfonyl-(6-fluor-2,2-dimethyl-chroman-4-yl)-amino]-essigsäure und 165 mg (1,0 mmol) 2-(Benzyl-methyl-amino)-ethanol 270 mg (67%) [Ethansulfonyl-(6-fluor-2,2-dimethyl-chroman-4-yl)-amino]-essigsäure-2-(benzyl-methyl-amino)-ethylester als Feststoff (Smp.

### Beispiel 50: 2-[Ethansulfonyl-(6-fluor-2,2-dimethyl-chroman-4-yl)-amino]-N-(2-ethyl-2H-pyrazol-3- yl)-acetamid

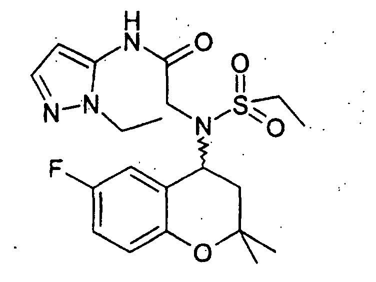

Analog Beispiel 22 erhält man aus 380 mg (1,0 mmol) [Ethylsulfonyl-(6-fluor-2,2-dimethyl-chroman-4-yl)-amino]-essigsäure-chlorid und 111 mg (1mmol) 5-Amino-1-ethyl-pyrazol 440mg (96 %) 2-[Ethansulfonyl-(6-fluor-2,2-dimethyl-chroman-4-yl)-amino]-N-(2-ethyl-2H-pyrazol-3- yl)-acetamid als Feststoff (Smp. 69°C).

### Beispiel 51: N-(1H-Benzoimidazol-2-ylmethyl)-2-[ethansulfonyl-(6-fluor-2,2-dimethyl-chroman-4-yl)-amino]-acetamid

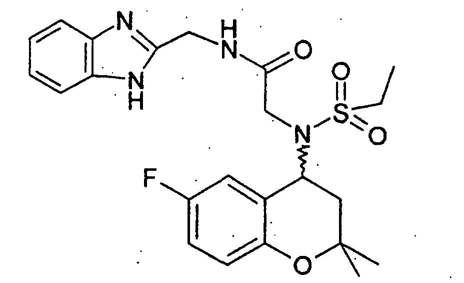

Analog Beispiel 22 erhält man aus 420 mg (1,1 mmol) [Ethylsulfonyl-(6-fluor-2,2-dimethyl-chroman-4-yl)-amino]-essigsäure-chlorid und 220 mg (1mmol) 2-Amino-methyl-benzimidazol 470mg (90 %) N-(1H-Benzoimidazol-2-ylmethyl)-2-[ethansulfonyl-(6-fluor-2,2-dimethyl-chroman-4-yl)-amino]-acetamid als Öl.

### Beispiel 52: 2-[Ethansulfonyl-(6-fluor-2,2-dimethyl-chroman-4-yl)-amino]-N-isothiazol-5-yl-acetamid

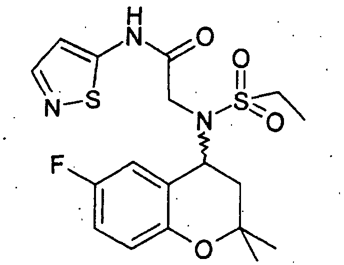

Analog Beispiel 22 erhält man aus 380 mg (1,0 mmol) [Ethylsulfonyl-(6-fluor-2,2-dimethyl-chroman-4-yl)-amino]-essigsäure-chlorid und 100 mg (1mmol) 2-Amino-thiazol 430 mg (96 %) 2-[Ethansulfonyl-(6-fluor-2,2-dimethyl-chroman-4-yl)-amino]-N-isothiazol-5-yl-acetamid als Feststoff (Smp. 167°C).

### Beispiel 53: 2-[Ethansulfonyl-(6-fluor-2,2-dimethyl-chroman-4-yl)-amino]-N-(5-methyl-isoxazol-3-yl)-acetamid

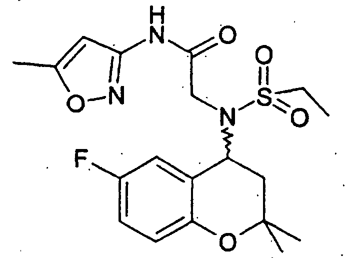

Analog Beispiel 22 erhält man aus 380 mg (1,0 mmol) [Ethylsulfonyl-(6-fluor-2,2-dimethyl-chroman-4-yl)-amino]-essigsäure-chlorid und 98 mg (1mmol) 3-Amino-5-methyl-isoxazol 425mg (95 %) 2-[Ethansulfonyl-(6-fluor-2,2-dimethyl-chroman-4-yl)-amino]-N-(5-methyl-isoxazol-3-yl)-acetamid als Feststoff (Smp. 183°C).

### Beispiel 54: 2-[Ethansulfonyl-(6-fluor-2,2-dimethyl-chroman-4-yl)-amino]-N-(1H-[1,2,4]triazol-3-yl)-acetamid

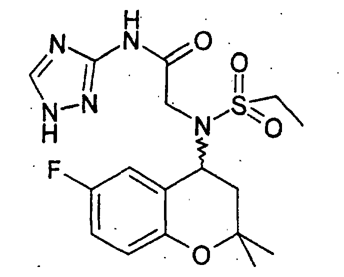

Analog Beispiel 22 erhält man aus 1,1 g (3,0 mmol) [Ethylsulfonyl-(6-fluor-2,2-dimethyl-chroman-4-yl)-amino]-essigsäure-chlorid und 210 mg (2,5 mmol) 3-Amino-triazol 610 mg (60 %) 2-[Ethansulfonyl-(6-fluor-2,2-dimethyl-chroman-4-yl)-amino]-N-(1H-[1,2,4]triazol-3-yl)-acetamid als Öl.

### Beispiel 55: 2-[Ethansulfonyl-(6-fluor-2,2-dimethyl-chroman-4-yl)-amino]-N-[2-(4-methoxy-3-sulfamoyl-phenyl)-ethyl]-acetamid

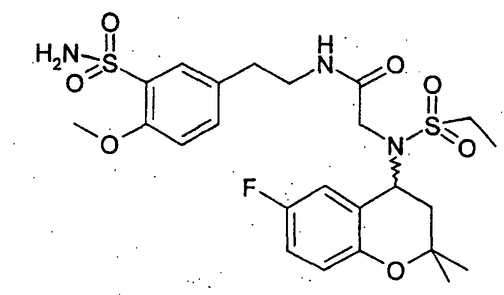

Analog Beispiel 22 erhält man aus 380 mg (1,0 mmol) [Ethylsulfonyl-(6-fluor-2,2-dimethyl-chroman-4-yl)-amino]-essigsäure-chlorid und 270 mg (1,0 mmol) 5-(2-Amino-ethyl)-2-methoxy-benzolsulfonamid-hydrochlorid 140 mg (25%) 2-[Ethansulfonyl-(6-fluor-2,2-dimethyl-chroman-4-yl)-amino]-N-[2-(4-methoxy-3-sulfamoyl-phenyl)-ethyl]-acetamid als Öl.

### Beispiel 56: N-(6-Butoxy-2,2-dimethyl-chroman-4-yl)-N-(2-guanidino-2-oxo-ethyl)-methansulfonamid

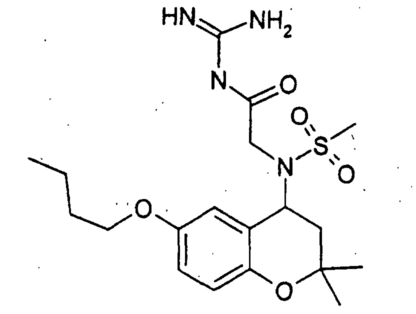

a) [(6-Butoxy-2,2-dimethyl-chroman-4-yl)-methansulfonyl-amino]-essigsäure Durch Verseifung von [(6-Butoxy-2,2-dimethyl-chroman-4-yl)-methansulfonyl-amino]-essigsäureethylester (Beispiel 41e) wurde die entsprechende Säure erhalten; F.p. 108-110°C.
b) Eine Suspension von 0,77 g (2 mmol) [(6-Butoxy-2,2-dimethyl-chroman-4-yl)-methansulfonyl-amino]-essigsäure und 0,39 g (2,4 mmol) CDI in 10 ml THF wurde über Nacht bei RT gerührt. Nach Zugabe von 0,59 g (10 mmol) Guanidin wurde noch 3 h gerührt und dann die Reaktionsmischung im Vakuum eingeengt. Durch Verrühren des Rückstandes mit 50 ml Wasser über Nacht, wurde ein kristallines Produkt erhalten, das abgesaugt und im Vakuum getrocknet wurde. Man erhielt 0,8 g N-(6-Butoxy-2,2-dimethyl-chroman-4-yl)-N-(2-guanidino-2-oxo-ethyl)-methansulfonamid; F.p. 112 - 114 °C.

### Beispiel 57: N-(6-Fluor-2,2-dimethyl-chroman-4-yl)-N-(2-guanidino-2-oxo-ethyl)-ethansulfonamid

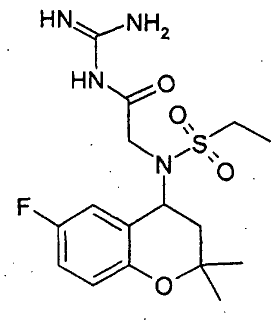

Analog Beispiel 56 erhielt man aus der entsprechenden Säure (Beispiel 7b) 310mg (91%) N-(6-Fluor-2,2-dimethyl-chroman-4-yl)-N-(2-guanidino-2-oxo-ethyl)-ethansulfonamid als Feststoff (Smp. 192°C).

### Beispiel 58: 2-[(6-Chlor-3-hydroxy-2,2-dimethyl-chroman-4-yl)-methansulfonylamino]-N-phenethyl- acetamid

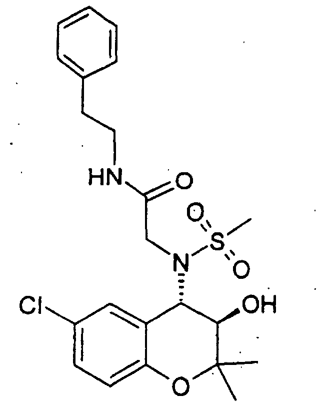

a) N-(6-Chlor-3-hydroxy-2,2-dimethyl-chroman-4-yl)-methansulfonamid
   Eine Lösung von 2,94 g (31 mmol) Methansulfonsäureamid in 12 ml DMSO wurde mit 0,71 g (24 mmol) 80proz. Natriumhydrid versetzt und 1 h bei RT gerührt. Dann fügte man 5,0 g (24 mmol) 6-Chlor-2,2-dimethyl-3,4-epoxy-chroman (J. Med. Chem. 26, 1983, 1582) hinzu und erhitzte 20 h auf 60°C. Man versetzte den Ansatz mit 50 ml Wasser, rührte 1 h nach, saugte das ausgefallene Produkt ab und erhielt 5,9 g N-(6-Chlor-3-hydroxy-2,2-dimethyl-chroman-4-yl)-methansulfonamid; F.p. 198-202°C.
b) [(6-Chlor-3-hydroxy-2,2-dimethyl-chroman-4-yl)-methansulfonyl-amino]-essigsäuremethylester
   Zu einer Lösung von 0,33 g (11,3 mmol) 80proz. Natriumhydrid in 25 ml DMF wurde eine Lösung von 3,0 g (10 mmol) N-(6-Chlor-3-hydroxy-2,2-dimethyl-chroman-4-yl)-methansulfonamid in 40 ml DMF zugetropft und noch 1 h bei RT nachgerührt. Dann wurden 1,53 g (10 mmol) Bromessigsäuremethylester zugegeben, über Nacht bei RT gerührt und dann das Lösungsmittel im Vakuum abgezogen. Der Rückstand wurde in EE und Wasser aufgenommen, die organische Phase wurde eingeengt und das Produkt durch Chromatographie an Kieselgel mit Cyclohexan/Essigester 9:1 gereinigt. Es wurden 2,0 g [(6-Chlor-3-hydroxy-2,2-dimethyl-chroman-4-yl)-methansulfonyl-amino]-essigsäuremethylester erhalten; F.p. 148-150°C.
c) [(6-Chlor-3-hydroxy-2,2-dimethyl-chroman-4-yl)-methansulfonyl-amino]-essigsäure
   Durch Hydrolyse des obigen Methylesters mit KOH in Methanol/Wasser bei RT übe Nacht wurde die entsprechende Säure erhalten; F.p. 163-167°C.
d) Eine Lösung von 0,5 g (1,37 mmol) [(6-Chlor-3-hydroxy-2,2-dimethyl-chroman-4-yl)-methansulfonyl-amino]-essigsäure und 0,27 g (1,65 mmol) CDI in 7 ml THF wurde 3 h bei RT gerührt. Dann wurden 0,33 g (2,75 mmol) Phenethylamin hinzugefügt und die Reaktionsmischung über Nacht gerührt. Nach dem Abziehen des Lösungsmittels wurde der Rückstand in EE aufgenommen und mit verd. Salzsäure und Wasser gewaschen. Nach Reinigung über eine kurze Chromatographiesäule wurden 0,56 g 2-[(6-Chlor-3-hydroxy-2,2-dimethyl-chroman-4-yl)-methansulfonyl-amino]-N-phenethyl- acetamid erhalten; F.p 168-170°C.

### Beispiel 59: 2-[Ethansulfonyl-(6-fluor-2,2-dimethyl-chroman-4-yl)-amino]-N-[2-(4-fluor-phenyl)- ethyl]-acetamid

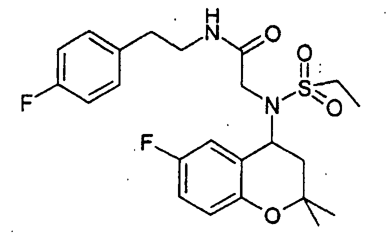

Analog Beispiel 7 wurden 420 mg der Substanz erhalten.

### Beispiel 60: 2-[(6-Chlor-3-hydroxy-2,2-dimethyl-chroman-4-yl)-methansulfonyl-amino]-N,N-dimethyl-acetamid

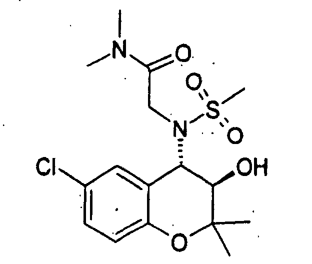

Analog Beispiel 58 wurden 150 mg der Substanz erhaften mit einem Schmelzpunkt von 263°C.

### Beispiel 61: N-(1H-Benzimidazol-2-yl)-2-[ethansulfonyl-(6-fluor-2,2-dimethyl-chroman-4-yl)- amino]-acetamid

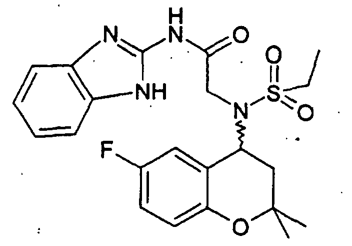

Analog Beispiel 7 wurden 340 mg der Substanz erhalten mit einem Schmelzpunkt von 127 - 133°C.

### Beispiel 62: N-[2-(Benzyl-methyl-amino)-ethyl]-2-[ethansulfonyl-(6-fluor-2,2-dimethyl-chroman-4-yl)-amino]-acetamid

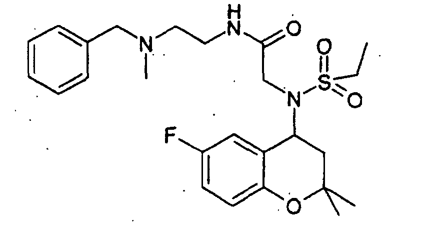

Die Verbindung wurde erhalten analog Beispiel 7 aus N-Benzyl-N-methyl-ethan-1,2-diamin (Arzneim. Forsch. 25, 1975, 1853) und der entsprechenden Säure (Beispiel 7c). Nach Reinigung durch Chromatographie wurden 280 mg als Öl erhalten.

### Pharmakologische Untersuchungen

I_{sK}-Kanäle aus Mensch, Ratte oder Meerschweinchen wurden in Xenopus-Oozyten exprimiert. Hierfür wurden zuerst Oozyten aus Xenopus Laevis isoliert und defollikuliert. Anschließend wurde in diese Oozyten in vitro synthetisierte I_{sK}-kodierende RNA injiziert. Nach 2 - 8 Tagen I_{sK}-Proteinexpression wurden an den Oozyten mit der Zwei-Mikroelektroden Voltage-Clamp Technik I_{sK}-Ströme gemessen. Die I_{sK}-Kanäle wurden hierbei in der Regel mit 15 s dauernden Spannungssprüngen auf -10 mV aktiviert. Das Bad wurde mit einer Lösung der nachfolgenden Zusammensetzung durchspült: NaCI 96 mM, KCI 2 mM, CaCl₂ 1,8 mM, MgCl₂ 1 mM, HEPES 5 mM (titriert mit NaOH auf pH 7,5). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADInstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des I_{sK}-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

### Literatur:

A.E. Busch, H.-G. Kopp, S. Waldegger, I. Samarzija, H. Süßbrich, G. Raber, K. Kunzelmann, J. P. Ruppersberg und F. Lang; "Inhibition of both exogenously expressed I_{sK} and endogenous K⁺ channels in Xenopus oocytes by isosorbide dinitrate"; J. Physiol. 491 (1995), 735-741;
T. Takumi, H. Ohkubo und S. Nakanishi; "Cloning of a membrane protein that induces a slow voltage-gated potassium current"; Science 242 (1989), 1042-1045; M.D. Varnum, A.E. Busch, C.T. Bond, J. Maylie und J.P. Adelman; "The minK channel underlies the cardiac potassium current and mediates species-specific responses to protein kinase"; C. Proc. Natl. Acad. Sci. USA 90 (1993), 11528-11532.

Auf die beschriebene Weise wurden unter Verwendung des humanen I_{sK}-Proteins für die erfindungsgemäßen Verbindungen folgende IC₅₀-Werte bestimmt:

| Verbindung | IC-₅₀ [µM] |
|---|---|
| Beispiel 4 | 1,0 |
| Beispiel 9 | 1,6 |
| Beispiel 10 | 0,79 |
| Beispiel 14 | 0,18 |
| Beispiel 17 | << 1 |
| Beispiel 21 | 0,046 |
| Beispiel 22 | 0,67 |
| Beispiel 23 | 0,55 |
| Beispiel 24 | 1,0 |
| Beispiel 25 | 0,45 |
| Beispiel 27 | 2,1 |
| Beispiel 28 | 0,47 |
| Beispiel 29 | < 1 |
| Beispiel 31 | 0,28 |
| Beispiel 32 | 0,25 |
| Beispiel 34 | ∼ 0,7 |
| Beispiel 38 | 1,42 |
| Beispiel 39 | 0,31 |
| Beispiel 40 | 0,35 |
| Beispiel 42 | 0,34 |
| Beispiel 43 | 7,8 |
| Beispiel 45 | 0,45 |
| Beispiel 47 | 0,75 |
| Beispiel 49 | 0,083 |
| Beispiel 49 | 0.083 |
| Beispiel 54 | 0.83 |
| Beispiel 56 | 3.4 |
| Beispiel 58 | 0.5 |
| Beispiel 59 | < 0.1 |
| Beispiel 61 | < 1 |
| Beispiel 62 | < 1 |

## Patentansprüche

1. Verbindungen der Formel I, 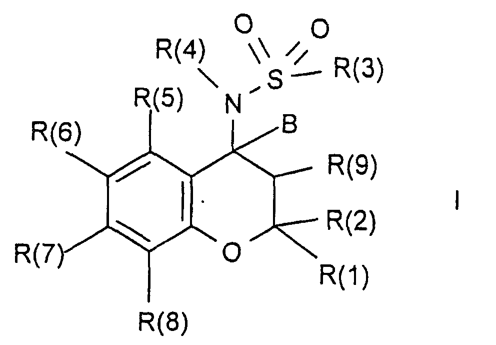 worin bedeuten:
R(1) und R(2) unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(1) und R(2) gemeinsam eine Alkylenkette mit 2, 3, 4, 5, 6, 7, 8, 9 oder 10 C-Atomen;
R(3) R(10)-CₙH₂ₙ-NR(11)- oder R(10)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-;
R(12a) Wasserstoff, Methyl oder Ethyl;
R(10) Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10) und R(11) gemeinsam eine Bindung, sofern n nicht kleiner als 3 ist;
R(4) R(13)-CᵣH₂ᵣ-Z-C_{q}H_{2q}-;
q 0, 1, 2, 3, 4, 5, 6, 7 oder 8;
r 0, 1, 2, 3, 4, 5, 6, 7 oder 8;
Z -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- oder
-CO-O-CₓH₂ₓ-NR(14)-,
wobei jeweils beide Verknüpfungsrichtungen möglich sind;
x 2, 3 oder 4;
R(14) Wasserstoff, Alkyl mit 1, 2 oder 3 C-Atomen,
-C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, C₃F₇, Cycloalkyl mit 3, 4, 5, 6, 7 oder 8 C-Atomen, -NR(15)R(16), -CONR(15)R(16), -C(=NR(17))NR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(15) und R(16) unabhängig voneinander Wasserstoff, Alkyl mit 1, 2, 3 oder 4 C-Atomen oder -C_{z}H_{2z}-phenyl,
worin Phenyl unsubstituiert ist oder substituiert mit 1 or 2 Substituenten ausgewählt aus der Gruppe bestehend aus F, Cl, Br, CF₃, NO₂, CN OH, Methyl, Ethyl, Methoxy, Dimethylamino, Sulfamoyl, Methylsulfonyl, Aminosulfonyl und Methylsulfonylamino;
oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff oder Alkyl mit 1, oder 3 C-Atomen;
R(5), R(6), R(7) und R(8) unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, -CN, -CF₃, -C₂F₅, -C₃F₇, -N₃, -NO₂, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -SO₂-O-, -SO₂NR(10c), -NR(10c)- oder -CONR(1 0c)-;
R(10c) Wasserstoff oder Alkyl mit 1, 2 oder 3 C-Atomen;
s Null, 1, 2, 3, 4, 5 oder 6;
R(18) Wasserstoff, CF₃, C₂F₅, C₃F₇, Cycloalkyl mit 3, 4, 5, 6, 7 oder 8 C-Atomen, -COOR(21), 1-Piperidyl, 1-Pyrrolidinyl, 4-Morpholinyl, 4-Methylpiperazin-1-yl, Pyridyl, Thienyl, Imidazolyl, Chinolyl, Isochinolyl oder Phenyl,
R(21) Wasserstoff oder Alkyl mit 1, 2 oder 3 C-Atomen;
R(9) Wasserstoff, OR(10d) oder OCOR(10d);
R(10d) Wasserstoff oder Alkyl mit 1, 2 oder 3 C-Atomen;
B Wasserstoff;
oder
R(9) und B gemeinsam eine Bindung;
sowie ihre physiologisch verträglichen Salze.

2. Verbindungen der Formel I nach Anspruch 1, in der bedeuten:
R(1) und R(2) unabhängig voneinander Wasserstoff, CF₃ oder Alkyl mit 1, 2, 3, 4, 5 oder 6 C-Atomen;
oder
R(1) und R(2) gemeinsam eine Alkylenkette mit 2, 3, 4, 5 oder 6 C-Atomen;
R(3) R(10)-CₙH₂ₙ-;
R(10) Methyl, CF₃ oder C₂F₅;
n Null, 1, oder 2;
R(4) R(13)-CᵣH₂ᵣ-Z-C_{q}H_{2q}-;
q 1, 2, 3, 4, 5, 6, 7 oder 8;
r 0, 1, 2, 3, 4, 5, 6, 7 oder 8;
Z -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- oder
-CO-O-CₓH₂ₓ-NR(14)-,
wobei jeweils beide Verknüpfungsrichtungen möglich sind;
x 2, 3 oder 4;
R(14) Wasserstoff, Alkyl mit 1, 2 oder 3 C-Atomen,
-C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, C₃F₇, Cycloalkyl mit 3, 4, 5, 6, 7 oder 8 C-Atomen, -NR(15)R(16), -CONR(15)R(16), -C(=NR(17))NR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(15) und R(16) unabhängig voneinander Wasserstoff, Alkyl mit 1, 2, 3 oder 4 C-Atomen oder -C_{z}H_{2z}-phenyl,
worin Phenyl unsubstituiert ist oder substituiert mit 1 or 2 Substituenten ausgewählt aus der Gruppe bestehend aus F, Cl, Br, CF₃, NO₂, CN OH, Methyl, Ethyl, Methoxy, Dimethylamino, Sulfarnoyl, Methylsulfonyl, Aminosulfonyl und Methylsulfonylamino;
oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff oder Alkyl mit 1, 2 oder 3 C-Atomen;
R(5), R(6), R(7) und R(8) unabhängig voneinander Wasserstoff, F, Cl, Br, I, Alkyl mit 1, 2, 3, 4 oder 5 C-Atomen, Cycloalkyl mit 3, 4, 5, 6 oder 7 C-Atomen, -CN, -CF₃, -C₂F₅, -C₃F₇, -NO₂, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -CO-O-, -O-CO-, -S-, -SO-, -SO₂-, -SO₂-O-, -SO₂NR(10c),
-NR(10c)- oder -CONR(10c)-;
R(10c) Wasserstoff oder Alkyl mit 1, 2 oder 3 C-Atomen;
s Null, 1, 2, 3, 4, 5 oder 6;
R(18) Wasserstoff, CF₃, C₂F₅, C₃F₇, Cycloalkyl mit 3, 4, 5, 6 oder 7 C-Atomen, -COOR(21), 1-Piperidyl, 1-Pyrrolidinyl, 4-Morpholinyl, 4-Methylpiperazin-1-yl, Pyridyl, Imidazolyl oder Phenyl,
R(21) Wasserstoff oder Alkyl mit 1, 2 oder 3 C-Atomen;
R(9) Wasserstoff oder OR(10d);
R(10d) Wasserstoff oder Methyl;
B Wasserstoff;
oder
R(9) und B gemeinsam eine Bindung;
sowie ihre physiologisch verträglichen Salze.

3. Verbindungen der Formel I nach Ansprüchen 1 bis 2, in der bedeuten:
R(1) und R(2) unabhängig voneinander Wasserstoff, CF₃ oder Alkyl mit 1, 2 oder 3 C-Atomen;
oder
R(1) und R(2) gemeinsam eine Alkylenkette mit 2, 3, 4 oder 5 C-Atomen;
R(3) R(10)-CₙH₂ₙ-;
R(10) Methyl, CF₃ oder C₂F₅;
n Null, 1, oder 2;
R(4) R(13)-CᵣH₂ᵣ-Z-C_{q}H_{2q}-;
q 1, 2, 3 oder 4;
r 0, 1, 2 oder 3;
Z -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- oder
-CO-O-CₓH₂ₓ-NR(14)-,
wobei jeweils beide Verknüpfungsrichtungen möglich sind;
x 2 oder 3;
R(14) Wasserstoff, Alkyl mit 1 oder 2 C-Atomen;
R(13) CH₃, CF₃, C₂F₅, Cycloalkyl mit 3, 4, 5, 6 oder 7 C-Atomen, -NR(15)R(16), -C(=NR(17))NR(15)R(16), -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(15) und R(16) unabhängig voneinander Wasserstoff, Alkyl mit 1, 2, 3 oder 4 C-Atomen oder -C_{z}H_{2z}-phenyl,
worin Phenyl unsubstituiert ist oder substituiert mit 1 or 2 Substituenten ausgewählt aus der Gruppe bestehend aus F, Cl, Br, CF₃, NO₂, CN OH, Methyl, Ethyl, Methoxy, Dimethylamino, Sulfamoyl, Methylsulfonyl, Aminosulfonyl und Methylsulfonylamino;
oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff oder Alkyl mit 1 oder 2 C-Atomen;
R(5) und R(6) unabhängig voneinander Wasserstoff, F, Cl, Br, Alkyl mit 1, 2, 3, 4 oder 5 C-Atomen, Cycloalkyl mit 3, 4, 5, 6 oder 7 C-Atomen, -CN, -CF₃, -C₂F₅, -NO₂, -Y-CₛH₂ₛ-R(18) oder Phenyl,
das unsubstituiert ist oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y -O-, -CO-, -SO₂- oder -CONR(10c)-;
R(10c) Wasserstoff oder Alkyl mit 1 oder 2 C-Atomen;
s Null, 1, 2, 3, 4, 5 oder 6;
R(18) Wasserstoff, CF₃, C₂F₅, Cycloalkyl mit 3, 4, 5, 6 oder 7 C-Atomen, 1-Piperidyl, 1-Pyrrolidinyl, 4-Morpholinyl, 4-Methylpiperazin-1-yl, Pyridyl, Imidazolyl oder Phenyl;
R(7) und R(8) Wasserstoff;
R(9) Wasserstoff oder OR(10d);
R(10d) Wasserstoff oder Methyl;
B Wasserstoff;
oder
R(9) und B gemeinsam eine Bindung;
sowie ihre physiologisch verträglichen Salze.

4. Verbindungen der Formel I nach Ansprüchen 1 bis 3, in der bedeuten:
R(1) und R(2) Methyl;
R(3) Methyl oder Ethyl;
R(4) R(13)-CᵣH₂ᵣ-Z-C_{q}H_{2q}-;
q 1, 2, 3 oder 4;
r 0, 1, 2 oder 3;
Z -CO-NR(14)-,
-OCO-NR(14)-,
-O-CₓH₂ₓ-NR(14)- oder
-CO-O-CₓH₂ₓ-NR(14)-;
x 2 oder 3;
R(14) Wasserstoff oder Methyl;
R(13) CH₃, CF₃, -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(17) Wasserstoff oder Alkyl mit 1 oder 2 C-Atomen;
R(5) Wasserstoff, Alkyl mit 1, 2, 3 oder 4 C-Atomen, F, Cl, Methoxy oder Ethoxy;
R(6) F, Cl, Alkyl mit 1, 2, 3, 4 oder 5 C-Atomen, -CF₃, -Y-CₛH₂ₛ-R(18) oder Phenyl, das unsubstituiert ist oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y -O-, -CO- oder -CONR(10c)-;
R(10c) Wasserstoff oder Methyl;
s 1, 2, 3, 4 oder 5;
R(18) Wasserstoff, CF₃ oder Phenyl;
R(7) und R(8) Wasserstoff;
R(9) Wasserstoff;
B Wasserstoff;
sowie ihre physiologisch verträglichen Salze.

5. Verbindungen der Formel I nach Ansprüchen 1 bis 3, in der bedeuten:
R(1) und R(2) Methyl;
R(3) Methyl oder Ethyl;
R(4) R(13)-CᵣH₂ᵣ-Z-C_{q}H_{2q}-;
q 1, 2, 3 oder 4;
r 0, 1, 2 oder 3;
Z -CO-NR(14)-,
-OCO-NR(14)-,
-O-CₓH₂ₓ-NR(14)- oder
-CO-O-CₓH₂ₓ-NR(14)-;
x 2 oder 3;
R(14) Wasserstoff oder Methyl;
R(13) CH₃, CF₃, -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(17) Wasserstoff oder Alkyl mit 1 oder 2 C-Atomen;
R(5) Wasserstoff, Alkyl mit 1, 2, 3 oder 4 C-Atomen, F, Cl, Methoxy oder Ethoxy;
R(6) F, Cl, Alkyl mit 1, 2, 3, 4 oder 5 C-Atomen, -CF₃, -Y-CₛH₂ₛ-R(18) oder Phenyl, das unsubstituiert ist oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
Y -O-, -CO- oder -CONR(10c)-;
R(10c) Wasserstoff oder Methyl;
s 1, 2, 3, 4 oder 5;
R(18) Wasserstoff, CF₃ oder Phenyl;
R(7) und R(8) Wasserstoff;
R(9) OH;
B Wasserstoff;
sowie ihre physiologisch verträglichen Salze.

6. Verbindungen der Formel I nach Ansprüchen 1 bis 3, in der bedeuten:
R(1) und R(2) Methyl;
R(3) Methyl oder Ethyl;
R(4) R(13)-CᵣH₂ᵣ-Z-C_{q}H_{2q}-;
q 1, 2, 3 oder 4;
r 0, 1, 2 oder 3;
Z -CO-NR(14)-,
-OCO-NR(14)-,
-O-CₓH₂ₓ-NR(14)- oder
-CO-O-CₓH₂ₓ-NR(14)-;
x 2 oder 3;
R(14) Wasserstoff oder Methyl;
R(13) CH₃, CF₃, -OR(17), -COOR(17), Phenyl oder ein N-haltiger Heterocyclus mit 1, 2, 3, 4, 5, 6, 7, 8 oder 9 C-Atomen,
wobei Phenyl und der N-haltige Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl, Aminosulfonyl und Methylsulfonylamino;
R(17) Wasserstoff oder Alkyl mit 1 oder 2 C-Atomen;
R(5) Wasserstoff, Alkyl mit 1, 2, 3 oder 4 C-Atomen, F, Cl, Methoxy oder Ethoxy;
R(6) F, Cl, Alkyl mit 1, 2, 3, 4 oder 5 C-Atomen, -CF₃, -Y-CₛH₂ₛ-R(18) oder Phenyl, das unsubstituiert ist oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonyfamino;
Y -O-, -CO- oder -CONR(10c)-;
R(10c) Wasserstoff oder Methyl;
s 1, 2, 3, 4 oder 5;
R(18) Wasserstoff, CF₃ oder Phenyl;
R(7) und R(8) Wasserstoff;
R(9) und B gemeinsam eine Bindung;
sowie ihre physiologisch verträglichen Salze.

7. Verbindungen der Formel I nach Anspruch 1 und ihre physiologisch verträglichen Salze zur Anwendung als Arzneimittel.

8. Pharmazeutische Zubereitung, enthaltend eine wirksame Menge mindestens einer Verbindung der Formel I nach einem oder mehreren der Ansprüche 1 bis 6 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

9. Verwendung einer Verbindung der Formel I nach einem oder mehreren der Ansprüche 1 bis 6 und/oder eines physiologisch verträglichen Salzes davon zur Herstellung eines Medikaments mit K⁺-Kanal-blockierender Wirkung zur Therapie und Prophylaxe von K⁺-Kanal mediierten Krankheiten.

10. Verwendung einer Verbindung der Formel I nach einem oder mehreren der Ansprüche 1 bis 6 und/oder eines physiologisch verträglichen Salzes davon zur Herstellung eines Medikaments zum Inhibieren der Magensäuresekretion.

11. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Ulcera des Magens oder des intestinalen Bereiches.

12. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe der Refluxösophagitis.

13. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Durchfallerkrankungen.

14. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe aller Typen von Arrhythmien, einschließlich atrialer, ventrikulärer und supraventrikulärer Arrhythmien.

15. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

16. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von atrialer Fibrillation oder atrialem Flattern.

17. Verwendung einer Verbindung der Formel I nach einem oder mehreren der Ansprüche 1 bis 6 und/oder eines physiologisch verträglichen Salzes davon zur Herstellung eines Medikaments zur Therapie oder Prophylaxe von Reentry-Arrhythmien oder zur Verhinderung des plötzlichen Herztodes infolge von Kammerflimmern.

18. Verwendung einer Verbindung der Formel I nach einem oder mehreren der Ansprüche 1 bis 6 und/oder eines physiologisch verträglichen Salzes davon zur Herstellung eines Medikaments zur Therapie der Herzinsuffizienz.

19. Verwendung einer Verbindung der Formel I nach einem oder mehreren der Ansprüche 1 bis 6 und/oder eines physiologisch verträglichen Salzes davon zum Herstellen eines Medikaments zum Inhibieren der stimulierten Magensäuresekretion, zur Therapie oder Prophylaxe von Ulcera des Magens oder des intestinalen Bereiches, der Refluxösophagitis, von Durchfallerkrankungen, zur Therapie oder Prophylaxe von Arrhythmien, einschließlich atrialer, ventrikulärer und supraventrikulärer Arrhythmien, atrialer Fibrillation und atrialem Flattern und von Reentry-Arrhythmien, oder zur Verhinderung des plötzlichen Herztodes infolge von Kammerflimmern.

## Claims

1. A compound of the formula I. 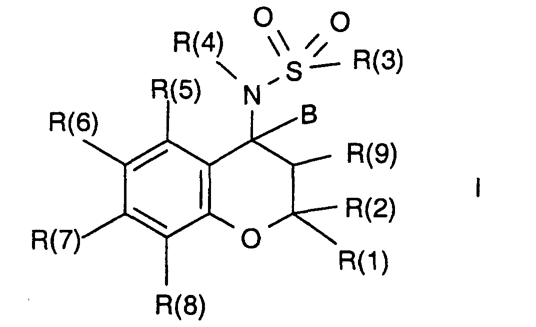 in which:
R(1) and R(2) independently of one another are hydrogen, CF₃, C₂F₅, C₃F₇, alkyl having 1, 2, 3, 4, 5 or 6 carbon atoms, or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(1) and R(2) together are an alkylene chain having 2, 3, 4, 5, 6, 7, 8, 9 or 10 carbon atoms;
R(3) is R(10)-CₙH₂ₙ-NR(11)- or R(10)-CₙH₂ₙ-,
where one CH₂ group in the groups CₙH₂ₙ can be replaced by -O-, -CO-, -S-, -SO-, -SO₂- or -NR(12a)-;
R(12a) is hydrogen, methyl or ethyl;
R(10) is hydrogen, methyl, cycloalkyl having 3, 4, 5, 6, 7 or 8 carbon atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 carbon atoms;
or
R(10) and R(11) together are a bond, provided n is not smaller than 3;
R(4) is R(13)-CᵣH₂ᵣ-Z-C_{q}H_{2q}-;
q is 0, 1, 2, 3, 4, 5, 6, 7 or 8;
r is 0, 1, 2, 3, 4, 5, 6, 7 or 8;
Z is -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- or
-CO-O-CₓH₂ₓ-NR(14)-,
where in each case both directions of linkage are possible;
x is 2, 3 or 4;
R(14) is hydrogen, alkyl having 1, 2 or 3 carbon atoms, -C_{y}H_{2y}-OR(12b), -C_{y}H_{2y}-NR(12b)₂;
R(12b) is hydrogen, methyl or ethyl;
y is 2 or 3;
R(13) is H, CF₃, C₂F₅, C₃F₇, cycloalkyl having 3, 4, 5, 6, 7 or 8 carbon atoms, -NR(15)R(16), -CONR(15)R(16), -C(=NR(17))NR(15)R(16), -OR(17), -COOR(17), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl, aminosulfonyl and methylsulfonylamino;
R(15) and R(16) independently of one another are hydrogen, alkyl having, 1, 2, 3 or 4 carbon atoms, or -C_{z}H_{2z}-phenyl, in which phenyl is unsubstituted or substituted by 1 or 2 substituents selected from the group consisting of F, Cl, Br, CF₃, NO₂, CN, OH, methyl, ethyl, methoxy, dimethylamino, sulfamoyl, methylsulfonyl, aminosulfonyl and methylsulfonylamino;
or
R(15) and R(16) together are a chain of 4 or 5 methylene groups, of which one CH₂ group can be replaced by -O-, -S-, -NH-, -N(CH₃)- or -N(benzyl)-;
R(17) is hydrogen or alkyl having 1, 2 or 3 carbon atoms;
R(5), R(6), R(7) and R(8) independently of one another are hydrogen, F, Cl, Br, I, alkyl having 1, 2, 3, 4 or 5 carbon atoms, cycloalkyl having 3, 4, 5, 6, 7 or 8 carbon atoms, -CN, -CF₃, -C₂F₅, -C₃F₇, -N₃, -NO₂, -Y-CₛH₂ₛ-R(18) or phenyl,
which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -SO₂-O-, -SO₂NR(10c), -NR(10c)- or -CONR(10c)-;
R(10c) is hydrogen or alkyl having 1, 2 or 3 carbon atoms;
s is zero, 1, 2, 3, 4, 5 or 6;
R(18) is hydrogen, CF₃, C₂F₅, C₃F₇, cycloalkyl having 3, 4, 5, 6, 7 or 8 carbon atoms, -COOR(21), 1-piperidyl, 1-pyrrolidinyl, 4-morpholinyl, 4-methylpiperazin-1-yl, pyridyl, thienyl, imidazolyl, quinolyl, isoquinolyl or phenyl;
R(21) is hydrogen or alkyl having 1, 2 or 3 carbon atoms;
R(9) is hydrogen, OR(10d) or OCOR(10d);
R(10d) is hydrogen or alkyl having 1, 2 or 3 carbon atoms;
B is hydrogen;
or
R(9) and B together are a bond;
or its physiologically tolerable salts.

2. A compound of the formula I as claimed in claim 1, in which:
R(1) and R(2) independently of one another are hydrogen, CF₃ or alkyl having 1, 2, 3, 4, 5 or 6 carbon atoms;
or
R(1) and R(2) together are an alkylene chain having 2, 3, 4, 5 or 6 carbon atoms;
R(3) is R(10)-CₙH₂ₙ-;
R(10) is methyl, CF₃ or C₂F₅;
n is zero, 1 or 2;
R(4) is R(13)-CᵣH₂ᵣ-Z-C_{q}H_{2q}-;
q is 1, 2, 3, 4, 5, 6, 7 or 8;
r is 0, 1, 2, 3, 4, 5, 6, 7 or 8;
Z is -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- or
-CO-O-CₓH₂ₓ-NR(14)-,
where in each case both directions of linkage are possible;
x is 2, 3 or 4;
R(14) is hydrogen, alkyl having 1, 2 or 3 carbon atoms, -C_{y}H_{2y}-OR(12b), -C_{y}H_{2y}-NR(12b)₂;
R(12b) is hydrogen, methyl or ethyl;
y is 2 or 3;
R(13) is H, CF₃, C₂F₅, C₃F₇, cycloalkyl having 3, 4, 5, 6, 7 or 8 carbon atoms, -NR(15)R(16), -CONR(15)R(16),
-C(=NR(17))NR(15)R(16), -OR(17), -COOR(17), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl,
aminosulfonyl and methylsulfonylamino;
R(15) and R(16) independently of one another are hydrogen, alkyl having 1, 2, 3 or 4 carbon atoms or -C_{z}H_{2z}-phenyl, in which phenyl is unsubstituted or substituted by 1 or 2 substituents selected from the group consisting of F, Cl, Br, CF3, NO2, CN, OH, methyl, ethyl, methoxy, dimethylamino, sulfamoyl, methylsulfonyl, aminosulfonyl and methylsulfonylamino;
or
R(15) and R(16) together are a chain of 4 or 5 methylene groups, of which one CH₂ group can be replaced by -O-, -S-, -NH-, -N(CH₃)- or -N(benzyl)-;
R(17) is hydrogen or alkyl having 1, 2 or 3 carbon atoms;
R(5), R(6), R(7) and R(8) independently of one another are hydrogen, F, Cl, Br, I, alkyl having 1, 2, 3, 4 or 5 carbon atoms, cycloalkyl having 3, 4, 5, 6 or 7 carbon atoms, -CN, -CF₃, -C₂F₅, -C₃F₇, -NO₂, -Y-CₛH₂ₛ-R(18) or phenyl, which is unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
Y is -O-, -CO-, -CO-O-, -O-CO-, -S-, -SO-, -SO₂-, -SO₂-O-, -SO₂NR(10c), -NR(10c)- or -CONR(10c)-;
R(10c) is hydrogen or alkyl having 1, 2 or 3 carbon atoms;
s is zero, 1, 2, 3, 4, 5 or 6;
R(18) is hydrogen, CF₃, C₂F₅, C₃F₇, cycloalkyl having 3, 4, 5, 6 or 7 carbon atoms, -COOR(21), 1-piperidyl, 1-pyrrolidinyl, 4-morpholinyl, 4-methylpiperazin-1-yl, pyridyl, imidazolyl or phenyl
R(21) is hydrogen or alkyl having 1, 2 or 3 carbon atoms;
R(9) is hydrogen or OR(10d);
R(10d) is hydrogen or methyl;
B is hydrogen;
or
R(9) and B together are a bond;
or its physiologically tolerable salts.

3. A compound of the formula I as claimed in claims 1 and 2, in which:
R(1) and R(2) independently of one another are hydrogen, CF₃ or alkyl having 1, 2 or 3 carbon atoms;
or
R(1) and R(2) together are an alkylene chain having 2, 3, 4 or 5 carbon atoms;
R(3) is R(10)-CₙH₂ₙ-;
R(10) is methyl, CF₃ or C₂F₅;
n is zero, 1 or 2;
R(4) is R(13)-CᵣH₂ᵣ-Z-C_{q}H_{2q}-;
q is 1 , 2, 3 or 4;
r is 0, 1, 2 or 3;
Z is -CO-NR(14)-,
-OCO-NR(14)-,
-O-CₓH₂ₓ-O-,
-O-CₓH₂ₓ-NR(14)-,
-O-CₓH₂ₓ-CO-O,
-CO-O-CₓH₂ₓ-O- or
-CO-O-CₓH₂ₓ-NR(14)-,
where in each case both directions of linkage are possible;
x is 2 or 3;
R(14) is hydrogen, alkyl having 1 or 2 carbon atoms;
R(13) is CH₃, CF₃, C₂F₅, cycloalkyl having 3, 4, 5, 6 or 7 carbon atoms, -NR(15)R(16), -C(=NR(17))NR(15)R(16), -OR(17), -COOR(17), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl, aminosulfonyl and methylsulfonylamino;
R(15) and R(16) independently of one another are hydrogen, alkyl having 1, 2, 3 or 4 carbon atoms or -C_{z}H_{2z}-phenyl, in which phenyl is unsubstituted or substituted by 1 or 2 substituents selected from the group consisting of F, Cl, Br, CF3, NO2, CN, OH, methyl, ethyl, methoxy, dimethylamino, sulfamoyl, methylsulfonyl, aminosulfonyl and methylsulfonylamino;
or
R(15) and R(16) together are a chain of 4 or 5 methylene groups, of which one CH₂ group can be replaced by -O-, -S-, -NH-, -N(CH₃)- or -N(benzyl)-;
R(17) is hydrogen or alkyl having 1 or 2 carbon atoms;
R(5) and R(6) independently of one another are hydrogen, F, Cl, Br, alkyl having 1, 2, 3, 4 or 5 carbon atoms, cycloalkyl having 3, 4, 5, 6 or 7 carbon atoms, -CN, -CF₃, -C₂F₅, -NO₂, -Y-CₛH₂ₛ-R(18) or phenyl,
which is unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
Y is -O-, -CO-, -SO₂- or -CONR(10c)-;
R(10c) is hydrogen or alkyl having 1 or 2 carbon atoms;
s is zero, 1, 2, 3, 4, 5 or 6;
R(18) is hydrogen, CF₃, C₂F₅, cycloalkyl having 3, 4, 5, 6 or 7 carbon atoms, 1-piperidyl, 1-pyrrolidinyl, 4-morpholinyl, 4-methylpiperazin-1-yl, pyridyl, imidazolyl or phenyl;
R(7) and R(8) are hydrogen;
R(9) is hydrogen or OR(10d);
R(10d) is hydrogen or methyl;
B is hydrogen;
or
R(9) and B together are a bond;
or its physiologically tolerable salts.

4. A compound of the formula I as claimed in claims 1 to 3, in which:
R(1) and R(2) are methyl;
R(3) is methyl or ethyl;
R(4) is R(13)-CᵣH₂ᵣ-Z-C_{q}H_{2q}-;
q is 1, 2, 3 or 4;
r is 0, 1, 2 or 3;
Z is -CO-NR(14)-,
-OCO-NR(14)-,
-O-CₓH₂ₓ-NR(14)- or
-CO-O-CₓH₂ₓ-NR(14)-;
x is 2 or 3;
R(14) is hydrogen or methyl;
R(13) is CH₃, CF₃, -OR(17), -COOR(17), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl, aminosulfonyl and methylsulfonylamino;
R(17) is hydrogen or alkyl having 1 or 2 carbon atoms;
R(5) is hydrogen, alkyl having 1, 2, 3 or 4 carbon atoms, F, Cl, methoxy or ethoxy;
R(6) is F, Cl, alkyl having 1, 2, 3, 4 or 5 carbon atoms, -CF₃, -Y-CₛH₂ₛ-R(18) or phenyl,
which is unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
Y is -O-, -CO- or -CONR(10c)-;
R(10c) is hydrogen or methyl;
s is 1, 2, 3, 4 or 5;
R(18) is hydrogen, CF₃ or phenyl;
R(7) and R(8) are hydrogen;
R(9) is hydrogen;
B is hydrogen;
or its physiologically tolerable salts.

5. A compound of the formula I as claimed in claims 1 to 3, in which:
R(1) and R(2) are methyl:
R(3) is methyl or ethyl;
R(4) is R(13)-CᵣH₂ᵣ-Z-C_{q}H_{2q}-;
q is 1, 2, 3 or 4;
r is 0, 1, 2 or 3;
Z is -CO-NR(14)-,
-OCO-NR(14)-,
-O-CₓH₂ₓ-NR(14)- or
-CO-O-CₓH₂ₓ-NR(14)-;
x is 2 or 3;
R(14) is hydrogen or methyl;
R(13) is CH₃, CF₃, -OR(17), -COOR(17), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl, aminosulfonyl and methylsulfonylamino;
R(17) is hydrogen or alkyl having 1 or 2 carbon atoms;
R(5) is hydrogen, alkyl having 1, 2, 3 or 4 carbon atoms, F, Cl, methoxy or ethoxy;
R(6) is F, Cl, alkyl having 1, 2, 3, 4 or 5 carbon atoms, -CF₃, -Y-CₛH₂ₛ-R(18) or phenyl,
which is unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
Y is -O-, -CO- or -CONR(10c)-;
R(10c) is hydrogen or methyl;
s is 1, 2, 3, 4 or 5;
R(18) is hydrogen, CF₃ or phenyl;
R(7) and R(8) are hydrogen;
R(9) is OH;
B is hydrogen;
or its physiologically tolerable salts.

6. A compound of the formula I as claimed in claims 1 to 3, in which:
R(1) and R(2) are methyl;
R(3) is methyl or ethyl;
R(4) is R(13)-CᵣH₂ᵣ-Z-C_{q}H_{2q}-;
q is 1, 2, 3 or 4;
r is 0, 1, 2 or 3;
Z is -CO-NR(14)-,
-OCO-NR(14)-,
-O-CₓH₂ₓ-NR(14)- or
-CO-O-CₓH₂ₓ-NR(14)-;
x is 2 or 3;
R(14) is hydrogen or methyl;
R(13) is CH₃, CF₃, -OR(17), -COOR(17), phenyl or an N-containing heterocycle having 1, 2, 3, 4, 5, 6, 7, 8 or 9 carbon atoms,
where phenyl and the N-containing heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl, aminosulfonyl and methylsulfonylamino;
R(17) is hydrogen or alkyl having 1 or 2 carbon atoms;
R(5) is hydrogen, alkyl having 1, 2, 3 or 4 carbon atoms, F, Cl, methoxy or ethoxy;
R(6) is F, Cl, alkyl having 1, 2, 3, 4 or 5 carbon atoms, -CF₃, -Y-CₛH₂ₛ-R(18) or phenyl,
which is unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
Y is -O-, -CO- or -CONR(10c)-;
R(10c) is hydrogen or methyl;
s is 1, 2, 3, 4 or 5;
R(18) is hydrogen, CF₃ or phenyl;
R(7) and R(8) are hydrogen;
R(9) and B together are a bond;
or its physiologically tolerable salts.

7. A compound of the formula I as claimed in claim 1 or its physiologically tolerable salts for use as a pharmaceutical.

8. A pharmaceutical preparation comprising an efficacious annount of at least one compound of the formula I as claimed in one or more of claims 1 to 6 and/or a physiologically tolerable salt thereof as active compound, together with pharmaceutically acceptable excipients and additives and, if appropriate, additionally one or more other pharmacological active compounds.

9. The use of a compound of the formula I as claimed in one or more of claims 1 to 6 and/or of a physiologically tolerable salt thereof for the production of a medicament having K⁺ channel-blocking action for the therapy and prophylaxis of K⁺ channel-mediated diseases.

10. The use of a compound of the formula I as claimed in one or more of claims 1 to 6 and/or of a physiologically tolerable salt thereof for the production of a medicament for inhibiting gastric acid secretion.

11. The use of a compound of the formula I as claimed in one or more of claims 1 to 6 and/or of a physiologically tolerable salt thereof for the production of a medicament for the therapy or prophylaxis of ulcers of the stomach or of the intestinal region.

12. The use of a compound of the formula I as claimed in one or more of claims 1 to 6 and/or of a physiologically tolerable salt thereof for the production of a medicament for the therapy or prophylaxis of reflux esophagitis.

13. The use of a compound of the formula I as claimed in one or more of claims 1 to 6 and/or of a physiologically tolerable salt thereof for the production of a medicament for the therapy or prophylaxis of diarrheal disorders.

14. The use of a compound of the formula I as claimed in one or more of claims 1 to 6 and/or of a physiologically tolerable salt thereof for the production of a medicament for the therapy or prophylaxis of all types of arrhythmias, including atrial, ventricular and supraventricular arrhythmias.

15. The use of a compound of the formula I as claimed in one or more of claims 1 to 6 and/or of a physiologically tolerable salt thereof for the production of a medicament for the therapy or prophylaxis of cardiac arrhythmias which can be eliminated by action potential prolongation.

16. The use of a compound of the formula I as claimed in one or more of claims 1 to 6 and/or of a physiologically tolerable salt thereof for the production of a medicament for the therapy or prophylaxis of atrial fibrillation or atrial flutters.

17. The use of a compound of the formula I as claimed in one or more of claims 1 to 6 and/or of a physiologically tolerable salt thereof for the production of a medicament for the therapy or prophylaxis of reentry arrhythmias or for the prevention of sudden heart death as a result of ventricular fibrillation.

18. The use of a compound of the formula I as claimed in one or more of claims 1 to 6 and/or of a physiologically tolerable salt thereof for the production of a medicament for the therapy of cardiac insufficiency.

19. The use of a compound of the formula I as claimed in one or more of claims 1 to 6 and/or of a physiologically tolerable salt thereof for the production of a medicament for inhibiting stimulated gastric acid secretion, for the therapy or prophylaxis of ulcers of the stomach or of the intestinal region, of reflux esophagitis, of diarrheal disorders, for the therapy or prophylaxis of arrhythmias, including atrial, ventricular and supraventricular arrhythmias, atrial fibrillation and atrial flutters and of reentry arrhythmias, or for the prevention of sudden heart death as a result of ventricular fibrillation.

## Revendications

1. Composés de formule I 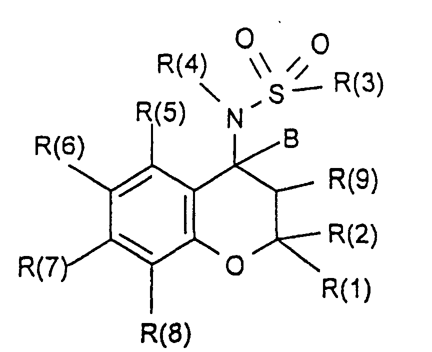 dans laquelle
R(1) et R(2) représentent, indépendamment l'un de l'autre, un atome d'hydrogène ou un groupe CF₃, C₂F₅, C₃F₇, alkyle de 1, 2, 3, 4, 5 ou 6 atomes de carbone ou phényle non substitué ou substitué par 1 ou 2 substituants choisis dans le groupe constitué par F, Cl, Br, I, CF₃, NO₂, CN, NH₂, OH, méthyle, éthyle, méthoxy, diméthylamino, sulfamoyle, méthylsulfonyle et méthylsulfonylamino;
ou
R(1) et R(2) forment ensemble une chaîne alkylène de 2, 3, 4, 5, 6, 7, 8, 9 ou 10 atomes de carbone;
R(3) représente un groupe R(10)-CₙH₂ₙ-NR(11)- ou R(10)-CₙH₂ₙ-, où un groupe CH₂ dans les groupes CₙH₂ₙ peut être remplacé par -O-, -CO-, -S-, -SO-, -SO₂- ou -NR(12a)-, R(12a) étant un atome d'hydrogène ou un groupe méthyle ou éthyle;
R(10) représente un atome d'hydrogène ou un groupe méthyle, cycloalkyle de 3, 4, 5, 6, 7 ou 8 atomes de carbone, CF₃, C₂F₅ ou C₃F₇;
n est 0, 1, 2, 3, 4, 5, 6, 7, 8, 9 ou 10;
R(11) représente un atome d'hydrogène ou un groupe alkyle de 1, 2, 3, 4, 5 ou 6 atomes de carbone; ou
R(10) et R(11) forment ensemble une liaison, dans la mesure où n est au moins égal à 3;
R(4) représente un groupe R(13)-CᵣH₂ᵣ-Z-C_{q}H_{2q}-;
q est égal à 0, 1, 2, 3, 4, 5, 6, 7 ou 8;
r est égal à 0, 1, 2, 3,4, 5, 6, 7 ou 8;
Z représente un groupe -CO-NR(14)-,
-OCO-NR(14)-,
-O-CₓH₂ₓ-O-,
-O-CₓH₂ₓ-NR(14)-,
-O-CₓH₂ₓ-CO-O-,
-CO-O-CₓH₂ₓ-O- ou
-CO-O-CₓH₂ₓ-NR(14)-,
les deux sens de liaison étant possibles à chaque fois;
x est égal à 2, 3 ou 4;
R(14) représente un atome d'hydrogène ou un groupe alkyle de 1, 2 ou 3 atomes de carbone, -C_{y}H_{2y}-OR(12b), -C_{y}H_{2y}-NR(12b)₂;
R(12b) est un atome d'hydrogène ou un groupe méthyle ou éthyle;
y est égal à 2 ou 3;
R(13) est H ou un groupe CF₃, C₂F₅, C₃F₇, cycloalkyle de 3, 4, 5, 6, 7 ou 8 atomes de carbone, -NR(15)R(16), -CONR(15)R(16),
-C(=NR(17))NR(15)R(16), -OR(17), -COOR(17) ou phényle ou un hétérocycle azoté contenant 1, 2, 3, 4, 5, 6, 7, 8 ou 9 atomes de carbone,
le groupe phényle et l'hétérocycle azoté étant non substitués ou substitués par 1 ou 2 substituants choisis dans le groupe constitué par F, Cl, Br, I, CF₃, NO₂, CN, NH₂, OH, méthyle, éthyle, méthoxy, diméthylamino, sulfamoyle, méthylsulfonyle, aminosulfonyle et méthylsulfonylamino;
R(15) et R(16) représentent indépendamment l'un de l'autre un atome d'hydrogène ou un groupe alkyle de 1, 2, 3 ou 4 atomes de carbone ou -C_{z}H_{2z}-phényle, le groupe phényle étant non substitué ou substitué par 1 ou 2 substituants choisis dans le groupe constitué par F, Cl, Br, CF₃, NO₂, CN, OH, méthyle, éthyle, méthoxy, diméthylamino, sulfamoyle, méthylsulfonyle, aminosulfonyle et méthylsulfonylamino; ou
R(15) et R(16) représentent ensemble une chaîne de 4 ou 5 groupes méthylène, dont un groupe CH₂ peut être remplacé par -O-, -S-, -NH-, -N(CH₃)- ou -N(benzyle)-;
R(17) représente un atome d'hydrogène ou un groupe alkyle de 1, 2 ou 3 atomes de carbone;
R(5), R(6), R(7) et R(8) représentent, indépendamment les uns des autres, un atome d'hydrogène, F, Cl, Br, I, ou un groupe alkyle de 1, 2, 3, 4 ou 5 atomes de carbone, cycloalkyle de 3, 4, 5, 6, 7 ou 8 atomes de carbone, -CN, -CF₃, -C₂F₅, -C₃F₇, -N₃, -NO₂, -Y-CₛH₂ₛ-R(18) ou phényle non substitué ou substitué par 1 ou 2 substituants choisis dans le groupe constitué par F, Cl, Br, I, CF₃, NO2, CN, NH₂, OH, méthyle, éthyle, méthoxy, diméthylamino, sulfamoyle, méthylsulfonyle et méthylsulfonylamino;
Y représente -O-, -CO-, -CO-O-, -O-CO-, -S-, -SO-, -SO₂-, -SO₂-O-, -SO₂NR(10c), -NR(10c)- ou -CONR(10c)-, où R(10c) est un atome d'hydrogène ou un groupe alkyle de 1, 2 ou 3 atomes de carbone;
s est égal à 0, 1, 2, 3, 4, 5 ou 6;
R(18) est un atome d'hydrogène ou un groupe CF₃, C₂F₅, C₃F₇, cycloalkyle de 3, 4, 5, 6, 7 ou 8 atomes de carbone, -COOR(21), 1-pipéridyle, 1-pyrrolidinyle, 4-morpholinyle, 4-méthylpipérazin-1-yle, pyridyle, thiényle, imidazolyle, quinoléyle, isoquinoléyle ou phényle;
R(21) est un atome d'hydrogène ou un groupe alkyle de 1, 2 ou 3 atomes de carbone;
R(9) est un atome d'hydrogène ou un groupe OR(10d) ou OCOR(10d);
R(10d) est un atome d'hydrogène ou un groupe alkyle de 1, 2 ou 3 atomes de carbone;
B est un atome d'hydrogène; ou
R(9) et B forment ensemble une liaison;
et leurs sels physiologiquement acceptables.

2. Composés de formule I selon la revendication 1, dans lesquels
R(1) et R(2) représentent, indépendamment l'un de l'autre, un atome d'hydrogène ou un groupe CF₃ ou alkyle de 1, 2, 3, 4, 5 ou 6 atomes de carbone; ou
R(1) et R(2) forment ensemble une chaîne alkylène de 2, 3, 4, 5 ou 6 atomes de carbone;
R(3) représente un groupe R(10)-CₙH₂ₙ-;
R(10) représente un groupe méthyle, CF₃ ou C₂F₅;
n est 0, 1 ou 2;
R(4) représente un groupe R(13)-CᵣH₂ᵣ-Z-C_{q}H_{2q}-;
q est égal à 1, 2, 3, 4, 5, 6, 7 ou 8;
r est égal à 0, 1, 2, 3, 4, 5, 6, 7 ou 8;
Z représente un groupe -CO-NR(14)-,
-OCO-NR(14)-,
-O-CₓH₂ₓ-O-,
-O-CₓH₂ₓ-NR(14)-,
-O-CₓH₂ₓ-CO-O-,
-CO-O-CₓH₂ₓ-O- ou
-CO-O-CₓH₂ₓ-NR(14)-,
les deux sens de liaison étant possibles à chaque fois;
x est égal à 2, 3 ou 4;
R(14) représente un atome d'hydrogène ou un groupe alkyle de 1, 2 ou 3 atomes de carbone, -C_{y}H_{2y}-OR(12b), -C_{y}H_{2y}-NR(12b)₂;
R(12b) est un atome d'hydrogène ou un groupe méthyle ou éthyle;
y est égal à 2 ou 3;
R(13) est H ou un groupe CF₃, C₂F₅, C₃F₇, cycloalkyle de 3, 4, 5, 6, 7 ou 8 atomes de carbone, -NR(15)R(16), -CONR(15)R(16),
-C(=NR(17))NR(15)R(16), -OR(17), -COOR(17) ou phényle ou un hétérocycle azoté contenant 1, 2, 3, 4, 5, 6, 7, 8 ou 9 atomes de carbone,
le groupe phényle et l'hétérocycle azoté étant non substitués ou substitués par 1 ou 2 substituants choisis dans le groupe constitué par F, Cl, Br, I, CF₃,
NO₂, CN, NH₂, OH, méthyle, éthyle, méthoxy, diméthylamino, sulfamoyle, méthylsulfonyle, aminosulfonyle et méthylsulfonylamino;
R(15) et R(16) représentent indépendamment l'un de l'autre un atome d'hydrogène ou un groupe alkyle de 1, 2, 3 ou 4 atomes de carbone ou -C_{z}H_{2z}-phényle, le groupe phényle étant non substitué ou substitué par 1 ou 2 substituants choisis dans le groupe constitué par F, Cl, Br, CF₃, NO₂, CN, OH, méthyle, éthyle, méthoxy, diméthylamino, sulfamoyle, méthylsulfonyle, aminosulfonyle et méthylsulfonylamino; ou
R(15) et R(16) représentent ensemble une chaîne de 4 ou 5 groupes méthylène, dont un groupe CH₂ peut être remplacé par -O-, -S-, -NH-, -N(CH₃)- ou -N(benzyle)-;
R(17) représente un atome d'hydrogène ou un groupe alkyle de 1, 2 ou 3 atomes de carbone;
R(5), R(6), R(7) et R(8) représentent, indépendamment les uns des autres, un atome d'hydrogène, F, Cl, Br, I, ou un groupe alkyle de 1, 2, 3, 4 ou 5 atomes de carbone, cycloalkyle de 3, 4, 5, 6, ou 7 atomes de carbone, -CN, -CF₃, -C₂F₅, -C₃F₇, -NO₂, -Y-CₛH₂ₛ-R(18) ou phényle non substitué ou substitué par 1 ou 2 substituants choisis dans le groupe constitué par F, Cl, Br, I, CF₃, NO₂, CN, NH₂, OH, méthyle, éthyle, méthoxy, diméthylamino, sulfamoyle, méthylsulfonyle et méthylsulfonylamino;
Y représente -O-, -CO-, -CO-O-, -O-CO-, -S-, -SO-, -SO₂-, -SO₂-O-, -SO₂NR(10c), -NR(10c)- ou -CONR(10c)-, où R(10c) est un atome d'hydrogène ou un groupe alkyle de 1, 2 ou 3 atomes de carbone;
s est égal à 0, 1, 2, 3, 4, 5 ou 6;
R(18) est un atome d'hydrogène ou un groupe CF₃, C₂F₅, C₃F₇, cycloalkyle de 3, 4, 5, 6 ou 7 atomes de carbone, -COOR(21), 1-pipéridyle, 1-pyrrolidinyle, 4-morpholinyle, 4-méthylpipérazin-1-yle, pyridyle, imidazolyle ou phényle;
R(21) est un atome d'hydrogène ou un groupe alkyle de 1, 2 ou 3 atomes de carbone;
R(9) est un atome d'hydrogène ou un groupe OR(10d);
R(10d) est un atome d'hydrogène ou un groupe méthyle;
B est un atome d'hydrogène; ou
R(9) et B forment ensemble une liaison;
et leurs sels physiologiquement acceptables.

3. Composés de formule I selon les revendications 1 à 2, dans lesquels
R(1) et R(2) représentent, indépendamment l'un de l'autre, un atome d'hydrogène ou un groupe CF₃ ou alkyle de 1, 2 ou 3 atomes de carbone; ou
R(1) et R(2) forment ensemble une chaîne alkylène de 2, 3, 4 ou 5 atomes de carbone;
R(3) représente un groupe R(10)-CₙH₂ₙ-;
R(10) représente un groupe méthyle, CF₃ ou C₂F₅;
n est 0, 1 ou 2;
R(4) représente un groupe R(13)-CᵣH₂ᵣ-Z-C_{q}H_{2q}-;
q est égal à 1, 2, 3 ou 4;
r est égal à 0, 1, 2 ou 3;
Z représente un groupe -CO-NR(14)-,
-OCO-NR(14)-,
-O-CₓH₂ₓ-O-,
-O-CₓH₂ₓ-NR(14)-,
-O-CₓH₂ₓ-CO-O-,
-CO-O-CₓH₂ₓ-O- ou
-CO-O-CₓH₂ₓ-NR(14)-,
les deux sens de liaison étant possibles à chaque fois;
x est égal à 2 ou 3;
R(14) représente un atome d'hydrogène ou un groupe alkyle de 1 ou 2 atomes de carbone;
R(13) est CH₃, CF₃, C₂F₅, C₃F₇, cycloalkyle de 3, 4, 5, 6 ou 7 atomes de carbone, -NR(15)R(16), -C(=NR(17))NR(15)R(16), -OR(17), -COOR(17) ou phényle ou un hétérocycle azoté contenant 1, 2, 3, 4, 5, 6, 7, 8 ou 9 atomes de carbone,
le groupe phényle et l'hétérocycle azoté étant non substitués ou substitués par 1 ou 2 substituants choisis dans le groupe constitué par F, Cl, Br, CF₃,
NO₂, CN, NH₂, OH, méthyle, éthyle, méthoxy, diméthylamino, sulfamoyle, méthylsulfonyle, aminosulfonyle et méthylsulfonylamino;
R(15) et R(16) représentent indépendamment l'un de l'autre un atome d'hydrogène ou un groupe alkyle de 1, 2, 3 ou 4 atomes de carbone ou -C_{z}H_{2z}-phényle, le groupe phényle étant non substitué ou substitué par 1 ou 2 substituants choisis dans le groupe constitué par F, Cl, Br, CF₃, NO₂, CN, OH, méthyle, éthyle, méthoxy, diméthylamino, sulfamoyle, méthylsulfonyle, aminosulfonyle et méthylsulfonylamino; ou
R(15) et R(16) représentent ensemble une chaîne de 4 ou 5 groupes méthylène, dont un groupe CH₂ peut être remplacé par -O-, -S-, -NH-, -N(CH₃)- ou -N(benzyle)-;
R(17) représente un atome d'hydrogène ou un groupe alkyle de 1 ou 2 atomes de carbone;
R(5) et R(6) représentent, indépendamment les uns des autres, un atome d'hydrogène, F, Cl, Br, ou un groupe alkyle de 1, 2, 3, 4 ou 5 atomes de carbone, cycloalkyle de 3, 4, 5, 6, ou 7 atomes de carbone, -CN, -CF₃, -C₂F₅, -NO₂, -Y-CₛH₂ₛ-R(18) ou phényle non substitué ou substitué par 1 ou 2 substituants choisis dans le groupe constitué par F, Cl, Br, CF₃, NO₂, CN, NH₂, OH, méthyle, éthyle, méthoxy, diméthylamino, sulfamoyle, méthylsulfonyle et méthylsulfonylamino;
Y représente -O-, -CO-, -SO₂- ou -CONR(10c)-, où R(10c) est un atome d'hydrogène ou un groupe alkyle de 1 ou 2 atomes de carbone;
s est égal à 0, 1, 2, 3, 4, 5 ou 6;
R(18) est un atome d'hydrogène ou un groupe CF₃, C₂F₅, cycloalkyle de 3, 4, 5, 6 ou 7 atomes de carbone, 1-pipéridyle, 1-pyrrolidinyle, 4-morpholinyle, 4-méthylpipérazin-1-yle, pyridyle, imidazolyle ou phényle;
R(7) et R(8) représentent des atomes d'hydrogène;
R(9) est un atome d'hydrogène ou un groupe OR(10d);
R(10d) est un atome d'hydrogène ou un groupe méthyle;
B est un atome d'hydrogène; ou
R(9) et B forment ensemble une liaison;
et leurs sels physiologiquement acceptables.

4. Composés de formule I selon les revendications 1 à 3, dans lesquels
R(1) et R(2) représentent des groupes méthyle;
R(3) représente un groupe méthyle ou éthyle;
R(4) représente un groupe R(13)-CᵣH₂ᵣ-Z-C_{q}H_{2q}-;
q est égal à 1, 2, 3 ou 4;
r est égal à 0, 1, 2 ou 3;
Z représente un groupe -CO-NR(14)-,
-OCO-NR(14)-,
-O-CₓH₂ₓ-NR(14)-, ou
-CO-O-CₓH₂ₓ-NR(14)-,
x est égal à 2 ou 3;
R(14) représente un atome d'hydrogène ou un groupe méthyle;
R(13) est un groupe CH₃, CF₃, -OR(17), -COOR(17) ou phényle ou un hétérocycle azoté contenant 1, 2, 3, 4, 5, 6, 7, 8 ou 9 atomes de carbone, le groupe phényle et l'hétérocycle azoté étant non substitués ou substitués par 1 ou 2 substituants choisis dans le groupe constitué par F, Cl, Br, CF₃, NO₂, CN, NH₂, OH, méthyle, éthyle, méthoxy, diméthylamino, sulfamoyle, méthylsulfonyle, aminosulfonyle et méthylsulfonylamino;
R(17) représente un atome d'hydrogène ou un groupe alkyle de 1 ou 2 atomes de carbone;
R(5) représente un atome d'hydrogène ou un groupe alkyle de 1, 2, 3 ou 4 atomes de carbone, F, Cl, méthoxy ou éthoxy;
R(6) représente F, Cl ou un groupe alkyle de 1, 2, 3, 4 ou 5 atomes de carbone, -CF₃, -Y-CₛH₂ₛ-R(18) ou phényle non substitué ou substitué par 1 ou 2 substituants choisis dans le groupe constitué par F, Cl, Br, CF₃, NO₂, CN, NH₂, OH, méthyle, éthyle, méthoxy, diméthylamino, sulfamoyle, méthylsulfonyle et méthylsulfonylamino;
Y représente -O-, -CO- ou -CONR(10c)-, où R(10c) est un atome d'hydrogène ou un groupe méthyle;
s est égal à 1, 2, 3, 4 ou 5;
R(18) est un atome d'hydrogène ou un groupe CF₃ ou phényle;
R(7) et R(8) représentent des atomes d'hydrogène;
R(9) est un atome d'hydrogène;
B est un atome d'hydrogène;
et leurs sels physiologiquement acceptables.

5. Composés de formule I selon les revendications 1 à 3, dans lesquels
R(1) et R(2) représentent des groupes méthyle;
R(3) représente un groupe méthyle ou éthyle;
R(4) représente un groupe R(13)-CᵣH₂ᵣ-Z-C_{q}H_{2q}-;
q est égal à 1, 2, 3 ou 4;
r est égal à 0, 1, 2 ou 3;
Z représente un groupe -CO-NR(14)-,
-OCO-NR(14)-,
-O-CₓH₂ₓ-NR(14)-, ou
-CO-O-CₓH₂ₓ-NR(14)-,
x est égal à 2 ou 3;
R(14) représente un atome d'hydrogène ou un groupe méthyle;
R(13) est un groupe CH₃, CF₃, -OR(17), -COOR(17) ou phényle ou un hétérocycle azoté contenant 1, 2, 3, 4, 5, 6, 7, 8 ou 9 atomes de carbone, le groupe phényle et l'hétérocycle azoté étant non substitués ou substitués par 1 ou 2 substituants choisis dans le groupe constitué par F, Cl, Br, CF₃, NO₂, CN, NH₂, OH, méthyle, éthyle, méthoxy, diméthylamino, sulfamoyle, méthylsulfonyle, aminosulfonyle et méthylsulfonylamino;
R(17) représente un atome d'hydrogène ou un groupe alkyle de 1 ou 2 atomes de carbone;
R(5) représente un atome d'hydrogène ou un groupe alkyle de 1, 2, 3 ou 4 atomes de carbone, F, Cl, méthoxy ou éthoxy;
R(6) représente F, Cl ou un groupe alkyle de 1, 2, 3, 4 ou 5 atomes de carbone, -CF₃, -Y-CₛH₂ₛ-R(18) ou phényle non substitué ou substitué par 1 ou 2 substituants choisis dans le groupe constitué par F, Cl, Br, CF₃, NO₂, CN, NH₂, OH, méthyle, éthyle, méthoxy, diméthylamino, sulfamoyle, méthylsulfonyle et méthylsulfonylamino;
Y représente -O-, -CO- ou -CONR(10c)-, où R(10c) est un atome d'hydrogène ou un groupe méthyle;
s est égal à 1, 2, 3, 4 ou 5;
R(18) est un atome d'hydrogène ou un groupe CF₃ ou phényle;
R(7) et R(8) représentent des atomes d'hydrogène;
R(9) est un groupe OH;
B est un atome d'hydrogène;
et leurs sels physiologiquement acceptables.

6. Composés de formule I selon les revendications 1 à 3, dans lesquels
R(1) et R(2) représentent des groupes méthyle;
R(3) représente un groupe méthyle ou éthyle;
R(4) représente un groupe R(13)-CᵣH₂ᵣ-Z-C_{q}H_{2q}-;
q est égal à 1, 2, 3 ou 4;
r est égal à 0, 1, 2 ou 3;
Z représente un groupe -CO-NR(14)-,
-OCO-NR(14)-,
-O-CₓH₂ₓ-NR(14)-, ou
-CO-O-CₓH₂ₓ-NR(14)-,
x est égal à 2 ou 3;
R(14) représente un atome d'hydrogène ou un groupe méthyle;
R(13) est un groupe CH₃, CF₃, -OR(17), -COOR(17) ou phényle ou un hétérocycle azoté contenant 1, 2, 3, 4, 5, 6, 7, 8 ou 9 atomes de carbone, le groupe phényle et l'hétérocycle azoté étant non substitués ou substitués par 1 ou 2 substituants choisis dans le groupe constitué par F, Cl, Br, CF₃, NO₂, CN, NH₂, OH, méthyle, éthyle, méthoxy, diméthylamino, sulfamoyle, méthylsulfonyle, aminosulfonyle et méthylsulfonylamino;
R(17) représente un atome d'hydrogène ou un groupe alkyle de 1 ou 2 atomes de carbone;
R(5) représente un atome d'hydrogène ou un groupe alkyle de 1, 2, 3 ou 4 atomes de carbone, F, Cl, méthoxy ou éthoxy;
R(6) représente F, Cl ou un groupe alkyle de 1, 2, 3, 4 ou 5 atomes de carbone, -CF₃, -Y-CₛH₂ₛ-R(18) ou phényle non substitué ou substitué par 1 ou 2 substituants choisis dans le groupe constitué par F, Cl, Br, CF₃, NO₂, CN, NH₂, OH, méthyle, éthyle, méthoxy, diméthylamino, sulfamoyle, méthylsulfonyle et méthylsulfonylamino;
Y représente -O-, -CO- ou -CONR(10c)-, où R(10c) est un atome d'hydrogène ou un groupe méthyle;
s est égal à 1, 2, 3, 4 ou 5;
R(18) est un atome d'hydrogène ou un groupe CF₃ ou phényle;
R(7) et R(8) représentent des atomes d'hydrogène;
R(9) et B forment ensemble une liaison;
et leurs sels physiologiquement acceptables.

7. Composés de formule I selon la revendication 1 et leurs sels physiologiquement acceptables pour une utilisation comme médicaments.

8. Composition pharmaceutique contenant comme substance active une quantité active d'au moins un composé de formule I selon l'une ou plusieurs des revendications 1 à 6 et/ou d'un de ses sels physiologiquement acceptables, avec des supports et additifs pharmaceutiquement acceptables et éventuellement une ou plusieurs autres substances pharmacologiques.

9. Utilisation d'un composé de formule I selon l'une ou plusieurs des revendications 1 à 6 et/ou d'un de ses sels physiologiquement acceptables pour la préparation d'un médicament ayant une action de blocage des canaux de K⁺ pour le traitement et la prophylaxie de maladies dues aux canaux de K⁺.

10. Utilisation d'un composé de formule I selon l'une ou plusieurs des revendications 1 à 6 et/ou d'un de ses sels physiologiquement acceptables pour la préparation d'un médicament destiné à inhiber la sécrétion de l'acide gastrique.

11. Utilisation d'un composé de formule I selon l'une ou plusieurs des revendications 1 à 6 et/ou d'un de ses sels physiologiquement acceptables pour la préparation d'un médicament destiné au traitement ou à la prophylaxie d'ulcères de l'estomac ou de la zone intestinale.

12. Utilisation d'un composé de formule I selon l'une ou plusieurs des revendications 1 à 6 et/ou d'un de ses sels physiologiquement acceptables pour la préparation d'un médicament destiné au traitement ou à la prophylaxie de l'oesophagite peptique.

13. Utilisation d'un composé de formule I selon l'une ou plusieurs des revendications 1 à 6 et/ou d'un de ses sels physiologiquement acceptables pour la préparation d'un médicament destiné au traitement ou à la prophylaxie de maladies diarrhéiques.

14. Utilisation d'un composé de formule I selon l'une ou plusieurs des revendications 1 à 6 et/ou d'un de ses sels physiologiquement acceptables pour la préparation d'un médicament destiné au traitement ou à la prophylaxie de tous les types d'arythmies, y compris des arythmies auriculaires, ventriculaires et supraventriculaires.

15. Utilisation d'un composé de formule I selon l'une ou plusieurs des revendications 1 à 6 et/ou d'un de ses sels physiologiquement acceptables pour la préparation d'un médicament destiné au traitement ou à la prophylaxie de troubles du rythme cardiaque qui peuvent être éliminés par une extension du potentiel d'action.

16. Utilisation d'un composé de formule I selon l'une ou plusieurs des revendications 1 à 6 et/ou d'un de ses sels physiologiquement acceptables pour la préparation d'un médicament destiné au traitement ou à la prophylaxie de la fibrillation auriculaire ou du flutter auriculaire.

17. Utilisation d'un composé de formule I selon l'une ou plusieurs des revendications 1 à 6 et/ou d'un de ses sels physiologiquement acceptables pour la préparation d'un médicament destiné au traitement ou à la prophylaxie d'arythmies de ré-entrée ou à éviter la mort brutale par arrêt cardiaque par suite d'une fibrillation ventriculaire.

18. Utilisation d'un composé de formule I selon l'une ou plusieurs des revendications 1 à 6 et/ou d'un de ses sels physiologiquement acceptables pour la préparation d'un médicament destiné au traitement de l'insuffisance cardiaque.

19. Utilisation d'un composé de formule I selon l'une ou plusieurs des revendications 1 à 6 et/ou d'un de ses sels physiologiquement acceptables pour la préparation d'un médicament destiné à l'inhibition de la sécrétion stimulée de l'acide gastrique, au traitement ou à la prophylaxie d'ulcères de l'estomac ou de la zone intestinale, de l'oesophagite peptique, de maladies diarrhéiques, d'arythmies, y compris des arythmies auriculaires, ventriculaires et supraventriculaires, de la fibrillation auriculaire et du flutter auriculaire et d'arythmies de ré-entrée, ou à éviter la mort brutale par arrêt cardiaque par suite d'une fibrillation ventriculaire.
